# EUROPEAN PATENT APPLICATION

(11) **EP 4 446 319 A1**
(43) Date of publication of application: **16.10.2024**
(21) Application number: 23736981.4
(22) Date of filing: 03.01.2023
(51) Int. Cl.: C07D 403/14, A61K 31/506, A61K 31/4245, A61P 35/00, A61P 37/00, A61P 17/00, A61P 1/00

(54) **CARBONYL BRIDGED HETEROCYCLIC COMPOUND, AND COMPOSITION AND APPLICATION THEREOF**

(30) Priority: 04.01.2022 CN 202210003492; 09.12.2022 CN 202211582187
(71) Applicant: Beijing Scitech-MQ Pharmaceuticals Limited, Beijing 101320 (CN)
(72) Inventor: ZHANG, Qiang, Beijing 101320 (CN); YANG, Leifu, Beijing 101320 (CN)
(74) Representative: Maiwald GmbH
(86) International application number: PCT/CN2023/070093
(87) International publication number: WO 2023/131116

(57) **Abstract**

Provided are a carbonyl bridged heterocyclic compound having a structure represented by formula (I), a stereoisomer or a pharmaceutically acceptable salt thereof, a pharmaceutical composition containing these compounds, and an application of these compounds or composition in drug preparation. The compounds, the stereoisomer, the pharmaceutically acceptable salt thereof and the like can be used for treating or preventing autoimmune diseases, tumors and neurodegenerative diseases related to protein 1 kinase (RIPK1).

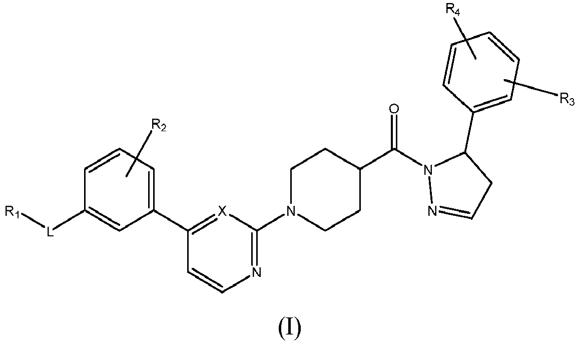

## Description

### TECHNICAL FIELD

The present disclosure belongs to the field of medicinal chemistry, and particularly relates to a class of carbonyl-bridged heterocyclic compounds, stereoisomers thereof, pharmaceutically acceptable salts thereof, and pharmaceutical compositions thereof, and use thereof in the manufacture of a medicament for the treatment of autoimmune diseases, tumors, inflammatory diseases and neurodegenerative diseases related to receptor-interacting protein 1 kinase (RIPK1).

### BACKGROUND

In the early days, it was thought that there are two main ways of cell death, i.e., apoptosis and necrosis of cells. Cell apoptosis is an autonomous and orderly death of cells controlled by genes in order to maintain the stability of the body's internal environment. It plays an important role in the evolution of an organism, the stability of an internal environment, and the development of a system. Cell necrosis is a pathological process in which cells are affected by physical, chemical and other environmental factors, such as mechanical damage, poisons, microorganisms, radiation, etc., causing cell death. In 2005, Degterev A et al. first discovered and reported an orderly cell necrosis process regulated by a series of biochemical molecules, and named it Necroptosis (also known as programmed necrosis). This process is a kind of programmed cell necrosis produced by stimulating death receptors with TNF-α, FasL or TRAIL, etc. Morphologically, it is manifested as cell swelling, cell volume increasing, organelle dysfunction, damage to cell membrane integrity, release of cell contents, and mass production of ROS, etc. Cell necroptosis was initially believed to be a defense mechanism evolved by the host to resist anti-apoptotic viruses. However, it was later discovered that the occurrence of necroptosis can lead to an imbalance of inflammatory factors, causing acute or chronic inflammatory diseases.

Receptor-interacting protein 1 kinase (RIPK1) belongs to the TKL family of serine/threonine protein kinases. Members of the RIPK serine/threonine kinase family have the same N-terminal kinase domain, but different binding domains. Studies have shown that receptor-interacting protein 1 can regulate the process of cell apoptosis. The death domain of RIPK-1 binds to death receptors such as TNFR1, Fas, TRAILR1, TRAILR2, etc. The death receptors can also bind to other proteins containing death domains, such as TRADD, FADD, etc. Binding to the latter is a necessary condition for activating caspase-8 and inducing apoptosis. The intermediate structure of RIPK-1 is the RIPK isotype interaction target, through which RIPK-1 can interact with RIPK-3. Kelliher et al. found that mice with congenital defects of RIPK-1 died less than 3 days after birth due to a large number of cell apoptosis, which indicates that RIPK-1 is also involved in regulating cell apoptosis. In addition, cells with congenital RIPK-1 deficiency are quite sensitive to TNF-induced cell death, perhaps because such cells cannot effectively activate NF-kB.

On the other hand, studies have also shown that RIPK-1 and RIPK-3 are also involved in the process of cell necroptosis. In most cell types, death receptors TNFR1, Fas and TRAILR mediate cell apoptosis. Activating TNFR1 can trigger the ubiquitination of RIPK-1 through cIAP1 and cIAP2, and ubiquitinated RIPK-1 determines the next step of cells: survival or death. When the apoptosis pathway is blocked by the pan-caspase inhibitor z-VAD-fmk, the death of cells will proceed to necroptosis. In this process, the activity of RIPK-1 is a key factor, which is regulated by FasL, TNF and TRAIL death receptors.

Recent studies have shown that the process of necroptosis is related to a variety of diseases, including tumors, autoimmune diseases, neurodegenerative diseases, inflammatory diseases and the like. Based on this, it can be known that RIP family kinases are closely related to the occurrence of tumors, autoimmune diseases, neurodegenerative diseases, inflammatory diseases and other diseases.

For example, in a study of Alzheimer's disease, Claudia Balducci et al. found that activated microglia play an important role in the evolution of Alzheimer's disease (Pharmacological Research. 2018; 130: 402-413). At the same time, microglia highly express RIPK-1, and RIPK-1 inhibitors can protect against Aβ-induced neuronal death and reduce microglial proliferation in vitro. Moreover, in an Alzheimer-like rat model, RIPK-1 inhibitors can improve learning and memory abilities of rats. In addition to Alzheimer's disease, RIPK-1 inhibitors are also expected to be used in a variety of other neurodegenerative diseases including Parkinson's disease, amyotrophic lateral sclerosis, and Huntington disease, etc.

At present, there are not many studies on RIPK-1 inhibitors, and only a small number of studies have entered a clinical stage. There is an urgent need for research and development of more drugs based on RIPK-1 inhibitors. On the other hand, there are even fewer studies on RIPK-1 inhibitors that can penetrate the blood-brain barrier. For Alzheimer's disease mentioned above and many other neurodegenerative diseases, the ability of drugs to penetrate the blood-brain barrier is crucial to the treatment effect.

In addition, in modern drug development, preclinical animal testing is a very important part, and rats or mice are usually used for testing in the early stages of research. From the perspective of translational medicine, animal models and their results should be consistent and adaptable to the pathophysiology and treatment of corresponding human diseases. Therefore, the compounds to be developed should preferably have small species differences in biological activity between humans and mice. GSK disclosed a large number of compounds with certain RIPK-1 inhibitory activity in patent application WO018/092089, but these compounds have poor inhibitory activity on mouse cells, which is not conducive to drug development through animal testing.

The present application provides a class of carbonyl-bridged heterocyclic compounds that exhibit good RIPK-1 inhibitory activity and the ability to penetrate the blood-brain barrier. It is worth emphasizing that the class of compounds not only have extremely strong inhibitory activity against human RIPK-1, but also have extremely strong inhibitory activity against mouse RIPK-1. In other words, the compounds of the present application show certain similarities and adaptabilities when extrapolated to human diseases using the results of animal testing, which is conducive to drug development through animal testing. The compounds of the present application are expected to be used as RIPK-1 inhibitors in the manufacture of a medicament for the treatment of tumors, autoimmune diseases, neurodegenerative diseases and inflammatory diseases.

### SUMMARY

The present disclosure provides a compound represented by formula (I), a pharmaceutically acceptable salt thereof, or a stereoisomer thereof, which can be used to prepare medicaments for the treatment or prevention of diseases related to RIPK1. 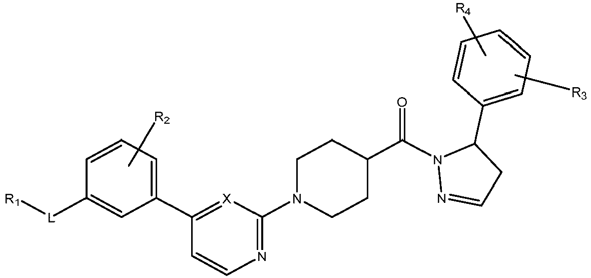 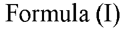 in formula (I),
in formula (I), X is CH or N;
L is O, S, NH, carbonyl, sulfonyl or sulfinyl;
R₁ is C₁-C₁₀ alkyl, C₃-C₈ cycloalkyl, 4- to 8-membered heteroalicyclyl, or C₁-C₁₀ alkyl substituted with 1 to 3 substituents selected from hydroxyl, C₁-C₆ alkoxy, cyano, -NR^{a}R^{b}, C₃-C₈ cycloalkyloxy, -CONH-R₅, C₃-C₈ cycloalkyl, hydroxyl- and/or C₁-C₄ alkyl-substituted C₃-C₈ cycloalkyl, carboxyl, halogen, C₁-C₆ haloalkoxy, -SO₂-R₅, -SO-R₅, -CO-R₅, C₂-C₆ alkynyl, C₂-C₆ alkenyl, C₁-C₄ alkoxyC₁-C₆ alkoxy, 4- to 8-membered heteroalicyclyl, oxo-substituted 4- to 8-membered heteroalicyclyl, hydroxyl- and/or C₁-C₄ alkyl-substituted 4- to 8-membered heteroalicyclyl and C₁-C₆ alkylthio,
the 4- to 8-membered heteroalicyclyl is a 4- to 8-membered heteroalicyclyl containing 1-2 atoms selected from N, O, and S as a ring atom,
R₅ is hydrogen, hydroxyl, C₁-C₆ alkyl, C₁-C₆ alkoxyC₁-C₆ alkyl, hydroxyl-substituted C₁-C₆ alkyl, C₃-C₈ cycloalkyl or 4- to 8-membered heteroalicyclyl-substituted C₁-C₆ alkyl,
R^{a} and R^{b} are each independently hydrogen, C₁-C₆ alkyl, C₃-C₈ cycloalkyl, C₁-C₆ alkoxy-substituted C₁-C₆ alkyl, hydroxyl-substituted C₁-C₆ alkyl, C₃-C₈ cycloalkylC₁-C₆ alkyl, 4- to 8-membered heteroalicyclyl-substituted C₁-C₆ alkyl, C₁-C₃ alkylthio-substituted C₁-C₆ alkyl, mono- or di-C₁-C₃ alkyl-substituted or unsubstituted amino-substituted C₁-C₆ alkyl;
R₂ is hydrogen, C₁-C₃ alkyl, C₁-C₃ alkoxy or halogen;
R₃ and R₄ are each independently hydrogen, halogen or methyl.

In some embodiments, alternatively, the compound has a structure of the following formula (II): 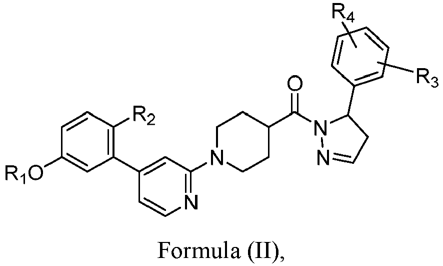 in formula (II),
R₁ is C₁-C₁₀ alkyl, C₃-C₈ cycloalkyl, 4- to 8-membered heteroalicyclyl, or C₁-C₁₀ alkyl substituted with 1 to 3 substituents selected from hydroxyl, C₁-C₆ alkoxy, cyano, -NR^{a}R^{b}, C₃-C₈ cycloalkyloxy, -CONH-R₅, C₃-C₈ cycloalkyl, hydroxyl- and/or C₁-C₄ alkyl-substituted C₃-C₈ cycloalkyl, carboxyl, halogen, C₁-C₆ haloalkoxy, -SO₂-R₅, -SO-Rs, -CO-Rs, C₂-C₆ alkynyl, C₂-C₆ alkenyl, C₁-C₄ alkoxyC₁-C₆ alkoxy, 4- to 8-membered heteroalicyclyl, oxo-substituted 4- to 8-membered heteroalicyclyl, hydroxyl- and/or C₁-C₄ alkyl-substituted 4- to 8-membered heteroalicyclyl and C₁-C₆ alkylthio,
the 4- to 8-membered heteroalicyclyl is a 4- to 8-membered heteroalicyclyl containing 1-2 atoms selected from N, O, and S as a ring atom,
R₅ is hydrogen, hydroxyl, C₁-C₆ alkyl, C₁-C₆ alkoxyC₁-C₆ alkyl, hydroxyl-substituted C₁-C₆ alkyl, C₃-C₈ cycloalkyl or 4- to 8-membered heteroalicyclyl-substituted C₁-C₆ alkyl,
R^{a} and R^{b} are each independently hydrogen, C₁-C₆ alkyl, C₃-C₈ cycloalkyl, C₁-C₆ alkoxy-substituted C₁-C₆ alkyl, hydroxyl-substituted C₁-C₆ alkyl, C₃-C₈ cycloalkylC₁-C₆ alkyl, 4- to 8-membered heteroalicyclyl-substituted C₁-C₆ alkyl, C₁-C₃ alkylthio-substituted C₁-C₆ alkyl, mono- or di-C₁-C₃ alkyl-substituted or unsubstituted amino-substituted C₁-C₆ alkyl;
R₂ is hydrogen, C₁-C₃ alkyl, C₁-C₃ alkoxy or halogen;
R₃ and R₄ are each independently hydrogen, halogen or methyl.

In some embodiments, alternatively, R₁ is C₁-C₈ alkyl, C₃-C₆ cycloalkyl, 4- to 6-membered heteroalicyclyl, or Ci-Cs alkyl substituted with 1 to 3 substituents selected from hydroxyl, C₁-C₃ alkoxy, cyano, C₃-C₆ cycloalkyloxy, C₃-C₆ cycloalkyl, hydroxyl- and/or C₁-C₄ alkyl-substituted C₃-C₆ cycloalkyl, halogen, 4- to 6-membered heteroalicyclyl, oxo-substituted 4- to 6-membered heteroalicyclyl, hydroxyl- and/or C₁-C₄ alkyl-substituted 4- to 6-membered heteroalicyclyl, and C₁-C₃ alkylthio,
the 4- to 6-membered heteroalicyclyl is a 4- to 6-membered heteroalicyclyl containing 1-2 atoms selected from N, O, and S as a ring atom.

In some embodiments, alternatively, R₁ is methyl, ethyl, propyl, isopropyl, butyl, isobutyl, tert-butyl, pentyl, isopentyl, hexyl, octyl, cyclobutyl, cyclopentyl, cyclohexyl, tetrahydrofuran-2-yl, tetrahydrofuran-3-yl, tetrahydropyran-2-yl, tetrahydropyran-3-yl, tetrahydropyran-4-yl, or C₁-C₈ alkyl substituted with 1 to 3 substituents selected from hydroxyl, methoxy, ethoxy, propoxy, isopropoxy, cyano, cyclobutyloxy, cyclopentyloxy, cyclohexyloxy, cyclobutyl, cyclopentyl, cyclohexyl, 4-hydroxylcyclohexyl, 4-hydroxyl-4-methylcyclohexyl, fluorine, chlorine, tetrahydrofuran-2-yl, tetrahydrofuran-3-yl, tetrahydropyran-2-yl, tetrahydropyran-3-yl, tetrahydropyran-4-yl, pyrrolidin-1-yl, pyrrolidin-2-yl, piperidin-1-yl, piperidin-4-yl, morpholinyl, thiomorpholinyl, 1-methyl-pyrrolidin-2-yl, 1-methyl-piperidin-4-yl, methylthio, ethylthio, propylthio, and isopropylthio.

In some embodiments, still alternatively, R₁ is methyl, ethyl, propyl, butyl, pentyl, hexyl, hydroxyethyl, hydroxypropyl, hydroxybutyl, hydroxypentyl, hydroxyhexyl, methoxyethyl, methoxypropyl, methoxybutyl, methoxypentyl, methoxyhexyl, tetrahydropyran-4-yl, 4-methyl-4-hydroxypentyl, tetrahydropyran-4-ylethyl, tetrahydropyran-4-ylmethyl, tetrahydropyran-4-ylpropyl, tetrahydropyran-4-ylbutyl, 3-methyl-3-hydroxylbutyl, 2-methyl-2-hydroxylpropyl, 5-methyl-5-hydroxylhexyl, fluoropropyl, fluoroethyl, 2,2-difluoro-3-hydroxyl-propyl.

In some embodiments, R₁ is Ci-Cs alkyl substituted with 1-hydroxycyclopropyl, 1-hydroxycyclobutyl, 1-hydroxycyclopentyl or 1-hydroxycyclohexyl;

Still alternatively, R₁ is 1-hydroxycyclopropylmethyl or 1-hydroxycyclobutylmethyl.

In some embodiments, still alternatively, R₁ is hydroxyl- and/or halogen-substituted C₁-C₆ alkyl; yet alternatively, R₁ is hydroxyethyl, hydroxypropyl, hydroxybutyl, hydroxypentyl, hydroxyhexyl, 2-methyl-2-hydroxypropyl, 3-methyl-3-hydroxybutyl, 4- methyl-4-hydroxypentyl, 5-methyl-5-hydroxyhexyl, fluoropropyl, fluoroethyl, or 2,2-difluoro-3-hydroxy-propyl.

In some embodiments, alternatively, R₂ is hydrogen, methyl, methoxy, fluorine, chlorine, or bromine;
Still alternatively, R₂ is fluorine.

In some embodiments, alternatively, R₃ and R₄ are each independently hydrogen, fluorine, chlorine, or methyl;
Still alternatively, R₃ and R₄ are each independently fluorine.

In some embodiments, alternatively, the compound has a structure of the following formula (III): 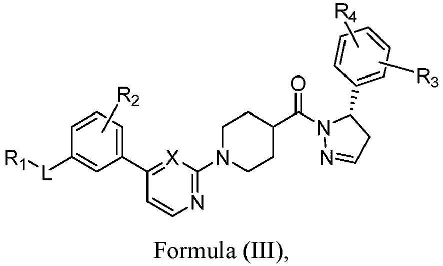
wherein X is CH or N;
L is O, S, NH, carbonyl, sulfonyl or sulfinyl;
R₁ is C₁-C₁₀ alkyl, C₃-C₈ cycloalkyl, 4- to 8-membered heteroalicyclyl, or C₁-C₁₀ alkyl substituted with 1 to 3 substituents selected from hydroxyl, C₁-C₆ alkoxy, cyano, -NR^{a}R^{b}, C₃-C₈ cycloalkyloxy, -CONH-R₅, C₃-C₈ cycloalkyl, hydroxyl- and/or C₁-C₄ alkyl-substituted C₃-C₈ cycloalkyl, carboxyl, halogen, C₁-C₆ haloalkoxy, -SO₂-R₅, -SO-Rs, -CO-Rs, C₂-C₆ alkynyl, C₂-C₆ alkenyl, C₁-C₄ alkoxyC₁-C₆ alkoxy, 4- to 8-membered heteroalicyclyl, oxo-substituted 4- to 8-membered heteroalicyclyl, hydroxyl- and/or C₁-C₄ alkyl-substituted 4- to 8-membered heteroalicyclyl and C₁-C₆ alkylthio,
the 4- to 8-membered heteroalicyclyl is a 4- to 8-membered heteroalicyclyl containing 1-2 atoms selected from N, O, and S as a ring atom,
R₅ is hydrogen, hydroxyl, C₁-C₆ alkyl, C₁-C₆ alkoxyC₁-C₆ alkyl, hydroxyl-substituted C₁-C₆ alkyl, C₃-C₈ cycloalkyl or 4- to 8-membered heteroalicyclyl-substituted C₁-C₆ alkyl,
R^{a} and R^{b} are each independently hydrogen, C₁-C₆ alkyl, C₃-C₈ cycloalkyl, C₁-C₆ alkoxy-substituted C₁-C₆ alkyl, hydroxyl-substituted C₁-C₆ alkyl, C₃-C₈ cycloalkylC₁-C₆ alkyl, 4- to 8-membered heteroalicyclyl-substituted C₁-C₆ alkyl, C₁-C₃ alkylthio-substituted C₁-C₆ alkyl or mono- or di-C₁-C₃ alkyl-substituted or unsubstituted amino-substituted C₁-C₆ alkyl;
R₂ is hydrogen, C₁-C₃ alkyl, C₁-C₃ alkoxy or halogen;
R₃ and R₄ are each independently hydrogen, halogen or methyl.

In some embodiments, alternatively, the compound has a structure of the following formula (IV): 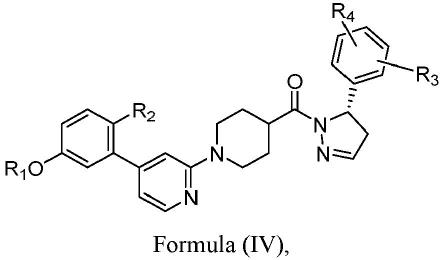
wherein R₁ is C₁-C₁₀ alkyl, C₃-C₈ cycloalkyl, 4- to 8-membered heteroalicyclyl, or C₁-C₁₀ alkyl substituted with 1 to 3 substituents selected from hydroxyl, C₁-C₆ alkoxy, cyano, -NR^{a}R^{b}, C₃-C₈ cycloalkyloxy, -CONH-R₅, C₃-C₈ cycloalkyl, hydroxyl- and/or C₁-C₄ alkyl-substituted C₃-C₈ cycloalkyl, carboxyl, halogen, C₁-C₆ haloalkoxy, -SO₂-R₅, -SO-Rs, -CO-Rs, C₂-C₆ alkynyl, C₂-C₆ alkenyl, C₁-C₄ alkoxyC₁-C₆ alkoxy, 4- to 8-membered heteroalicyclyl, oxo-substituted 4- to 8-membered heteroalicyclyl, hydroxyl- and/or C₁-C₄ alkyl-substituted 4- to 8-membered heteroalicyclyl and C₁-C₆ alkylthio,
the 4- to 8-membered heteroalicyclyl is a 4- to 8-membered heteroalicyclyl containing 1-2 atoms selected from N, O, and S as a ring atom,
R₅ is hydrogen, hydroxyl, C₁-C₆ alkyl, C₁-C₆ alkoxyC₁-C₆ alkyl, hydroxyl-substituted C₁-C₆ alkyl, C₃-C₈ cycloalkyl or 4- to 8-membered heteroalicyclyl-substituted C₁-C₆ alkyl,
R^{a} and R^{b} are each independently hydrogen, C₁-C₆ alkyl, C₃-C₈ cycloalkyl, C₁-C₆ alkoxy-substituted C₁-C₆ alkyl, hydroxyl-substituted C₁-C₆ alkyl, C₃-C₈ cycloalkylC₁-C₆ alkyl, 4- to 8-membered heteroalicyclyl-substituted C₁-C₆ alkyl, C₁-C₃ alkylthio-substituted C₁-C₆ alkyl or mono- or di-C₁-C₃ alkyl-substituted or unsubstituted amino-substituted C₁-C₆ alkyl;
R₂ is hydrogen, C₁-C₃ alkyl, C₁-C₃ alkoxy or halogen;
R₃ and R₄ are each independently hydrogen, halogen or methyl.

In some embodiments, alternatively, R₁ is Ci-Cs alkyl, C₃-C₆ cycloalkyl, 4- to 6-membered heteroalicyclyl, or Ci-Cs alkyl substituted with 1 to 3 substituents selected from hydroxyl, C₁-C₃ alkoxy, cyano, C₃-C₆ cycloalkyloxy, C₃-C₆ cycloalkyl, hydroxyl- and/or C₁-C₄ alkyl-substituted C₃-C₆ cycloalkyl, halogen, 4- to 6-membered heteroalicyclyl, oxo-substituted 4- to 6-membered heteroalicyclyl, hydroxyl- and/or C₁-C₄ alkyl-substituted 4- to 6-membered heteroalicyclyl, and C₁-C₃ alkylthio,
the 4- to 6-membered heteroalicyclyl is a 4- to 6-membered heteroalicyclyl containing 1-2 atoms selected from N, O, and S as a ring atom.

In some embodiments, alternatively, R₁ is methyl, ethyl, propyl, isopropyl, butyl, isobutyl, tert-butyl, pentyl, isopentyl, hexyl, octyl, cyclobutyl, cyclopentyl, cyclohexyl, tetrahydrofuran-2-yl, tetrahydrofuran-3-yl, tetrahydropyran-2-yl, tetrahydropyran-3-yl, tetrahydropyran-4-yl, or C₁-C₈ alkyl substituted with 1 to 3 substituents selected from hydroxyl, methoxy, ethoxy, propoxy, isopropoxy, cyano, cyclobutyloxy, cyclopentyloxy, cyclohexyloxy, cyclobutyl, cyclopentyl, cyclohexyl, 4-hydroxylcyclohexyl, 4-hydroxyl-4-methylcyclohexyl, fluorine, chlorine, tetrahydrofuran-2-yl, tetrahydrofuran-3-yl, tetrahydropyran-2-yl, tetrahydropyran-3-yl, tetrahydropyran-4-yl, pyrrolidin-1-yl, pyrrolidin-2-yl, piperidin-1-yl, piperidin-4-yl, morpholinyl, thiomorpholinyl, 1-methyl-pyrrolidin-2-yl, 1-methyl-piperidin-4-yl, methylthio, ethylthio, propylthio, and isopropylthio.

In some embodiments, still alternatively, R₁ is methyl, ethyl, propyl, butyl, pentyl, hexyl, hydroxyethyl, hydroxypropyl, hydroxybutyl, hydroxypentyl, hydroxyhexyl, methoxyethyl, methoxypropyl, methoxybutyl, methoxypentyl, methoxyhexyl, tetrahydropyran-4-yl, 4-methyl-4-hydroxypentyl, tetrahydropyran-4-ylethyl, tetrahydropyran-4-ylmethyl, tetrahydropyran-4-ylpropyl, tetrahydropyran-4-ylbutyl, 3-methyl-3-hydroxylbutyl, 2-methyl-2-hydroxylpropyl, 5-methyl-5-hydroxylhexyl, fluoropropyl, fluoroethyl, or 2,2-difluoro-3-hydroxyl-propyl.

In some embodiments, R₁ is Ci-Cs alkyl substituted with 1-hydroxycyclopropyl, 1-hydroxycyclobutyl, 1-hydroxycyclopentyl or 1-hydroxycyclohexyl;

Still alternatively, R₁ is 1-hydroxycyclopropylmethyl or 1-hydroxycyclobutylmethyl.

In some embodiments, still alternatively, R₁ is hydroxyl- and/or halogen-substituted C₁-C₆ alkyl; yet alternatively, R₁ is hydroxyethyl, hydroxypropyl, hydroxybutyl, hydroxypentyl, hydroxyhexyl, 2-methyl-2-hydroxypropyl, 3-methyl-3-hydroxybutyl, 4- methyl-4-hydroxypentyl, 5-methyl-5-hydroxyhexyl, fluoropropyl, fluoroethyl, or 2,2-difluoro-3-hydroxy-propyl.

In some embodiments, alternatively, R₂ is hydrogen, methyl, methoxy, fluorine, chlorine, or bromine;
Still alternatively, R₂ is fluorine.

In some embodiments, alternatively, R₃ and R₄ are each independently hydrogen, fluorine, chlorine, or methyl;
Still alternatively, R₃ and R₄ are each independently fluorine.

In some embodiments, the compound of the present application is a deuterated compound of the aforementioned compound, still alternatively, a compound deuterated at the 5-position of the pyrazolyl in any one of the aforementioned structural formulas (I) to (IV).

Typical compounds of this application are shown below:

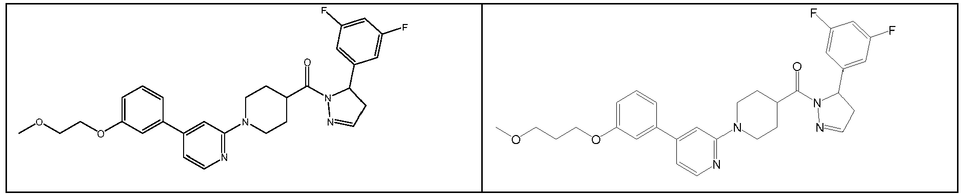

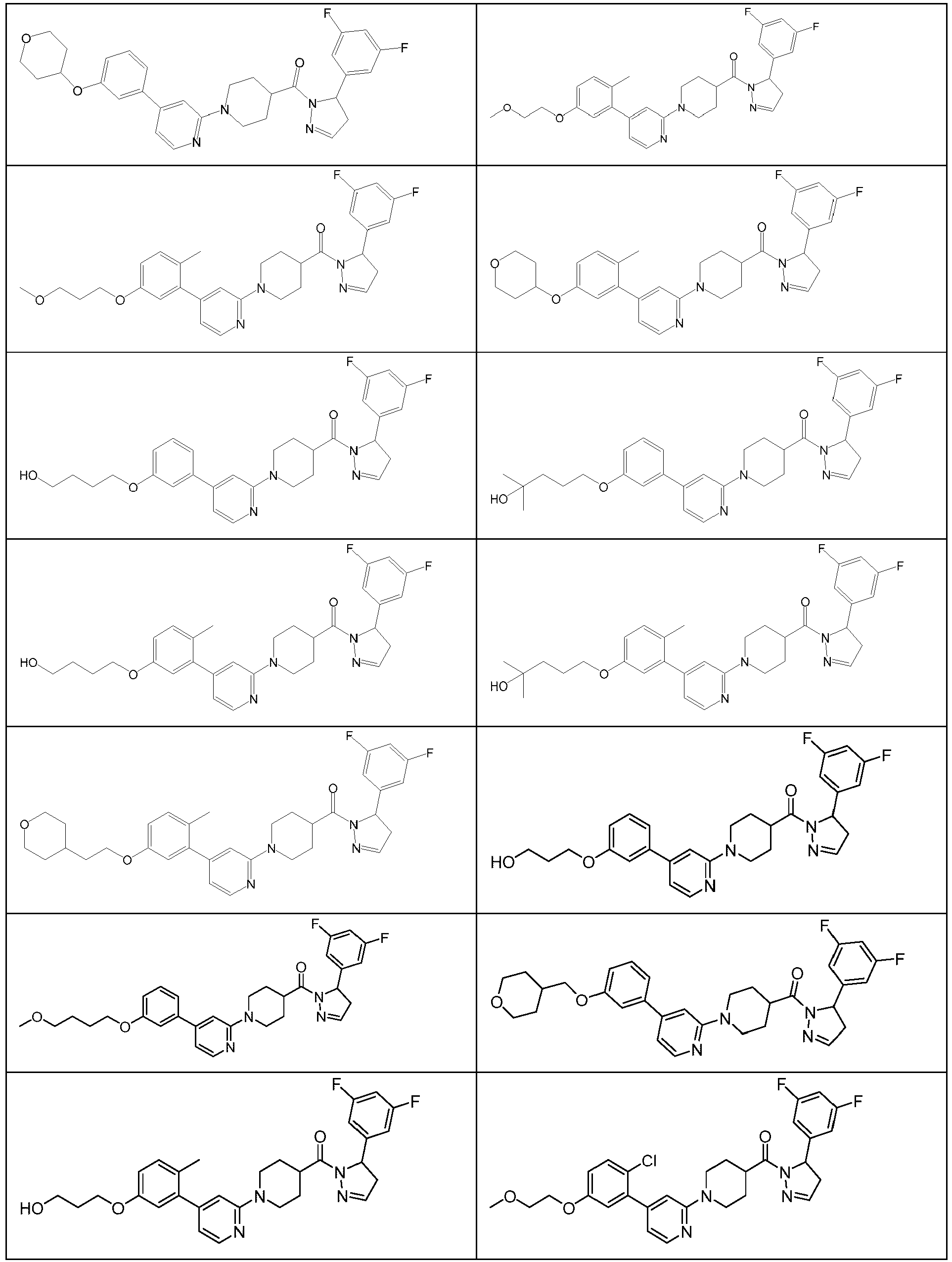

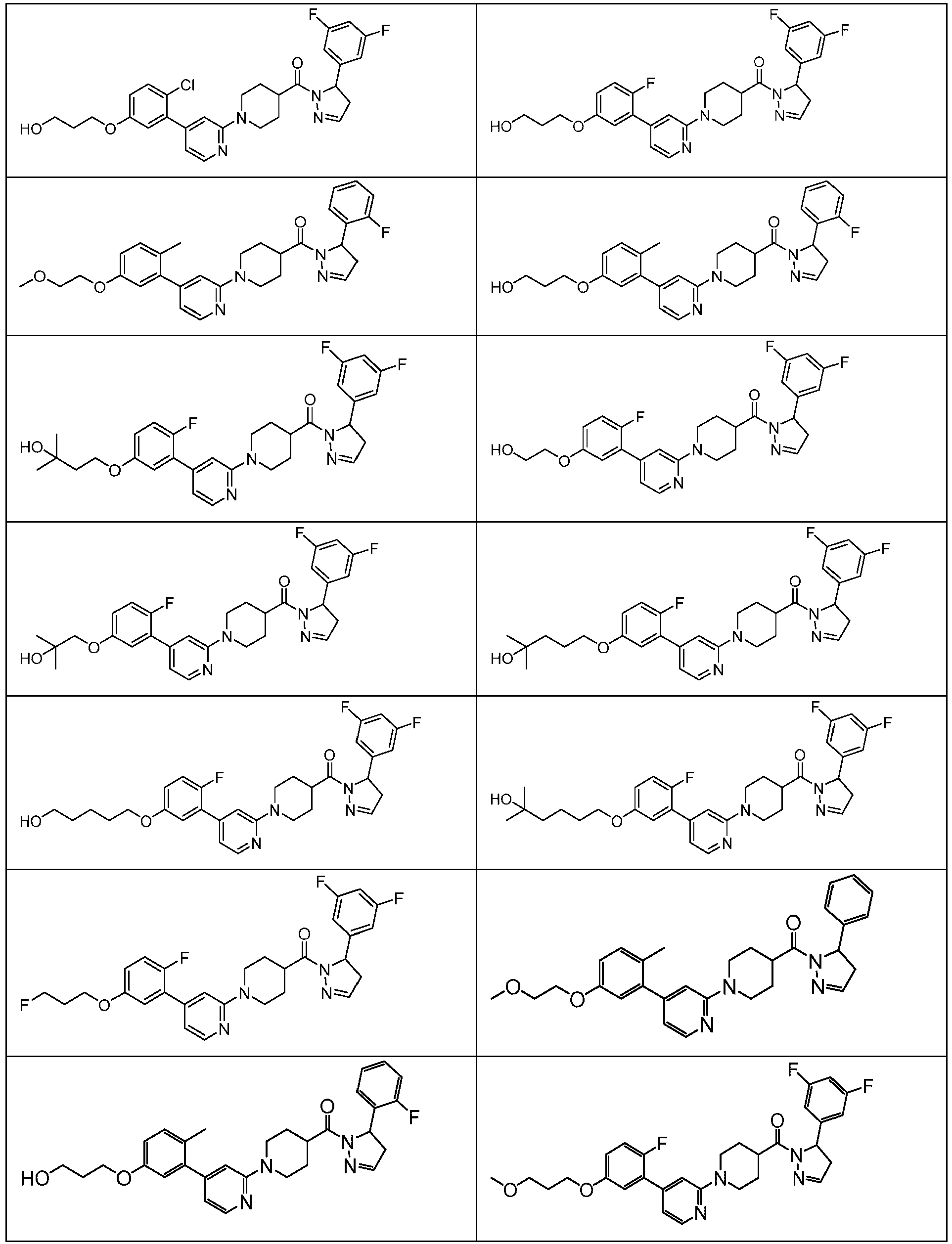

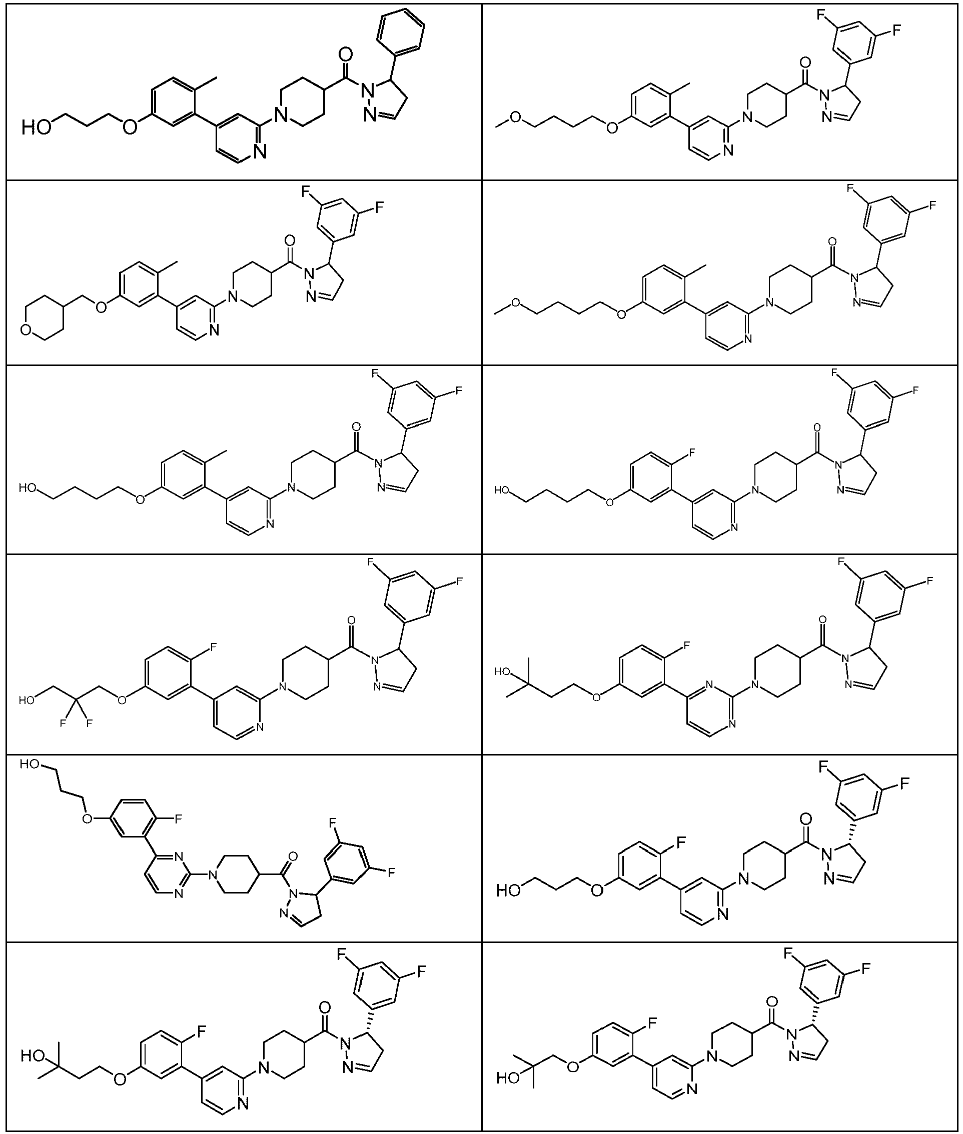

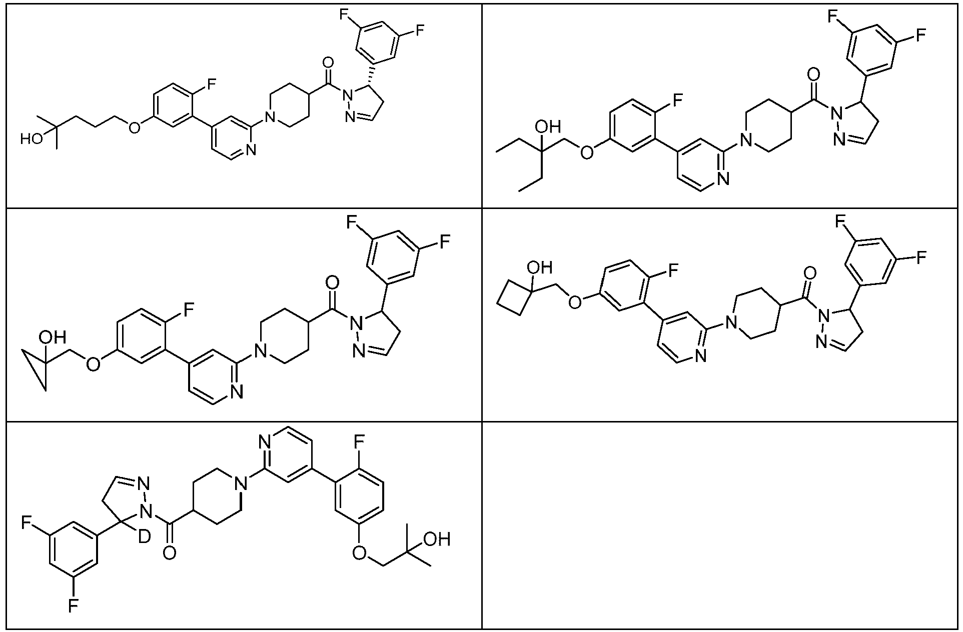

According to some embodiments of the present disclosure, the pharmaceutically acceptable salt of the compound is selected from one or more of hydrochloride, hydrobromide, hydroiodide, perchlorate, sulfate, nitrate, phosphate, formate, acetate, propionate, glycolate, lactate, succinate, maleate, tartrate, malate, citrate, fumarate, gluconate, benzoate, mandelate, methanesulfonate, isethionate, benzenesulfonate, oxalate, palmitate, 2-naphthalenesulfonate, p-toluenesulfonate, cyclohexylsulfamate, salicylate, hexonate, trifluoroacetate, aluminum salt, calcium salt, chloroprocaine salt, choline salt, diethanolamine salt, ethylenediamine salt, lithium salt, magnesium salt, potassium salt, sodium salt and zinc salt.

Another aspect of the present disclosure relates to use of the compound, or a pharmaceutically acceptable salt, stereoisomer, solvate, or deuterated derivative thereof in the manufacture of a medicament for the treatment of an RIPK1-related disease, wherein the RIPK1-related disease includes ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic granulocytic leukemia, acute myelocytic leukemia, non-Hodgkin's lymphoma, nasopharyngeal cancer, esophageal cancer, brain tumor, B-cell and T-cell lymphoma, lymphoma, multiple myeloma, biliary tract carcinosarcoma, cholangiocarcinoma, inflammatory bowel disease, ulcerative colitis, retinal detachment, retinitis pigmentosa, macular degeneration, pancreatitis, atopic dermatitis, spondyloarthritis, gout, SoJIA, Sjogren's syndrome, systemic scleroderma, antiphospholipid syndrome, vasculitis, osteoarthritis, non-alcoholic steatohepatitis, alcoholic steatohepatitis, autoimmune hepatitis, autoimmune hepatobiliary disease, primary sclerosing cholangitis, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic diseases, asthma, atopic dermatitis, multiple sclerosis, type I diabetes, Wegener's granulomatosis, pulmonary sarcoidosis, Behcet's disease, interleukin-1 converzyme-related fever syndrome, chronic obstructive pulmonary disease, tumor necrosis factor receptor related periodic syndrome and periodontitis.

Another aspect of the present disclosure provides a pharmaceutical composition, which includes the amide-bridged heterocyclic compounds of the present disclosure, or a stereoisomer, solvate, pharmaceutically acceptable salt or deuterated derivative thereof, and one or more pharmaceutically acceptable carriers or excipients.

According to some embodiments of the present application, the pharmaceutical composition may also include one or more other therapeutic agents.

The present disclosure also relates to a method for treating diseases or disorders mediated by RIPK1 kinase, which comprises administering a therapeutically effective amount of a compound of formula (I) or a salt thereof to a patient (human or other mammals, especially human) in need thereof. The diseases or disorders mediated by RIPK1 kinase include those mentioned above.

### BRIEF DESCRIPTION OF DRAWINGS

Fig. 1 shows a Western Blot picture of the inhibition of RIPK1 phosphorylation by the compound of Example 40;
Fig. 2 shows the ratio of RIPK1 phosphorylation relative to the total RIPK1 protein expression in the presence of the compound of Example 40.

### DETAILED DESCRIPTION

Unless otherwise stated, the following terms used in this application (including the specification and claims) have the definitions given below. In this application, the use of "or" or "and" means "and/or" unless stated otherwise. In addition, the use of the term "comprising" and other forms such as "including", "containing" and "having" is not limiting. The chapter headings used herein are for organizational purposes only and should not be interpreted as limitations on the topics described.

Unless otherwise specified, an alkyl group refers to a saturated linear and branched hydrocarbon group having a specified number of carbon atoms, and the term C₁-C₁₀ alkyl refers to an alkyl moiety containing from 1 to 10 carbon atoms. Similarly, C₁-C₃ alkyl refers to an alkyl moiety containing from 1 to 3 carbon atoms. For example, C₁-C₆ alkyl includes methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl and 2-methylpentyl, etc.

When a substituent term such as "alkyl" is used in combination with other substituent term, such as in terms "C₁-C₃ alkoxy C₁-C₆ alkylthio" or "hydroxy-substituted C₁-C₁₀ alkyl", the linking substituent terms (e.g., alkyl or alkylthio) are intended to encompass a divalent moiety, wherein the point of attachment is through the linking substituent. Examples of "C₁-C₃ alkoxy C₁-C₆ alkylthio" include, but are not limited to, methoxymethylthio, methoxyethylthio and ethoxypropylthio, etc. Examples of "hydroxy-substituted C₁-C₁₀ alkyl" include, but are not limited to, hydroxymethyl, hydroxyethyl, and hydroxyisopropyl, etc.

An alkoxy group is an alkyl-O- group formed by a linear or branched alkyl group described previously and -O-, e.g., methoxy, ethoxy, and the like. Similarly, an alkylthio group is an alkylS- group formed by a linear or branched alkyl group as described previously and -S-, e.g., methylthio, ethylthio, and the like.

Alkenyl and alkynyl groups include linear or branched alkenyl or alkynyl groups, and the term C₂-C₆ alkenyl or C₂-C₆ alkynyl refers to linear or branched hydrocarbon groups having at least one alkenyl or alkynyl group.

The term "C₁-C₁₀ haloalkyl" refers to a group having one or more halogen atoms, which may be the same or different, on one or more carbon atoms of an alkyl moiety comprising 1 to 10 carbon atoms. Examples of "C₁-C₁₀ haloalkyl" may include, but are not limited to, - CF₃ (trifluoromethyl), -CCl₃ (trichloromethyl), 1,1-difluoroethyl, 2,2,2-trifluoroethyl and hexafluoroisopropyl, etc. Similarly, the term "C₁-C₁₀ haloalkoxy" refers to a haloalkyl-O- group formed by the C₁-C₁₀ haloalkyl and -O-, which can be, for example, trifluoromethoxy, trichloromethoxy, etc.

The term "C₁-C₃ acyl" includes formyl (-CHO), acetyl (CH₃CO-), and acetyl (C₂H₅CO-).

The terms "-CO-Rs, -SO₂-R₅, -SO-Rs, -CONH-Rs" represent 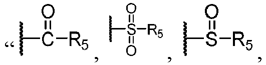 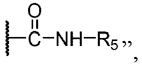 respectively.

The term "cycloalkyl" refers to a non-aromatic, saturated, cyclic hydrocarbon group containing a specified number of carbon atoms. For example, the term "(C₃-C₆)cycloalkyl" refers to a non-aromatic cyclic hydrocarbon ring having 3-6 ring carbon atoms. Exemplary "(C₃-C₆)cycloalkyl" includes cyclopropyl, cyclobutyl, cyclopentyl, and cyclohexyl.

The term "aryl" refers to a group or moiety comprising an aromatic monocyclic or bicyclic hydrocarbon radical, which contains from 6 to 12 carbon ring atoms and has at least one aromatic ring. Examples of "aryl" are phenyl, naphthyl, indenyl and dihydroindenyl (indanyl). Generally, in the compounds of the present disclosure, the aryl is phenyl.

Unless otherwise specified, the term "heteroalicyclyl" as used herein refers to an unsubstituted or substituted stable 4- to 8-membered non-aromatic monocyclic saturated ring system consisting of carbon atoms and 1 to 3 heteroatoms selected from N, O, and S, wherein the N or S heteroatom can be randomly oxidized, and the N heteroatom can also be randomly quaternized. Examples of such heterocycles include, but are not limited to, azetidinyl, oxetanyl, thietanyl, pyrrolidinyl, pyrrolinyl, pyrazolidinyl, pyrazolinyl, imidazolidinyl, imidazolinyl, oxazolinyl, thiazolinyl, tetrahydrofuranyl, dihydrofuranyl, tetrahydrothienyl, 1,3-dioxolanyl, piperidinyl, piperazinyl, tetrahydropyranyl, dihydropyranyl, tetrahydrothiopyranyl, 1,3-dioxanyl, 1,4-dioxanyl, 1,3-oxathiolanyl, 1,3-oxathianyl, 1,3-dithianyl, 1,4-oxathiolanyl, 1,4-oxathianyl, 1,4-dithianyl, morpholinyl, and thiomorpholinyl.

The term "heteroaryl" as used herein represents a group or moiety containing an aromatic monocyclic or bicyclic radical (which contains 5 to 10 ring atoms), which includes 1 to 3 heteroatoms independently selected from nitrogen, oxygen and sulfur. The term also includes bicyclic heterocyclic aryl, which contains an aryl ring moiety fused to a heterocycloalkyl ring moiety, or a heteroaryl ring moiety fused to a cycloalkyl ring moiety. Unless otherwise specified, the term "heteroaryl" represents an unsubstituted or substituted stable 5- or 6-membered monocyclic aromatic ring system, and can also represents an unsubstituted or substituted benzo-fused heteroaromatic ring system or bicyclic heteroaromatic ring system with 9 or 10 ring atoms, which is composed of carbon atoms and 1 to 3 heteroatoms selected from N, O, and S, where N and S heteroatoms can be oxidized, and N heteroatom can also be quaternized. Heteroaryl groups can be connected to any heteroatom or carbon atom to form a stable structure. Illustrative examples of heteroaryl groups include, but are not limited to, furanyl, thienyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridyl, oxo-pyridyl (pyridyl-N-oxide), pyridazinyl, pyrazinyl, pyrimidinyl, triazinyl, benzofuranyl, isobenzofuranyl, 2,3-dihydrobenzofuranyl, 1,3-benzodioxolyl, dihydrobenzodioxinyl, benzothienyl, indazinyl, indolyl, isoindolyl, indolinyl, benzimidazolyl, dihydrobenzimidazolyl, benzoxazolyl, dihydrobenzoxazolyl, benzothiazolyl, benzoisothiazolyl, dihydrobenzisothiazolyl, indazolyl, imidazopyridyl, pyrazolopyridyl, benzotriazolyl, triazolopyridyl, purinyl, quinolinyl, tetrahydroquinolinyl, isoquinolinyl, tetrahydroisoquinolinyl, quinoxalinyl, cinnolinyl, phthalazinyl, quinazolinyl, 1,5-naphthyridinyl, 1,6-naphthyridinyl, 1,7-naphthyridinyl, 1,8-naphthyridinyl, and pteridyl.

The term "carbonyl" refers to a -C(O)- group. The terms "halogen" and "halo" refer to chlorine, fluorine, bromine or iodine substituent. "Oxo" refers to an oxygen moiety with double bond; for example, "oxo" may be directly attached to a carbon atom to form a carbonyl moiety (C=O). "Hydroxy" is intended to refer to a radical -OH. The term "cyano" as used herein refers to a group -CN.

The term "each independently" means that when more than one substituent is selected from a number of possible substituents, those substituents may be the same or different.

It is clear that the compound of formula I, or an isomer, crystalline form or prodrug thereof, and a pharmaceutically acceptable salt thereof, may exist in a solvated or non-solvated form. For example, the solvated form can be a water-soluble form. The present disclosure includes all the solvated and non-solvated forms.

The compounds of the present disclosure may have asymmetric carbon atoms. According to physical and chemical differences, a diastereomeric mixture can be separated into single diastereomers by known, technically mature methods, such as chromatography or fractional crystallization. The separation of enantiomers can be carried out by first reacting with a suitable, optically active compound to convert an enantiomeric mixture into a diastereomeric mixture, separating the diastereoisomers, and then converting (hydrolyzing) the single diastereomers into the corresponding pure enantiomers. All such isomers, including diastereomeric mixtures and pure enantiomers, are considered as part of the present disclosure.

The compound of the present disclosure as an active ingredient, and the method of preparing the same, are both included in the present disclosure. Moreover, the crystalline form of some of the compounds may exist as polymorphs, and such forms may also be included in the present disclosure. Additionally, some of the compounds may form solvates with water (i.e., hydrates) or common organic solvents, and such solvates are also included within the scope of the disclosure.

The compounds of the disclosure may be used in the free form for treatment or, when appropriate, in the form of a pharmaceutically acceptable salt or other derivative for treatment. As used herein, the term "pharmaceutically acceptable salt" refers to organic and inorganic salts of the compounds of the present disclosure which are suitable for use in human and lower animals without undue toxicity, irritation, allergic response, etc., and have reasonable benefit/risk ratio. Pharmaceutically acceptable salts of amines, carboxylic acids, phosphonates, and other types of compounds are well known in the art. The salt can be formed by reacting a compound of the disclosure with a suitable free base or acid, including, but not limited to, inorganic acids such as hydrochloric acid, hydrobromic acid, phosphoric acid, sulfuric acid, perchloric acid or organic acids such as acetic acid, oxalic acid, maleic acid, tartaric acid, citric acid, succinic acid, malonic acid. Or the salts may be obtained by methods well known in the art, such as ion exchange. Other pharmaceutically acceptable salts include adipate, alginate, ascorbate, aspartate, besylate, benzoate, bisulfate, borate, butyrate, camphorate, camphorsulfonate, citrate, digluconate, lauryl sulfate, ethanesulfonate, formate, fumarate, glucoheptonate, glycerol phosphate, glyconate, hemisulfate, hexanoate, hydroiodide, 2-hydroxyethanesulfonate, lactobionate, lactate, laurate, lauryl sulfate, malate, maleate, mesylate, 2-naphthalenesulfonate, nicotinate, nitrate, oleate, palmitate, pamoate, pectate, persulphate, per-3-phenylpropionate, phosphate, picrate, propionate, stearate, sulfate, thiocyanate, p-toluenesulfonate, undecanoate, and the like. Representative alkali or alkaline earth metal salts include salts of sodium, lithium, potassium, calcium, magnesium, and the like. Other pharmaceutically acceptable salts include suitable non-toxic salts of ammonium, quaternary ammonium, and amine cations formed from halides, hydroxides, carboxylates, sulfates, phosphates, nitrates, lower alkyl sulfonates and aryl sulfonates.

Pharmaceutical compositions of this disclosure comprise the compound of formula (I) described herein or a pharmaceutically acceptable salt thereof; an additional active agent selected from a kinase inhibitory agent (small molecule, polypeptide, antibody, etc.), an immunosuppressant, an anticancer agent, an anti-viral agent, anti-inflammatory agent, antifungal agent, antibiotic, and an anti-vascular hyper proliferation compound; and any pharmaceutically acceptable carrier, adjuvant or excipient.

The compounds of the present disclosure may be used alone or in combination with one or more of other compounds of the present disclosure or with one or more of other agents. When administered in combination, the therapeutic agents can be formulated for simultaneous or sequential administration at different times, or the therapeutic agents can be administered as a single composition. By "combination therapy", it refers to the use of a compound of the present disclosure in combination with another agent, in a manner whereby each agent is co-administered simultaneously or administered sequentially, in either case, for the purpose of achieving the optimal effect of drugs. Co-administration includes dosage form for simultaneous delivery, as well as separate dosage forms for each compound. Thus, administration of the compounds of the disclosure can be combined with other therapies known in the art, for example, radiation therapy or cytostatic agents, cytotoxic agents, other anticancer agents, and the like as used in the treatment of cancer, in order to improve the symptoms of cancer. The administration sequence is not limited in the present disclosure. The compounds of the present disclosure may be administered before, simultaneously, or after other anticancer or cytotoxic agents.

To prepare the pharmaceutical ingredient of the present disclosure, one or more compounds of Formula (I) or salts thereof as an active ingredient can be intimately mixed with a pharmaceutical carrier, which is carried out according to conventional pharmaceutical formulation techniques. The carrier can be used in a wide variety of forms depending on the form of preparation which is designed for different modes of administration (for example, oral or parenteral administration). Suitable pharmaceutically acceptable carriers are well known in the art. A description of some of these pharmaceutically acceptable carriers can be found in the Handbook of Pharmaceutical Excipients, published jointly by the American Pharmaceutical Association and the Pharmaceutical Society of Great Britain.

The pharmaceutical composition of the present disclosure may have the following forms, for example, those suitable for oral administration, such as tablets, capsules, pills, powders, sustained release forms, solutions or suspensions; those for parenteral injections such as clear solutions, suspensions, emulsion; or those for topical application such as ointments, creams; or as a suppository for rectal administration. The pharmaceutical ingredients may also be presented in unit dosage form for single administration in a precise dosage. The pharmaceutical ingredient will include a conventional pharmaceutical carrier or excipient and a compound as an active ingredient prepared according to the present disclosure, and may also include other medical or pharmaceutical preparations, carriers, adjuvants, and the like.

Therapeutic compounds can also be administered to mammals other than humans. The drug dosage for a mammal will depend on the species of the animal and its disease condition or its disordered condition. The therapeutic compound can be administered to the animal in the form of a capsule, a bolus, a tablet or liquid. The therapeutic compound can also be introduced into the animal by injection or infusion. These drug forms are prepared in a traditional manner complying with standard veterinary practice. As an alternative, the pharmaceutical synthetic drugs can be fed to the animal in a mixture with the animal feed, so that the concentrated feed additive or premix can be prepared by mixing ordinary animal feed.

It is a further object of the present disclosure to provide a method for treating cancer in a subject in need thereof, comprising administering to the subject a therapeutically effective amount of a composition containing the compound of the present disclosure.

The present disclosure also includes use of the compound of the present disclosure or a pharmaceutically acceptable derivative thereof in the manufacture of a medicament for treating an RIPK1-related disease, including ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic granulocytic leukemia, acute myelocytic leukemia, non-Hodgkin's lymphoma, nasopharyngeal cancer, esophageal cancer, brain tumor, B-cell and T-cell lymphoma, lymphoma, multiple myeloma, biliary tract carcinosarcoma, cholangiocarcinoma, inflammatory bowel disease, ulcerative colitis, retinal detachment, retinitis pigmentosa, macular degeneration, pancreatitis, atopic dermatitis, spondyloarthritis, gout, SoJIA, Sjogren's syndrome, systemic scleroderma, antiphospholipid syndrome, vasculitis, osteoarthritis, non-alcoholic steatohepatitis, alcoholic steatohepatitis, autoimmune hepatitis, autoimmune hepatobiliary disease, primary sclerosing cholangitis, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic diseases, asthma, atopic dermatitis, multiple sclerosis, type I diabetes, Wegener's granulomatosis, pulmonary sarcoidosis, Behcet's disease, interleukin-1 converzyme-related fever syndrome, chronic obstructive pulmonary disease, tumor necrosis factor receptor related periodic syndrome and periodontitis.

The present disclosure also provides methods for preparing the corresponding compounds. Various synthetic methods can be used to prepare the compounds described herein, including the following methods involved in the examples. The compounds of the present disclosure, or pharmaceutically acceptable salts, isomers or hydrates thereof can be synthesized using the methods described below, synthetic methods known in the art of organic chemistry synthesis, or variants of these methods understood by those skilled in the art. Alternative methods include, but are not limited to, the methods described below.

In order to make the objectives, technical solutions and advantages of the present disclosure clearer, the present disclosure will be further described in detail below in conjunction with specific examples. It should be understood that the specific examples described here are only used to explain the present disclosure and are not intended to limit the present disclosure. If no specific technology or conditions are indicated in examples, the technology or conditions described in the literature in the art or the product specification shall be followed. If manufacturers are not indicated for reagents or instruments used, the reagents or instruments are all conventional products that are commercially available. The term "and/or" as used herein includes any and all combinations of one or more related listed items. Examples are provided below to better illustrate the disclosure, and all temperatures refer to °C unless otherwise noted. The names of some compounds in this disclosure are generated by Chemdraw.

### Abbreviations

- HATU -: N,N,N',N'-Tetramethyl-O-(7-azabenzotriazol-1-yl)uronium hexafluorophosphate
- DIEA -: N,N-Diisopropylethylamine
- DMF -: N,N-dimethylformamide
- DMSO -: Dimethyl sulfoxide
- PE -: Petroleum ether (boiling point 60-90°C)
- EA -: Ethyl acetate

### Synthesis of compounds

### Preparation of intermediate A

### Synthesis of Intermediate A-1

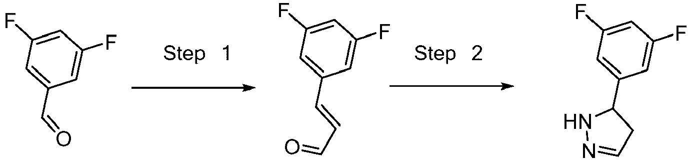

Step 1): 3,5-difluorobenzaldehyde (30 g, 211.1 mmol) was dissolved in DMF, and (triphenylphosphoranylidene)acetaldehyde (CAS No. 2136-75-6) (70.7 g, 232.2 mmol) was added in batches under stirring at room temperature. The mixture was heated to 80 °C for 15 hours. The reaction solution was cooled to room temperature, and extracted with water and ethyl acetate. The organic phase was washed with saturated brine, dried, and concentrated. The residue was purified by column chromatography (eluent: PE/EtOAc = 100:0 to 100:10) to obtain 25.1 g of 3-(3,5-difluorophenyl)propenal, with a yield of 71%, MS:169[M+H]⁺;

Step 2): N₂H₄.H₂O (7.15 g, 223.03 mmol) was added to ethanol (300 mL), and acetic acid (14.5 mL, 252 mmol) was added under stirring. The mixture was heated to 40 °C, and a solution of 3-(3,5-difluorophenyl)propenal (25 g, 149 mmol) in ethanol (20 mL) was slowly added dropwise. The mixture was reacted at 80 °C overnight. The reaction solution was concentrated, and purified by column chromatography (eluent: PE/EtOAc=100:0 to 3:1) to obtain 20 g of the product, with a yield of 74%, MS: 183 [M+H]⁺;

The following intermediates A-2 and A-3 were synthesized by the same method:

**Table 1. Structure, name and characterization data of intermediates A-2 and A-3**

| | Structure | Name | MS ([M+H]⁺) |
|---|---|---|---|
| A-2 | 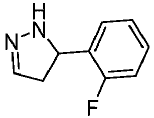 | 5-(2-fluorophenyl)-4,5-dihydro-1H-pyrazole | 165 |
| A-3 | 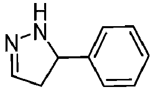 | 5-phenyl-4,5-dihydro-1H-pyrazole | 147 |

### Synthesis of intermediate B-1: 2-(2-fluoro-5-(3-methoxypropoxy)phenyl)-4,4,5,5-tetramethyl-1,3,2-dioxaborolane

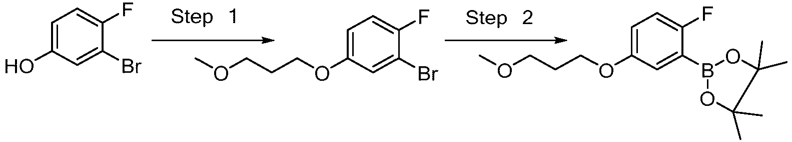

Step 1): 3-bromo-4-fluorophenol (330 mg, 1.73 mmol), potassium carbonate (718.98 mg, 5.20 mmol), and 1-bromo-3-methoxypropane (540 mg, 3.50 mmol) were added in DMF (5 mL). The mixture was reacted at 80 °C for 2 hours. Water was added to the reaction solution, and the mixture was extracted three times with EA. The organic phase was washed with saturated brine, dried over anhydrous sodium sulfate, and filtered. The filtrate was concentrated. The crude product was purified by thin layer chromatography (with PE:EA=5:1 as the mobile phase) to obtain 2-bromo-1-fluoro-(4-methoxypropyl)phenyl ether (421 mg, 93% yield);

Step 2): 2-Bromo-1-fluoro-(4-methoxypropyl)phenyl ether (421 mg, 1.60 mmol), pinacol ester (815 mg, 3.21 mmol), KOAc (472 mg, 4.81 mmol), and Pd(dppf)Cl₂ (235 mg, 321 µmol) were added to 1,4-dioxane (10 mL). The mixture was reacted at 100 °C for 16 hours under argon protection. The reaction solution was diluted with water and extracted with ethyl acetate. The organic phase was washed with saturated brine, dried over anhydrous sodium sulfate, and filtered. The filtrate was concentrated under reduced pressure, and purified by column chromatography (with PE:EA=5: 1 as the mobile phase) to obtain 395 mg of 2-(2-fluoro-5-(3-methoxypropoxy)phenyl)-4,4,5,5-tetramethyl-1,3,2-dioxaborolane, with a yield of 80%.

The following intermediates B-2 to B-30 were synthesized by the same method:

**Table 2. Structures and names of intermediates B-2 to B-30**

| No. | Structure | Name |
|---|---|---|
| B-2 | 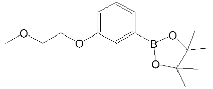 | 2-(3-(2-methoxyethoxy)phenyl)-4,4,5,5-tetramethyl-1,3,2-dioxaborolane |
| B-3 | 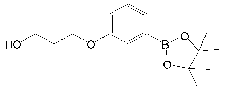 | 3-(3-(4,4,5,5-tetramethyl-1,3,2-dioxaborolane-2-yl)phenoxy)propan-1-ol |
| B-4 | 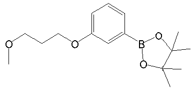 | 2-(3-(3-methoxypropoxy)phenyl)-4,4,5,5-tetramethyl-1,3,2-dioxaborolane |
| B-5 | 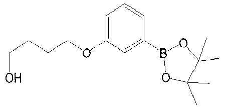 | 4-(3-(4,4,5,5-tetramethyl-1,3,2-dioxaborolane-2-yl)phenoxy)butan-1 -ol |
| B-6 | 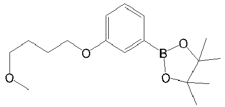 | 2-(3-(4-methoxybutoxy)phenyl)-4,4,5,5-tetramethyl-1,3,2-dioxaborolane |
| B-7 | 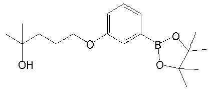 | 2-methyl-5-(3-(4,4,5,5-tetramethyl-1,3,2-dioxaborolane-2-yl)phenoxy)pentan-2-ol |
| B-8 | 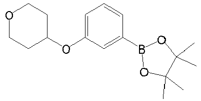 | 4,4,5,5-tetramethyl-2-(3-((tetrahydro-2H-pyran-4-yl)oxy)phenyl)-1,3 ,2-dioxaborolane |
| B-9 | 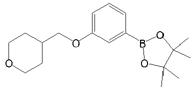 | 4,4,5,5-tetramethyl-2-(3-((tetrahydro-2H-pyran-4-yl)methoxy)phenyl)-1,3 ,2-dioxaborolane |
| B-10 | 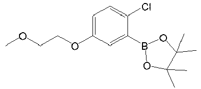 | 2-(2-chloro-5-(2-methoxyethoxy)phenyl)-4,4,5,5-tetramethyl-1,3 ,2-dioxaborolane |
| B-11 | 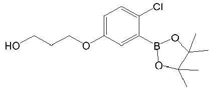 | 3-(4-chloro-3-(4,4,5,5-tetramethyl-1,3,2-dioxaborolane-2-yl)phenoxy)propan-1-ol |
| B-12 | 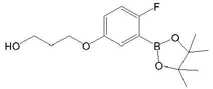 | 3-(4-fluoro-3-(4,4,5,5-tetramethyl-1,3,2-dioxaborolane-2-yl)phenoxy)propan-1-ol |
| B-13 | 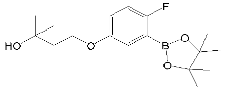 | 4-(4-fluoro-3-(4,4,5,5-tetramethyl-1,3,2-dioxaborolane-2-yl)phenoxy)-2-methylbutan-2-ol |
| B-14 | 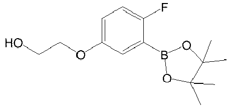 | 2-(4-fluoro-3-(4,4,5,5-tetramethyl-1,3,2-dioxaborolane-2-yl)phenoxy)ethan-1-ol |
| B-15 | 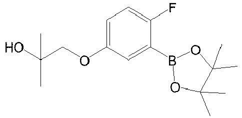 | 1-(4-fluoro-3-(4,4,5,5-tetramethyl-1,3,2-dioxaborolane-2-yl)phenoxy)-2-methylpropan-2-ol |
| B-16 | 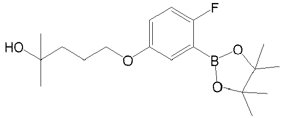 | 5-(4-fluoro-3-(4,4,5,5-tetramethyl-1,3,2-dioxaborolane-2-yl)phenoxy)-2-methylpentan-2-ol |
| B-17 | 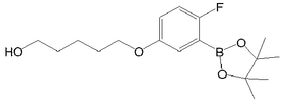 | 5-(4-fluoro-3-(4,4,5,5-tetramethyl-1,3,2-dioxaborolane-2-yl)phenoxy)pentan-1-ol |
| B-18 | 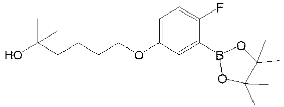 | 6-(4-fluoro-3-(4,4,5,5-tetramethyl-1,3,2-dioxaborolane-2-yl)phenoxy)-2-methylhexan-2-ol |
| B-19 | 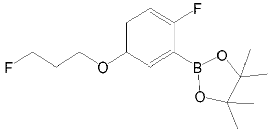 | 2-(2-fluoro-5-(3-fluoropropoxy)phenyl)-4,4,5,5-tetramethyl-1,3,2-dioxaborolane |
| B-20 | 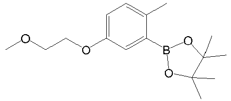 | 2-(5-(2-methoxyethoxy)-2-methylphenyl)-4,4,5,5-tetramethyl-1,3,2-dioxaborolane |
| B-21 | 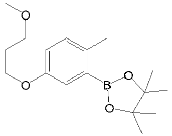 | 2-(5-(3-methoxypropoxy)-2-methylphenyl)-4,4,5,5-tetramethyl-1,3,2-dioxaborolane |
| B-22 | 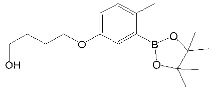 | 4-(4-methyl-3-(4,4,5,5-tetramethyl-1,3,2-dioxaborolane-2-yl)phenoxy)butan-1-ol |
| B-23 | 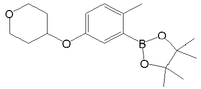 | 4,4,5,5-tetramethyl-2-(2-methyl-5-((tetrahydro-2H-pyran-4-yl)oxy)phenyl)-1,3,2-dioxaborolane |
| B-24 | 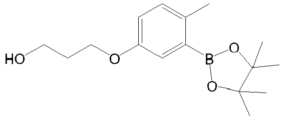 | 3-(4-methyl-3-(4,4,5,5-tetramethyl-1,3,2-dioxaborolane-2-yl)phenoxy)propan-1-ol |
| B-25 | 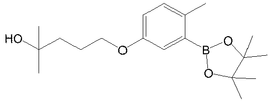 | 2-methyl-5-(4-methyl-3-(4,4,5,5-tetramethyl-1,3,2-dioxaborolane-2-yl)phenoxy)pentan-2-ol |
| B-26 | 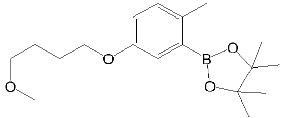 | 2-(5-(4-methoxybutoxy)-2-methylphenyl)-4,4,5,5-tetramethyl-1,3,2-dioxaborolane |
| B-27 | 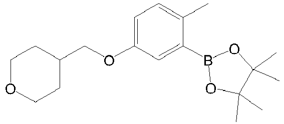 | 4,4,5,5-tetramethyl-2-(2-methyl-5-((tetrahydro-2H-pyran-4-yl)methoxy)phenyl)-1,3,2-dioxaborolane |
| B-28 | 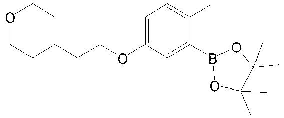 | 4,4,5,5-tetramethyl-2-(2-methyl-5-(2-(tetrahydro-2H-pyran-4-yl)ethoxy)phenyl)-1,3,2-dioxaborolane |
| B-29 | 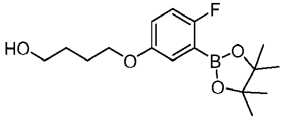 | 4-(4-fluoro-3-(4,4,5,5-tetramethyl-1,3,2-dioxaborolane-2-yl)phenoxy)butan-1-ol |
| B-30 | 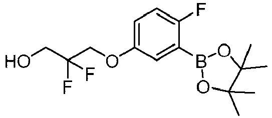 | 2,2-difluoro-3-(4-fluoro-3-(4,4,5,5-tetramethyl-1,3,2-dioxaborolane-2-yl)phenoxy)propan-1-ol |

### Example

### Example 1: (5-(3,5-difluorophenyl)-4,5-dihydro-1H-pyrazol-1-yl)(1-(4-(3-(2-methoxyethoxy)phenyl)pyridin-2-yl)piperidin-4-yl)methanone

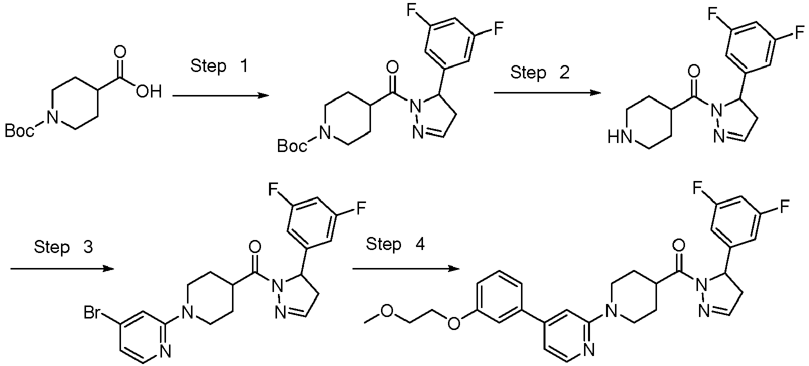

Step 1): 1-(tert-butoxycarbonyl)piperidin-4-carboxylic acid (24g, 105mmol), intermediate A-1 (5-(3,5-difluorophenyl)-4,5-dihydro-1H-pyrazole) (21.9g, 120 mmol), HATU(39.9 g, 105 mmol) and DIEA (27.1 g, 210 mmol) were added to DMF (150 mL). The mixture was reacted at 25 °C for 1 hour under argon protection. The reaction solution was diluted with dichloromethane, washed with saturated brine, dried over anhydrous sodium sulfate, and filtered. The filtrate was concentrated under reduced pressure. The obtained product was further purified by column chromatography to finally obtain 33.0 g, with a yield of 80%. MS:394[M+H]⁺;

Step 2): tert-butyl 4-[3-(3,5-difluorophenyl)-3,4-dihydropyrazole-2-carbonyl]piperidin-1-carboxylate (26 g, 66.09 mmol) was dissolved in 1,4-dioxane (60 mL), and then hydrochloric acid solution (4 M solution system in 1,4-dioxane, 82.61 mL) was added. The mixture was stirred at room temperature overnight. After the reaction was completed, the mixture was filtered by suction. The solid was dissolved in methanol, and then sodium carbonate solid was added. The mixture was adjusted to pH 8, stirred for 30 minutes, and then diluted with ethyl acetate. The mixture was filtered by suction, and the solid was washed twice with ethyl acetate. The filtrate was rotary evaporated to dryness and purified by column chromatography (dichloromethane/methanol/triethylamine = 500/100/5) to obtain 15.5 g, with a yield of 80%. MS: 294[M+H]⁺;

Step 3): A solution of (5-(3,5-difluorophenyl)-4,5-dihydro-1H-pyrazol-1-yl)(piperidin-4-yl)methanone (3.0 g, 10 mmol), 2-fluoro-4-bromopyridine (3.5 g, 20 mmol) and triethylamine (3.05 g, 30 mmol) in DMSO (20 ml) was heated to 80 °C and reacted for 16 hours. The mixture was cooled, and diluted with ethyl acetate. The mixture was washed with water, dried, and concentrated. The residue was purified by column chromatography to obtain 3.93 g of a yellow solid product, with a yield of 88%, MS: 449, 451[M+H]⁺;

Step 4): (1-(4-bromopyridin-2-yl)piperidin-4-yl)(5-(3,5-difluorophenyl)-4,5-dihydro-1H-pyrazol-1-yl)methanone (450mg, 1mmol), intermediate B-2 (2-(3-(2-methoxyethoxy)phenyl)-4,4,5,5-tetramethyl-1,3,2-dioxaborolane)(250mg, 1mmol), potassium carbonate (210mg, 2mmol) and Pd(PPh₃)₄ (58mg, 0.05mmol) were dissolved in a mixed solvent of 1,4-dioxane (8 mL) and water (0.8 mL). The mixture was heated to 80 °C under argon protection and stirred to react for 5 hours. The reaction solution was filtered, concentrated, and purified by column chromatography (mobile phase: PE/EtOAc) to finally obtain 130 mg of a yellow solid product. ¹H NMR (400 MHz, DMSO-*d*₆) δ 8.14 (d, *J* = 5.2 Hz, 1H), 7.38 (t, *J* = 7.9 Hz, 1H), 7.33 - 7.22 (m, 3H), 7.12 - 7.10 (m, 1H), 7.05 (s, 1H), 7.01 (d, *J* = 8.1 Hz, 1H), 6.93 - 6.80 (m, 3H), 5.34 (dd, *J* = 11.9, 4.9 Hz, 1H), 4.50 - 4.41 (m, 2H), 4.23 - 4.14 (m, 2H), 3.71 -3.68 (m, 2H), 3.57 - 3.42 (m, 2H), 3.32 (s, 3H), 3.00 - 2.87 (m, 2H), 2.74 (dd, *J* = 19.0, 5.0 Hz, 1H), 1.90 (d, *J* = 12.8 Hz, 1H), 1.76 (d, *J* = 13.0 Hz, 1H), 1.58 - 1.54 (m, 2H). MS: 521[M+H]⁺.

### Example 2: (5-(3,5-difluorophenyl)-4,5-dihydro-1H-pyrazol-1-yl)(1-(4-(3-(3-methoxypropoxy)phenyl)pyridin-2-yl)piperidin-4 -yl)methanone

The preparation was carried out in a similar manner to Example 1, except that in step 4, intermediate B-4 was used instead of intermediate B-2 for reaction. ¹H NMR (400 MHz, DMSO-*d*₆) δ 8.14 (d, *J* = 5.2 Hz, 1H), 7.38 (t, *J* = 7.9 Hz, 1H), 7.27 (d, *J* = 19.0 Hz, 3H), 7.11 (t, *J* = 9.4 Hz, 1H), 7.06 - 6.96 (m, 2H), 6.92 - 6.80 (m, 3H), 5.34 (dd, *J* = 12.0, 5.0 Hz, 1H), 4.45 (d, *J =* 13.0 Hz, 2H), 4.10 (t, *J* = 6.4 Hz, 2H), 3.50 - 3.48 (m, 4H), 3.26 (s, 3H), 2.92 (dd, *J* = 12.7, 9.5 Hz, 2H), 2.74 (dd, *J* = 19.0, 5.1 Hz, 1H), 2.03 - 1.86 (m, 3H), 1.76 (d, *J* = 12.7 Hz, 1H), 1.65 - 1.48 (m, 2H). MS:535[M+H]⁺.

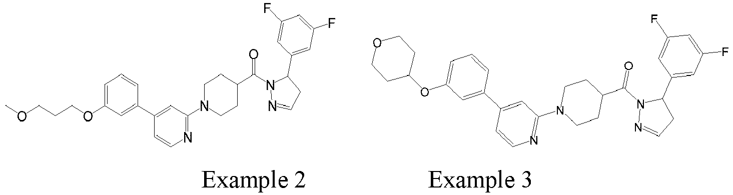

### Example 3: (5-(3,5-difluorophenyl)-4,5-dihydro-1H-pyrazol-1-yl)(1-(4-(3-((tetrahydro-2H-pyran-4-yl)oxy)phenyl)pyridin-2-yl)piperidin-4-yl)methanone

The preparation was carried out in a similar manner to Example 1, except that in step 4, intermediate B-8 was used instead of intermediate B-2 for reaction. ¹H NMR (400 MHz, DMSO-*d*₆) δ 8.14 (d, *J* = 5.2 Hz, 1H), 7.38 (dd, *J* = 8.9, 7.2 Hz, 1H), 7.32 - 7.22 (m, 3H), 7.16 - 7.00 (m, 3H), 6.92 - 6.79 (m, 3H), 5.34 (dd, *J* = 11.9, 4.9 Hz, 1H), 4.69 (dt, *J* = 8.7, 4.5 Hz, 1H), 4.45 (d, *J* = 11.8 Hz, 2H), 3.86 (dt, *J* = 11.6, 4.4 Hz, 2H), 3.55 - 3.39 (m, 4H), 2.93 (dt, *J* = 13.0, 10.3 Hz, 2H), 2.75 (dd, *J* = 19.1, 5.0 Hz, 1H), 2.00 - 1.98 (m, 3H), 1.90 (d, *J* = 12.5 Hz, 1H), 1.76 (d, *J=* 13.0 Hz, 1H), 1.67 - 1.43 (m, 3H). MS: 547 [M+H]⁺.

### Example 4: (5-(3,5-difluorophenyl)-4,5-dihydro-1H-pyrazol-1-yl)(1-(4-(5-(2-methoxyethoxy)-2-methylphenyl)pyridin-2-yl)piperidin-4-yl)methanone

The preparation was carried out in a similar manner to Example 1, except that in step 4, intermediate B-20 was used instead of intermediate B-2 for reaction. ¹H NMR (400 MHz, DMSO-*d*₆) δ 8.12 (d, *J* = 5.1 Hz, 1H), 7.27 - 7.07 (m, 3H), 6.92 - 6.72 (m, 5H), 6.59 (d, *J* = 5.1 Hz, 1H), 5.34 (dd, *J* = 12.1, 5.0 Hz, 1H), 4.43 - 4.34 (m, 2H), 4.13 - 4.05 (m, 2H), 3.68 - 3.60 (m, 2H), 3.51 - 3.49 (m, 2H), 3.30 (s, 3H), 3.00 - 2.86 (m, 2H), 2.80 - 2.68 (m, 1H), 2.16 (s, 3H), 1.88 (d, *J* = 12.9 Hz, 1H), 1.74 (d, *J* = 13.0 Hz, 1H), 1.53 - 1.50 (m, 2H). MS:535 [M+H]⁺.

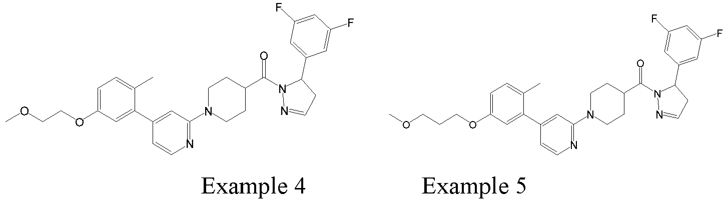

### Example 5: (5-(3,5-difluorophenyl)-4,5-dihydro-1H-pyrazol-1-yl)(1-(4-(5-(3-methoxypropoxy)-2-methylphenyl)pyridin-2-yl)piperidin-4-yl)methanone

The preparation was carried out in a similar manner to Example 1, except that in step 4, intermediate B-21 was used instead of intermediate B-2 for reaction. ¹H NMR (400 MHz, DMSO-*d*₆) δ 8.12 (d, *J* = 5.1 Hz, 1H), 7.27 - 7.06 (m, 3H), 6.91 - 6.79 (m, 3H), 6.78 - 6.71 (m, 2H), 6.57 (dd, *J* = 5.1, 1.2 Hz, 1H), 5.34 (dd, *J* = 12.0, 5.0 Hz, 1H), 4.39 (d, *J* = 12.0 Hz, 2H), 4.01 (t, *J* = 6.4 Hz, 2H), 3.55 - 3.41 (m, 4H), 3.24 (s, 3H), 2.99 - 2.85 (m, 2H), 2.74 (dd, *J* = 19.0, 5.0 Hz, 1H), 2.15 (s, 3H), 1.98 - 1.84 (m, 3H), 1.74 (d, *J* = 12.8 Hz, 1H), 1.64 - 1.43 (m, 2H). MS:549 [M+H]⁺.

### Example 6: (5-(3,5-difluorophenyl)-4,5-dihydro-1H-pyrazol-1-yl)(1-(4-(2-methyl-5-((tetrahydro-2H-pyran-4-yl))oxy)phenyl)pyridin-2-yl)piperidin-4-yl)methanone

The preparation was carried out in a similar manner to Example 1, except that in step 4, intermediate B-23 was used instead of intermediate B-2 for reaction. ¹H NMR (400 MHz, DMSO-*d*₆) δ 8.12 (d, *J* = 5.1 Hz, 1H), 7.27 - 7.06 (m, 3H), 6.96 - 6.77 (m, 4H), 6.73 (s, 1H), 6.57 (dd, *J* = 5.1, 1.2 Hz, 1H), 5.34 (dd, *J* = 12.0, 5.0 Hz, 1H), 4.57 (dt, *J* = 8.7, 4.5 Hz, 1H), 4.39 (d, *J* = 13.0 Hz, 2H), 3.83 (dt, *J* = 11.6, 4.4 Hz, 2H), 3.55 - 3.41 (m, 4H), 2.92 (q, *J* = 11.2 Hz, 2H), 2.74 (dd, *J* = 19.0, 5.0 Hz, 1H), 2.15 (s, 3H), 2.02 - 1.84 (m, 4H), 1.73 (d, *J* = 13.0 Hz, 1H), 1.63 - 1.46 (m, 3H). MS:561 [M+H]⁺.

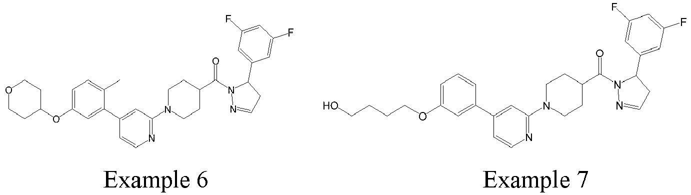

### Example 7: (5-(3,5-difluorophenyl)-4,5-dihydro-1H-pyrazol-1-yl)(1-(4-(3-(4-hydroxybutoxy)phenyl)pyridin-2-yl)piperidin-4-yl)methanone

The preparation was carried out in a similar manner to Example 1, except that in step 4, intermediate B-5 was used instead of intermediate B-2 for reaction. ¹H NMR (400 MHz, DMSO-*d*₆) δ 8.14 (d, *J* = 5.2 Hz, 1H), 7.38 (t, *J* = 7.9 Hz, 1H), 7.32 - 7.21 (m, 3H), 7.16 - 7.07 (m, 1H), 7.06 - 6.95 (m, 2H), 6.93 - 6.80 (m, 3H), 5.34 (dd, *J* = 12.0, 5.0 Hz, 1H), 4.51 - 4.41 (m, 3H), 4.06 (t, *J* = 6.5 Hz, 2H), 3.51 - 3.42 (m, 4H), 2.93 (q, *J* = 11.5 Hz, 2H), 2.74 (dd, *J* = 19.1, 5.1 Hz, 1H), 1.90 (d, *J* = 12.9 Hz, 1H), 1.83 - 1.71 (m, 3H), 1.65 - 1.45 (m, 4H). MS: 535 [M+H]⁺.

### Example 8: (5-(3,5-difluorophenyl)-4,5-dihydro-1H-pyrazol-1-yl)(1-(4-(3-((4-hydroxy-4-methylpentyl)oxy)phenyl)pyridin-2-yl)piperidin-4-yl)methanone

The preparation was carried out in a similar manner to Example 1, except that in step 4, intermediate B-7 was used instead of intermediate B-2 for reaction. ¹H NMR (400 MHz, DMSO-*d*₆) δ 8.14 (d, *J* = 5.2 Hz, 1H), 7.38 (t, *J* = 7.9 Hz, 1H), 7.31 - 7.20 (m, 3H), 7.12 (tt, *J* = 9.3, 2.3 Hz, 1H), 7.06 - 6.95 (m, 2H), 6.92 - 6.79 (m, 3H), 5.39 - 5.30 (m, 1H), 4.45 (d, *J* = 13.4 Hz, 2H), 4.20 (s, 1H), 4.04 (t, *J* = 6.5 Hz, 2H), 3.54 - 3.36 (m, 2H), 2.93 (td, *J* = 12.6, 9.4 Hz, 2H), 2.75 (dd, *J* = 19.0, 5.0 Hz, 1H), 2.06 - 1.84 (m, 2H), 1.84 - 1.72 (m, 3H), 1.64 - 1.46 (m, 3H), 1.11 (s, 6H). MS:563 [M+H]⁺.

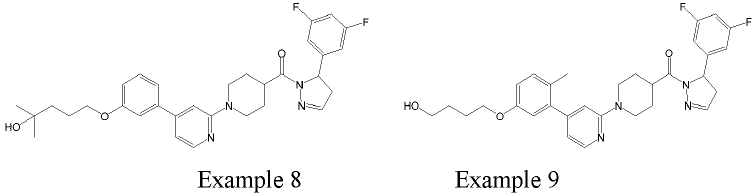

### Example 9: (5-(3,5-difluorophenyl)-4,5-dihydro-1H-pyrazol-1-yl)(1-(4-(5-(4-hydroxybutoxy)-2-methylphenyl)pyridin-2-yl)piperidin-4-yl)methanone

The preparation was carried out in a similar manner to Example 1, except that in step 4, intermediate B-22 was used instead of intermediate B-2 for reaction. ¹H NMR (400 MHz, DMSO-*d*₆) δ 8.12 (d, *J* = 5.0 Hz, 1H), 7.27 - 7.07 (m, 3H), 6.86 - 6.84 (m, 3H), 6.78 - 6.70 (m, 2H), 6.57 (d, *J* = 5.1 Hz, 1H), 5.39 - 5.29 (m, 1H), 4.48 - 4.34 (m, 3H), 3.97 (t, *J* = 6.5 Hz, 2H), 3.54 - 3.39 (m, 4H), 2.92 (q, *J* = 11.0, 10.6 Hz, 2H), 2.81 - 2.68 (m, 1H), 2.15 (s, 3H), 2.06 - 1.95 (m, 1H), 1.88 (d, *J* = 12.9 Hz, 1H), 1.75 - 1.65 (mp, 3H), 1.63 - 1.43 (m, 3H). MS:549 [M+H]⁺.

### Example 10: (5-(3,5-difluorophenyl)-4,5-dihydro-1H-pyrazol-1-yl)(1-(4-(5-((4-hydroxy-4-methylpentyl)oxy)-2-methylphenyl))pyridin-2-yl)piperidin-4-yl)methanone

The preparation was carried out in a similar manner to Example 1, except that in step 4, intermediate B-25 was used instead of intermediate B-2 for reaction. ¹H NMR (400 MHz, DMSO-*d*₆) δ 8.12 (d, *J* = 5.1 Hz, 1H), 7.27 - 7.06 (m, 3H), 6.87 - 6.84 (m, 3H), 6.77 - 6.70 (m, 2H), 6.57 (dd, *J* = 5.1 Hz, 1H), 5.34 (dd, *J* = 12.1, 4.9 Hz, 1H), 4.39 (d, *J* = 12.9 Hz, 2H), 4.17 (s, 1H), 3.95 (t, *J* = 6.6 Hz, 2H), 3.54 - 3.39 (m, 2H), 2.99 - 2.88 (m, 2H), 2.74 (dd, *J* = 19.1, 5.0 Hz, 1H), 2.15 (s, 3H), 2.05 - 1.99 (m, 1H), 1.88 (d, *J* = 12.9 Hz, 1H), 1.80 - 1.68 (m, 3H), 1.61 - 1.42 (m, 3H), 1.10 (s, 6H). MS: 577 [M+H]⁺.

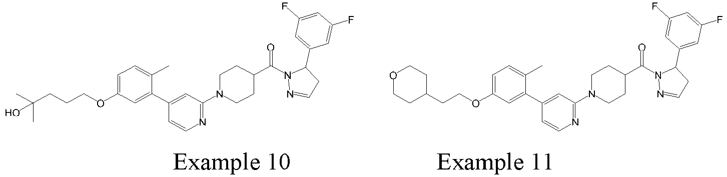

### Example 11: (5-(3,5-difluorophenyl)-4,5-dihydro-1H-pyrazol-1-yl)(1-(4-(2-methyl)-5-(2-(tetrahydro-2H-pyran-4-yl)ethoxy)phenyl)pyridin-2-yl)piperidin-4-yl)methanone

The preparation was carried out in a similar manner to Example 1, except that in step 4, intermediate B-28 was used instead of intermediate B-2 for reaction. ¹H NMR (400 MHz, DMSO-*d*₆) δ 8.12 (d, *J* = 5.1 Hz, 1H), 7.27 - 7.06 (m, 3H), 6.91 - 6.79 (m, 3H), 6.79 - 6.71 (m, 2H), 6.58 (d, *J* = 5.1Hz, 1H), 5.34 (dd, *J* = 12.0, 5.0 Hz, 1H), 4.38 (d, *J* = 11.8 Hz, 2H), 4.01 (t, *J* = 6.3 Hz, 2H), 3.87 - 3.78 (m, 2H), 3.49 - 3.48 (m, 2H), 3.27 - 3.25 (m, 4H), 2.92 (q, *J* = 11.1, 10.6 Hz, 2H), 2.74 (dd, *J* = 19.1, 5.1 Hz, 1H), 2.15 (s, 3H), 1.99 (t, *J* = 7.4 Hz, 1H), 1.88 (d, *J* = 12.9 Hz, 1H), 1.78 - 1.43 (m, 7H). MS:589 [M+H]⁺.

### Example 12: (5-(3,5-difluorophenyl)-4,5-dihydro-1H-pyrazol-1-yl)(1-(4-(3-(3-hydroxypropoxy)phenyl)pyridin-2-yl)piperidin-4-yl)methanone

The preparation was carried out in a similar manner to Example 1, except that in step 4, intermediate B-3 was used instead of intermediate B-2 for reaction. ¹H NMR (400 MHz, DMSO-*d*₆) δ 8.14 (d, *J* = 5.2 Hz, 1H), 7.38 (t, *J* = 7.9 Hz, 1H), 7.32 - 7.22 (m, 3H), 7.12 (tt, *J* = 9.4, 2.3 Hz, 1H), 7.06 - 6.97 (m, 2H), 6.92 - 6.79 (m, 3H), 5.34 (dd, *J* = 11.9, 5.0 Hz, 1H), 4.57 (t, *J* = 5.1 Hz, 1H), 4.45 (d, *J* = 13.4 Hz, 2H), 4.11 (t, *J* = 6.4 Hz, 2H), 3.63 - 3.42 (m, 4H), 2.93 (dt, *J* = 13.2, 9.9 Hz, 2H), 2.74 (dd, *J* = 19.0, 5.0 Hz, 1H), 1.89 (p, *J* = 6.2 Hz, 3H), 1.76 (d, *J* = 13.2 Hz, 1H), 1.65 - 1.48 (m, 2H). MS:521 [M+H]⁺.

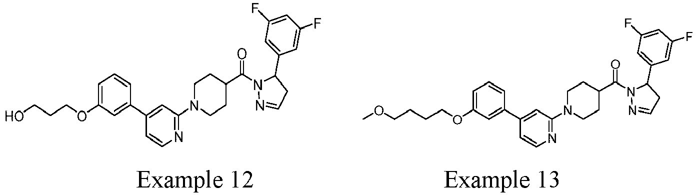

### Example 13: (5-(3,5-difluorophenyl)-4,5-dihydro-1H-pyrazol-1-yl)(1-(4-(3-(4-methoxybutoxy)phenyl)pyridin-2-yl)piperidin-4-yl)methanone

The preparation was carried out in a similar manner to Example 1, except that in step 4, intermediate B-6 was used instead of intermediate B-2 for reaction. ¹H NMR (400 MHz, DMSO-*d*₆) δ 8.14 (d, *J* = 5.2 Hz, 1H), 7.38 (t, *J* = 7.9 Hz, 1H), 7.32 - 7.21 (m, 3H), 7.11 (t, *J* = 9.4 Hz, 1H), 7.06 - 6.96 (m, 2H), 6.92 - 6.79 (m, 3H), 5.34 (dd, *J* = 11.9, 5.0 Hz, 1H), 4.49 - 4.40 (m, 2H), 4.06 (t, *J* = 6.3 Hz, 2H), 3.54 - 3.36 (m, 4H), 3.24 (s, 3H), 2.93 (dt, *J* = 12.9, 9.8 Hz, 2H), 2.74 (dd, *J* = 19.0, 5.0 Hz, 1H), 1.90 (d, *J* = 12.5 Hz, 1H), 1.81 - 1.64 (m, 5H), 1.55 (dt, *J* = 20.8, 12.2 Hz, 2H). MS:549 [M+H]⁺.

### Example 14: (5-(3,5-difluorophenyl)-4,5-dihydro-1H-pyrazol-1-yl)(1-(4-(3-((tetrahydro-2H-pyran-4-yl)methoxy)phenyl)pyridin-2-yl)piperidin-4-yl)methanone

The preparation was carried out in a similar manner to Example 1, except that in step 4, intermediate B-9 was used instead of intermediate B-2 for reaction. ¹H NMR (400 MHz, DMSO-*d*₆) δ 8.14 (d, *J* = 5.2 Hz, 1H), 7.38 (t, *J* = 7.9 Hz, 1H), 7.32 - 7.21 (m, 3H), 7.12 (t, *J* = 9.3 Hz, 1H), 7.06 - 6.97 (m, 2H), 6.93 - 6.80 (m, 3H), 5.34 (dd, *J* = 11.9, 4.9 Hz, 1H), 4.49 - 4.41 (m, 2H), 3.95 - 3.84 (m, 4H), 3.55 - 3.40 (m, 4H), 2.93 (td, *J* = 12.6, 9.4 Hz, 2H), 2.75 (dd, *J* = 19.0, 4.9 Hz, 1H), 2.06 - 1.95 (m, 1H), 1.90 (d, *J* = 12.7 Hz, 1H), 1.80 - 1.66 (m, 3H), 1.55 (dd, *J* = 21.9, 12.5 Hz, 2H), 1.36 - 1.32 (m, 2H). MS: 561 [M+H]⁺.

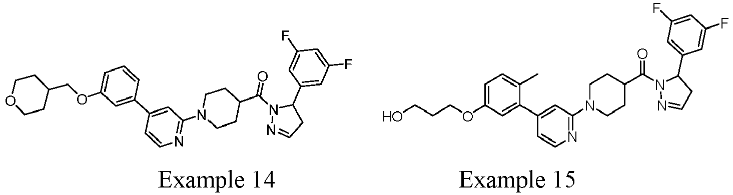

### Example 15: (5-(3,5-difluorophenyl)-4,5-dihydro-1H-pyrazol-1-yl)(1-(4-(5-(3-hydroxypropoxy)-2-methylphenyl)pyridin-2-yl)piperidin-4-yl)methanone

The preparation was carried out in a similar manner to Example 1, except that in step 4, intermediate B-24 was used instead of intermediate B-2 for reaction. ¹H NMR (400 MHz, DMSO-*d*₆) δ 8.12 (d, *J* = 5.1 Hz, 1H), 7.27 - 7.22 (m, 1H), 7.19 (d, *J* = 8.5 Hz, 1H), 7.12 (t, *J* = 9.3 Hz, 1H), 6.91 - 6.80 (m, 3H), 6.78 - 6.70 (m, 2H), 6.57 (d, *J* = 5.1 Hz, 1H), 5.34 (dd, *J* = 11.9, 5.0 Hz, 1H), 4.53 (t, *J* = 5.2 Hz, 1H), 4.38 (dd, *J* = 13.2, 3.8 Hz, 2H), 4.02 (t, *J* = 6.4 Hz, 2H), 3.59 - 3.41 (m, 4H), 2.92 (dt, *J* = 12.8, 9.5 Hz, 2H), 2.74 (dd, *J* = 19.0, 5.0 Hz, 1H), 2.15 (s, 3H), 1.92 - 1.79 (m, 3H), 1.74 (d, *J* = 12.9 Hz, 1H), 1.64 - 1.44 (m, 2H). MS: 535 [M+H]⁺.

### Example 16: (1-(4-(2-chloro-5-(2-methoxyethoxy)phenyl)pyridin-2-yl)piperidin-4-yl)(5-(3,5-difluorophenyl)-4,5-dihydro-1H-pyrazol-1-yl)methanone

The preparation was carried out in a similar manner to Example 1, except that in step 4, intermediate B-10 was used instead of intermediate B-2 for reaction. ¹H NMR (400 MHz, DMSO-*d*₆) δ 8.15 (d, *J* = 5.1 Hz, 1H), 7.45 (d, *J* = 8.7 Hz, 1H), 7.25 (s, 1H), 7.17 - 7.07 (m, 1H), 7.06 - 6.95 (m, 2H), 6.88 - 6.80 (m, 3H), 6.66 (d, *J* = 5.1 Hz, 1H), 5.39 - 5.29 (m, 1H), 4.39 (d, *J* = 13.1 Hz, 2H), 4.18 - 4.10 (m, 2H), 3.69 - 3.62 (m, 2H), 3.50 - 3.48 (m, 2H), 3.30 (s, 3H), 2.95 - 2.92 (m, 2H), 2.74 (dd, *J* = 18.9, 5.0 Hz, 1H), 1.89 (d, *J* = 12.9 Hz, 1H), 1.74 (d, *J* = 13.0 Hz, 1H), 1.61 - 1.43 (m, 2H). MS:555 [M+H]⁺.

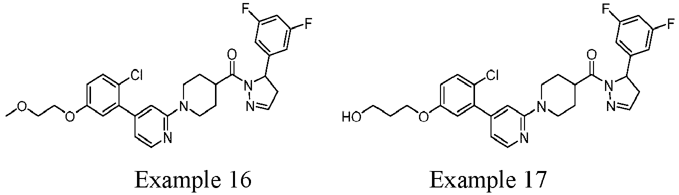

### Example 17: (1-(4-(2-chloro-5-(3-hydroxypropoxy)phenyl)pyridin-2-yl)piperidin-4-yl)(5-(3,5-difluorophenyl)-4,5-dihydro-1H-pyrazol-1-yl)methanone

The preparation was carried out in a similar manner to Example 1, except that in step 4, intermediate B-11 was used instead of intermediate B-2 for reaction. ¹H NMR (400 MHz, DMSO-*d*₆) δ 8.15 (d, *J* = 5.1 Hz, 1H), 7.44 (d, *J* = 8.8 Hz, 1H), 7.27 - 7.23 (m, 1H), 7.13 - 7.11 (m, 1H), 7.04 - 6.92 (m, 2H), 6.85 - 6.82 (m, 3H), 6.65 (d, *J* = 5.1 Hz, 1H), 5.39 - 5.29 (m, 2H), 4.38 (d, *J* = 12.2 Hz, 2H), 3.59 - 3.41 (m, 4H), 2.93 (d, *J* = 11.9 Hz, 2H), 2.74 (dd, *J* = 19.0, 5.0 Hz, 1H), 2.0 - 1.97 (m, 2H), 1.88 - 1.85 (m, 3H), 1.74 (d, *J* = 13.0 Hz, 1H), 1.61 - 1.43 (m, 2H). MS: 555 [M+H]⁺.

### Example 18: (5-(3,5-difluorophenyl)-4,5-dihydro-1H-pyrazol-1-yl)(1-(4-(2-fluoro-5-(3-hydroxypropoxy)phenyl)pyridin-2-yl))piperidin-4-yl)methanone

The preparation was carried out in a similar manner to Example 1, except that in step 4, intermediate B-12 was used instead of intermediate B-2 for reaction. ¹H NMR (400 MHz, DMSO-*d*₆) δ 8.15 (d, *J* = 5.1 Hz, 1H), 7.27 - 7.19 (m, 2H), 7.16 - 7.04 (m, 2H), 6.99 (d, *J* = 9.0 Hz, 1H), 6.93 (s, 1H), 6.86 - 6.82 (m, 2H), 6.76 (d, *J* = 5.1 Hz, 1H), 5.34 (dd, *J* = 12.0, 5.0 Hz, 1H), 4.56 (t, *J* = 5.2 Hz, 1H), 4.40 (d, *J* = 11.9 Hz, 2H), 4.06 (t, *J* = 6.4 Hz, 2H), 3.60 - 3.40 (m, 4H), 2.93 (q, *J* = 10.8 Hz, 2H), 2.74 (dd, *J* = 19.0, 5.0 Hz, 1H), 1.87 - 1.85 (m, 3H), 1.74 (d, *J* = 13.0 Hz, 1H), 1.63 - 1.42 (m, 2H). MS:539 [M+H]⁺.

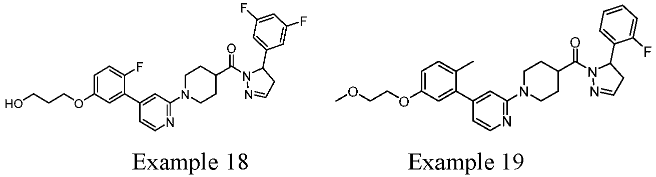

### Example 19: (5-(2-fluorophenyl)-4,5-dihydro-1H-pyrazol-1-yl)(1-(4-(5-(2-methoxyethoxy)-2-methylphenyl)pyridin-2-yl)piperidin-4-yl)methanone

The preparation was carried out in a similar manner to Example 1, except that in step 1, intermediate A-2 was used instead of intermediate A-1, and in step 4, intermediate B-20 was used instead of intermediate B-2 for reaction. ¹H NMR (400 MHz, DMSO-*d*₆) δ 8.12 (d, *J* = 5.0 Hz, 1H), 7.34 - 7.24 (m, 2H), 7.23 - 7.10 (m, 3H), 7.05 (t, *J* = 7.7 Hz, 1H), 6.88 (d, *J* = 8.4 Hz, 1H), 6.80 - 6.71 (m, 2H), 6.58 (d, *J* = 5.1 Hz, 1H), 5.45 (dd, *J* = 12.1, 5.1 Hz, 1H), 4.38 (d, *J* = 13.0 Hz, 2H), 4.12 - 4.05 (m, 2H), 3.67 - 3.60 (m, 2H), 3.54 - 3.50 (m, 2H), 3.30 (s, 3H), 2.92 (td, *J* = 12.9, 7.5 Hz, 2H), 2.71 (dd, *J* = 19.0, 5.2 Hz, 1H), 2.15 (s, 3H), 1.86 (d, *J* = 12.9 Hz, 1H), 1.73 (d, *J* = 12.5 Hz, 1H), 1.58 - 1.46 (m, 2H). MS: 517 [M+H]⁺.

### Example 20: (5-(2-fluorophenyl)-4,5-dihydro-1H-pyrazol-1-yl)(1-(4-(5-(3-hydroxypropoxy)-2-methylphenyl)pyridin-2-yl)piperidin-4-yl)methanone

The preparation was carried out in a similar manner to Example 1, except that in step 1, intermediate A-2 was used instead of intermediate A-1, and in step 4, intermediate B-24 was used instead of intermediate B-2 for reaction. ¹H NMR (400 MHz, DMSO-*d*₆) δ 8.12 (d, *J* = 5.1 Hz, 1H), 7.34 - 7.25 (m, 2H), 7.22 - 7.12 (m, 3H), 7.05 (t, *J* = 7.7 Hz, 1H), 6.87 (d, *J* = 8.4 Hz, 1H), 6.75 (s, 1H), 6.72 (s, 1H), 6.57 (d, *J* = 5.1 Hz, 1H), 5.45 (dd, *J* = 12.0, 5.1 Hz, 1H), 4.53 (t, *J* = 5.2 Hz, 1H), 4.38 (d, *J* = 13.2 Hz, 2H), 4.02 (t, *J* = 6.4 Hz, 2H), 3.59 - 3.51 (m, 2H), 3.32 (br, 2H), 2.91 (q, *J* = 11.1, 10.6 Hz, 2H), 2.77 - 2.64 (m, 1H), 2.15 (s, 3H), 2.08 - 2.00 (m, 1H), 1.85 (q, *J* = 6.4 Hz, 2H), 1.73 (d, *J* = 13.0 Hz, 1H), 1.62 - 1.42 (m, 2H). MS: 517 [M+H]⁺.

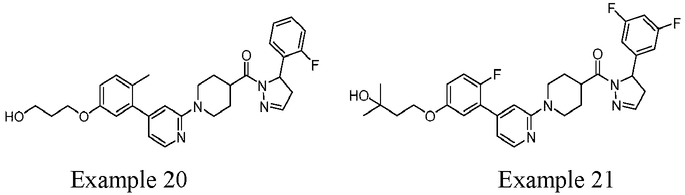

### Example 21: (5-(3,5-difluorophenyl)-4,5-dihydro-1H-pyrazol-1-yl)(1-(4-(2-fluoro-5-(3-hydroxy-3-methylbutoxy)phenyl)pyridine)-2-yl)piperidin-4-yl)methanone

The preparation was carried out in a similar manner to Example 1, except that in step 4, intermediate B-13 was used instead of intermediate B-2 for reaction. ¹H NMR (400 MHz, DMSO-*d*₆) δ 8.16 (d, *J* = 5.1 Hz, 1H), 7.30 - 7.19 (m, 2H), 7.18 - 7.03 (m, 2H), 7.00 (t, *J* = 9.0 Hz, 1H), 6.93 (s, 1H), 6.89 - 6.79 (m, 2H), 6.76 (d, *J* = 5.0 Hz, 1H), 5.34 (dd, *J* = 12.0, 4.9 Hz, 1H), 4.47 - 4.31 (m, 3H), 4.11 (t, *J* = 7.2 Hz, 2H), 3.48 - 3.40 (m, 2H), 2.93 (dt, *J* = 12.7, 10.3 Hz, 2H), 2.74 (dd, *J* = 19.0, 5.0 Hz, 1H), 1.95 - 1.80 (m, 3H), 1.75 (d, *J* = 12.7 Hz, 1H), 1.55 - 1.52 (m, 2H), 1.16 (s, 6H). MS: 567 [M+H]⁺.

### Example 22: (5-(3,5-difluorophenyl)-4,5-dihydro-1H-pyrazol-1-yl)(1-(4-(2-fluoro-5-(2-hydroxyethoxy)phenyl)pyridin-2-yl))piperidin-4-yl)methanone

The preparation was carried out in a similar manner to Example 1, except that in step 4, intermediate B-14 was used instead of intermediate B-2 for reaction. ¹H NMR (400 MHz, DMSO-*d*₆) δ 8.16 (d, *J* = 5.1 Hz, 1H), 7.29 - 7.19 (m, 2H), 7.18 - 6.96 (m, 3H), 6.93 (s, 1H), 6.89 - 6.73 (m, 3H), 5.34 (dd, *J* = 12.0, 4.9 Hz, 1H), 4.90 (br, 1H), 4.45 - 4.36 (m, 2H), 4.03 (t, *J* = 5.0 Hz, 2H), 3.72 (t, *J* = 4.9 Hz, 2H), 3.55 - 3.29 (m, 2H), 3.01 - 2.86 (m, 2H), 2.74 (dd, *J* = 19.0, 5.0 Hz, 1H), 1.89 (d, *J* = 12.2 Hz, 1H), 1.75 (d, *J* = 12.5 Hz, 1H), 1.56 - 1.53 (m, 2H). MS: 525 [M+H]⁺.

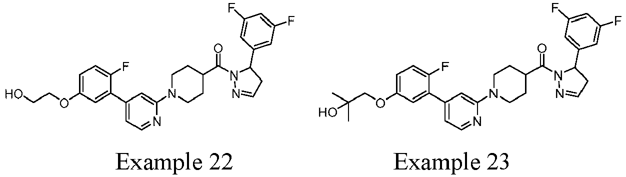

### Example 23: (5-(3,5-difluorophenyl)-4,5-dihydro-1H-pyrazol-1-yl)(1-(4-(2-fluoro-5-(2-hydroxy-2-methylpropoxy)phenyl)pyridine)-2-yl)piperidin-4-yl)methanone

The preparation was carried out in a similar manner to Example 1, except that in step 4, intermediate B-15 was used instead of intermediate B-2 for reaction. ¹H NMR (400 MHz, DMSO-*d*₆) δ 8.16 (d, *J* = 5.1 Hz, 1H), 7.28 - 7.18 (m, 2H), 7.17 - 6.96 (m, 3H), 6.93 (s, 1H), 6.88 - 6.80 (m, 2H), 6.77 (dd, *J* = 5.2, 1.7 Hz, 1H), 5.34 (dd, *J* = 12.0, 4.9 Hz, 1H), 4.64 (s, 1H), 4.40 (d, *J* = 12.9 Hz, 2H), 3.76 (s, 2H), 3.48 - 3.40 (m, 2H), 2.94 (q, *J* = 10.7 Hz, 2H), 2.74 (dd, *J* = 19.0, 5.0 Hz, 1H), 1.89 (d, *J* = 12.8 Hz, 1H), 1.75 (d, *J* = 12.8 Hz, 1H), 1.63 - 1.43 (m, 2H), 1.20 (s, 6H). MS:553 [M+H]⁺.

### Example 24: (5-(3,5-difluorophenyl)-4,5-dihydro-1H-pyrazol-1-yl)(1-(4-(2-fluoro-5-((4-hydroxy-4-methylpentyl)oxy))phenyl)pyridin-2-yl)piperidin-4-yl)methanone

The preparation was carried out in a similar manner to Example 1, except that in step 4, intermediate B-16 was used instead of intermediate B-2 for reaction. ¹H NMR (400 MHz, DMSO-*d*₆) δ 8.16 (d, *J* = 5.2 Hz, 1H), 7.28 - 7.18 (m, 2H), 7.18 - 6.94 (m, 3H), 6.93 (s, 1H), 6.89 - 6.73 (m, 3H), 5.34 (dd, *J* = 12.0, 4.9 Hz, 1H), 4.45 - 4.36 (m, 2H), 4.20 (s, 1H), 3.99 (t, *J* = 6.5 Hz, 2H), 3.48 - 3.40 (m, 2H), 2.93 (dt, *J* = 13.4, 10.0 Hz, 2H), 2.74 (dd, *J* = 19.0, 5.0 Hz, 1H), 1.94 - 1.85 (m, 1H), 1.82 - 1.69 (m, 3H), 1.64 - 1.44 (m, 4H), 1.10 (s, 6H). MS:581 [M+H]⁺.

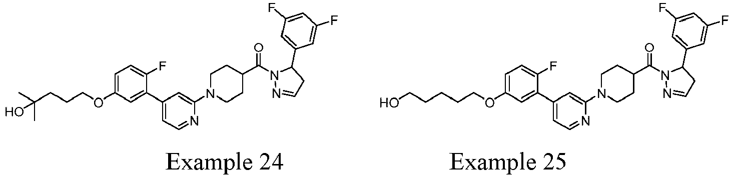

### Example 25: (5-(3,5-difluorophenyl)-4,5-dihydro-1H-pyrazol-1-yl)(1-(4-(2-fluoro-5-((5-hydroxypentyl)oxy)phenyl)pyridin-2-yl)piperidin-4-yl)methanone

The preparation was carried out in a similar manner to Example 1, except that in step 4, intermediate B-17 was used instead of intermediate B-2 for reaction. ¹H NMR (400 MHz, DMSO-*d*₆) δ 8.15 (*d, J* = 5.1 Hz, 1H), 7.28 - 7.18 (m, 2H), 7.18 - 6.95 (m, 3H), 6.93 (s, 1H), 6.89 - 6.73 (m, 3H), 5.34 (dd, *J* = 12.0, 4.9 Hz, 1H), 4.42 - 4.38 (m, 3H), 4.00 (t, *J* = 6.5 Hz, 2H), 3.55 - 3.30 (m, 4H), 2.93 (q, *J* = 11.4 Hz, 2H), 2.74 (dd, *J* = 19.0, 5.0 Hz, 1H), 1.89 (d, *J* = 12.9 Hz, 1H), 1.76 - 1.70 (m, 3H), 1.62 - 1.39 (m, 6H). MS:567 [M+H]⁺.

### Example 26: (5-(3,5-difluorophenyl)-4,5-dihydro-1H-pyrazol-1-yl)(1-(4-(2-fluoro-5-((5-hydroxy-5-methylhexyl)oxy))phenyl)pyridin-2-yl)piperidin-4-yl)methanone

The preparation was carried out in a similar manner to Example 1, except that in step 4, intermediate B-18 was used instead of intermediate B-2 for reaction. ¹H NMR (400 MHz, DMSO-*d*₆) δ 8.16 (d, *J* = 5.1 Hz, 1H), 7.28 - 7.18 (m, 2H), 7.18 - 6.95 (m, 3H), 6.93 (s, 1H), 6.88 - 6.80 (m, 2H), 6.77 (d, *J* = 5.0 Hz, 1H), 5.34 (dd, *J* = 12.0, 5.0 Hz, 1H), 4.45 - 4.39 (m, 2H), 4.11 (s, 1H), 4.00 (t, *J* = 6.4 Hz, 2H), 3.48 - 3.40 (m, 2H), 2.93 (q, *J* = 11.2 Hz, 2H), 2.74 (dd, *J* = 19.0, 5.0 Hz, 1H), 1.94 - 1.85 (m, 1H), 1.79 - 1.65 (m, 3H), 1.62 - 1.34 (m, 6H), 1.07 (s, 6H). MS:595 [M+H]⁺.

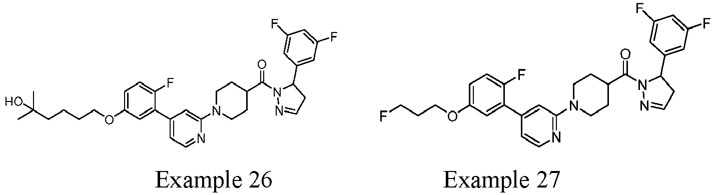

### Example 27: (5-(3,5-difluorophenyl)-4,5-dihydro-1H-pyrazol-1-yl)(1-(4-(2-fluoro-5-(3-fluoropropoxy)phenyl)pyridin-2-yl))piperidin-4-yl)methanone

The preparation was carried out in a similar manner to Example 1, except that in step 4, intermediate B-19 was used instead of intermediate B-2 for reaction. ¹H NMR (400 MHz, DMSO-*d*₆) δ 8.16 (d, *J* = 5.2 Hz, 1H), 7.29 - 7.21 (m, 2H), 7.17 - 6.98 (m, 3H), 6.94 (s, 1H), 6.87 - 6.80 (m, 2H), 6.77 (dt, *J* = 5.1, 1.7 Hz, 1H), 5.34 (dd, *J* = 12.0, 5.0 Hz, 1H), 4.68 (t, *J* = 5.9 Hz, 1H), 4.56 (t, *J* = 5.9 Hz, 1H), 4.40 (dd, *J* = 13.3, 3.5 Hz, 2H), 4.12 (t, *J* = 6.2 Hz, 2H), 3.55 - 3.36 (m, 2H), 2.93 (dt, *J* = 12.9, 9.9 Hz, 2H), 2.74 (ddd, *J* = 19.0, 5.0, 1.8 Hz, 1H), 2.18 - 2.04 (m, 2H), 1.94 - 1.85 (m, 1H), 1.75 (d, *J* = 12.2 Hz, 1H), 1.64 - 1.43 (m, 2H). MS:541 [M+H]⁺.

### Example 28: (1-(4-(5-(2-methoxyethoxy)-2-methylphenyl)pyridin-2-yl)piperidin-4-yl)(5-phenyl-4,5-dihydro-1H-pyrazol-1-yl)methanone

The preparation was carried out in a similar manner to Example 1, except that in step 1, intermediate A-3 was used instead of intermediate A-1, and in step 4, intermediate B-20 was used instead of intermediate B-2 for reaction. ¹H NMR (400 MHz, DMSO-*d*₆) δ 8.12 (d, *J* = 5.1 Hz, 1H), 7.36 - 7.15 (m, 5H), 7.13 -7.10 (m, 2H), 6.88 (d, *J* = 8.4 Hz, 1H), 6.80 - 6.70 (m, 2H), 6.58 (d, *J* = 5.0 Hz, 1H), 5.34 - 5.29 (m, 1H), 4.42 - 4.33 (m, 2H), 4.12 - 4.05 (m, 2H), 3.68 - 3.60 (m, 2H), 3.42 - 3.36 (m, 2H), 3.30 (s, 3H), 2.94 - 3.88 (m, 2H), 2.67 (dd, *J* = 18.9, 4.7 Hz, 1H), 2.15 (s, 3H), 1.91 - 1.82 (m, 1H), 1.73 (d, *J* = 13.0 Hz, 1H), 1.55 - 1.51 (m, 2H). MS: 499 [M+H]⁺.

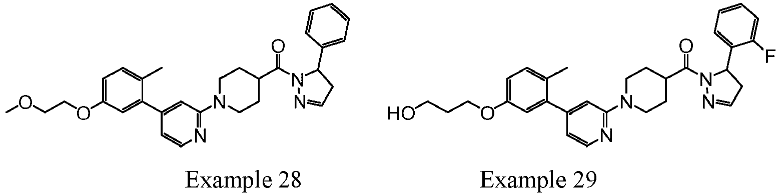

### Example 29: (5-(2-fluorophenyl)-4,5-dihydro-1H-pyrazol-1-yl)(1-(4-(5-(3-hydroxypropoxy)-2-methylphenyl)pyridin-2-yl)piperidin-4-yl)methanone

The preparation was carried out in a similar manner to Example 1, except that in step 1, intermediate A-2 was used instead of intermediate A-1, and in step 4, intermediate B-24 was used instead of intermediate B-2 for reaction. ¹H NMR (400 MHz, DMSO-*d*₆) δ 8.12 (d, *J* = 5.1 Hz, 1H), 7.34 - 7.24 (m, 2H), 7.22 - 7.12 (m, 3H), 7.10 - 7.01 (m, 1H), 6.87 (d, *J* = 8.4 Hz, 1H), 6.78 - 6.70 (m, 2H), 6.57 (d, *J* = 4.9 Hz, 1H), 5.43 - 5.38(m, 1H), 4.53 (s, 1H), 4.37 (d, *J* = 13.2 Hz, 2H), 4.02 (t, *J* = 6.4 Hz, 2H), 3.58 - 3.46 (m, 4H), 2.92 (d, *J* = 9.1 Hz, 2H), 2.71 (dd, *J* = 19.0, 5.1 Hz, 1H), 2.15 (s, 3H), 2.07 - 1.95 (m, 1H), 1.87-1.81 (m, 2H), 1.73 (d, *J* = 12.9 Hz, 1H), 1.63 - 1.45 (m, 2H). MS: 517 [M+H]⁺.

### Example 30: (5-(3,5-difluorophenyl)-4,5-dihydro-1H-pyrazol-1-yl)(1-(4-(2-fluoro-5-(3-methoxypropoxy)phenyl)pyridin-2-yl)piperidin-4-yl)methanone

The preparation was carried out in a similar manner to Example 1, except that in step 4, intermediate B-1 was used instead of intermediate B-2 for reaction. ¹H NMR (400 MHz, DMSO-*d*₆) δ 8.15 (d, *J* = 5.2 Hz, 1H), 7.29 - 7.19 (m, 2H), 7.17 - 7.04 (m, 2H), 7.00 (d, *J* = 9.0 Hz, 1H), 6.93 (s, 1H), 6.86 - 6.82 (m, 2H), 6.79 - 6.73 (m, 1H), 5.34 (dd, *J* = 12.0, 4.9 Hz, 1H), 4.40 (d, *J* = 13.0 Hz, 2H), 4.05 (t, *J* = 6.3 Hz, 2H), 3.55 - 3.41 (m, 4H), 3.24 (s, 3H), 2.93 (dt, *J* = 13.3, 10.5 Hz, 2H), 2.74 (dd, *J* = 19.1, 5.0 Hz, 1H), 2.01 - 1.84 (m, 3H), 1.75 (d, *J* = 13.1 Hz, 1H), 1.58 - 1.52 (m, 2H). MS: 553 [M+H]⁺.

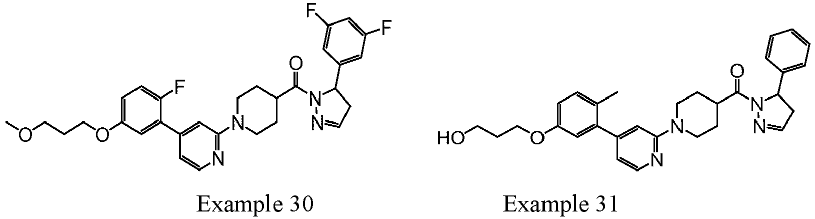

### Example 31: (1-(4-(5-(3-hydroxypropoxy)-2-methylphenyl)pyridin-2-yl)piperidin-4-yl)(5-phenyl-4,5-dihydro-1H-pyrazol-1-yl)methanone

The preparation was carried out in a similar manner to Example 1, except that in step 1, intermediate A-3 was used instead of intermediate A-1, and in step 4, intermediate B-24 was used instead of intermediate B-2 for reaction. ¹H NMR (400 MHz, DMSO-*d*₆) δ 8.12 (d, *J* = 5.1 Hz, 1H), 7.36 - 7.27 (m, 2H), 7.27 - 7.14 (m, 3H), 7.14 - 7.07 (m, 2H), 6.87 (dd, *J* = 8.4, 2.7 Hz, 1H), 6.78 - 6.69 (m, 2H), 6.57 (dd, *J* = 5.1, 1.3 Hz, 1H), 5.34 - 5.29 (m, 2H), 4.38 (d, *J* = 12.8 Hz, 2H), 4.02 (t, *J* = 6.4 Hz, 2H), 3.59 - 3.43 (m, 2H), 2.98 - 2.88 (m, 2H), 2.74 - 2.62 (m, 1H), 2.15 (s, 3H), 2.02 - 1.96 (m, 3H), 1.85 (q, *J* = 6.4 Hz, 2H), 1.73 (d, *J* = 12.7 Hz, 1H), 1.66 - 1.38 (m, 2H). MS: 499 [M+H]⁺.

### Example 32: (5-(3,5-difluorophenyl)-4,5-dihydro-1H-pyrazol-1-yl)(1-(4-(5-(4-methoxybutoxy)-2-methylphenyl)pyridin-2-yl)piperidin-4-yl)methanone

The preparation was carried out in a similar manner to Example 1, except that in step 4, intermediate B-26 was used instead of intermediate B-2 for reaction. ¹H NMR (400 MHz, DMSO-*d*₆) δ 8.12 (d, *J* = 5.1 Hz, 1H), 7.27 - 7.07 (m, 3H), 6.85 (dd, *J* = 11.4, 8.4 Hz, 3H), 6.78 - 6.71 (m, 2H), 6.58 (d, *J* = 5.3 Hz, 1H), 5.34 (dd, *J* = 12.0, 5.0 Hz, 1H), 4.38 (dd, *J* = 13.2, 3.6 Hz, 2H), 3.97 (t, *J* = 6.4 Hz, 2H), 3.60 - 3.41 (m, 4H), 3.22 (s, 3H), 3.00 - 2.85 (m, 2H), 2.74 (dd, *J* = 18.9, 5.0 Hz, 1H), 2.15 (s, 3H), 1.88 (d, *J* = 12.8 Hz, 1H), 1.79 - 1.44 (m, 7H). MS: 563 [M+H]⁺.

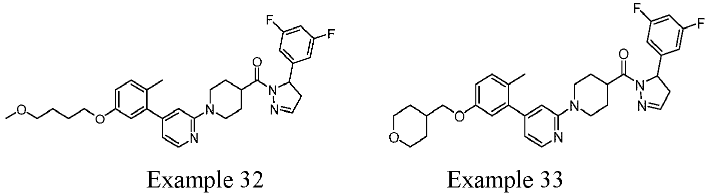

### Example 33: (5-(3,5-difluorophenyl)-4,5-dihydro-1H-pyrazol-1-yl)(1-(4-(2-methyl-5-((tetrahydro-2H-pyran-4-yl))methoxy)phenyl)pyridin-2-yl)piperidin-4-yl)methanone

The preparation was carried out in a similar manner to Example 1, except that in step 4, intermediate B-27 was used instead of intermediate B-2 for reaction. ¹H NMR (400 MHz, DMSO-*d*₆) δ 8.12 (d, *J* = 5.1 Hz, 1H), 7.27 - 7.06 (m, 3H), 6.85 (dd, *J* = 15.5, 8.4 Hz, 3H), 6.78 - 6.70 (m, 2H), 6.57 (d, *J* = 5.1 Hz, 1H), 5.34 (dd, *J* = 12.0, 5.0 Hz, 1H), 4.38 (d, *J*= 12.5 Hz, 2H), 3.91 - 3.79 (m, 4H), 3.54 - 3.41 (m, 4H), 2.92 (q, *J* = 10.7 Hz, 2H), 2.74 (dd, *J* = 19.0, 5.0 Hz, 1H), 2.15 (s, 3H), 1.99 (br, 1H), 1.88 (d, *J* = 12.8 Hz, 1H), 1.70 (dd, *J* = 27.6, 12.7 Hz, 3H), 1.61 - 1.46 (m, 2H), 1.31 (qd, *J* = 12.2, 4.5 Hz, 2H). MS: 575 [M+H]⁺.

### Example 34: (5-(3,5-difluorophenyl)-4,5-dihydro-1H-pyrazol-1-yl)(1-(4-(5-(4-methoxybutoxy)-2-methylphenyl)pyridin-2-yl)piperidin-4-yl)methanone

The preparation was carried out in a similar manner to Example 1, except that in step 4, intermediate B-26 was used instead of intermediate B-2 for reaction. ¹H NMR (400 MHz, DMSO-*d*₆) δ 8.12 (d, *J* = 5.1 Hz, 1H), 7.27 - 7.06 (m, 3H), 6.88 - 6.82 (m, 3H), 6.78 - 6.70 (m, 2H), 6.57 (d, *J* = 5.0 Hz, 1H), 5.34 (dd, *J* = 12.0, 5.0 Hz, 1H), 4.43 - 4.34 (m, 2H), 3.97 (t, *J* = 6.3 Hz, 2H), 3.50 - 3.40 (m, 2H), 3.36 (t, *J* = 6.3 Hz, 2H), 3.22 (s, 3H), 2.92 (q, *J* = 11.2 Hz, 2H), 2.74 (ddd, *J* = 18.9, 5.0, 1.8 Hz, 1H), 2.15 (s, 3H), 1.88 (d, *J* = 13.1 Hz, 1H), 1.78 - 1.46 (m, 7H). MS:563 [M+H]⁺.

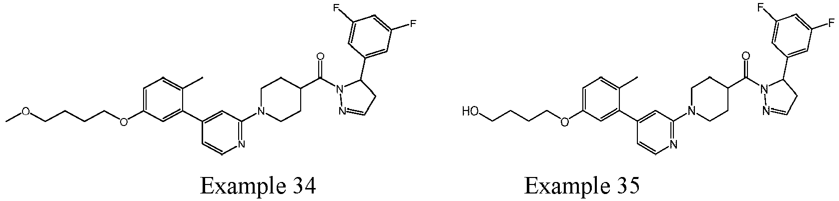

### Example 35: (5-(3,5-difluorophenyl)-4,5-dihydro-1H-pyrazol-1-yl)(1-(4-(5-(4-hydroxybutoxy)-2-methylphenyl)pyridin-2-yl)piperidin-4-yl)methanone

The preparation was carried out in a similar manner to Example 1, except that in step 4, intermediate B-22 was used instead of intermediate B-2 for reaction. ¹H NMR (400 MHz, DMSO-*d*₆) δ 8.12 (d, *J* = 5.1 Hz, 1H), 7.27 - 7.07 (m, 3H), 6.88 - 6.81 (m, 3H), 6.77 - 6.70 (m, 2H), 6.57 (d, *J* = 5.2 Hz, 1H), 5.35 - 5.31 (m, 2H), 4.47 - 4.34 (m, 2H), 3.96 (t, *J* = 6.5 Hz, 2H), 3.56 - 3.46 (m, 2H), 2.92 (q, *J* = 11.0 Hz, 2H), 2.80 - 2.68 (m, 1H), 2.15 (s, 3H), 2.03-1.98 (m, 4H), 1.88 (d, *J=* 13.2 Hz, 1H), 1.75 - 1.69 (m, 2H), 1.58 - 1.50 (m, 3H). MS:549 [M+H]⁺.

### Example 36: (5-(3,5-difluorophenyl)-4,5-dihydro-1H-pyrazol-1-yl)(1-(4-(2-fluoro-5-(4-hydroxybutoxy)phenyl)pyridine)-2-yl)piperidin-4-yl)methanone

The preparation was carried out in a similar manner to Example 1, except that in step 4, intermediate B-29 was used instead of intermediate B-2 for reaction. ¹H NMR (400 MHz, DMSO-*d*₆) δ 8.16 (d, *J* = 5.1 Hz, 1H), 7.28 - 7.18 (m, 2H), 7.17 - 6.95 (m, 3H), 6.93 (s, 1H), 6.89 - 6.73 (m, 3H), 5.34 (dd, *J* = 12.0, 5.0 Hz, 1H), 4.49 - 4.35 (m, 4H), 4.01 (t, *J* = 6.5 Hz, 2H), 3.55 - 3.42 (m, 3H), 3.01 - 2.86 (m, 2H), 2.74 (ddd, *J* = 19.1, 5.0, 1.8 Hz, 1H), 1.94 - 1.85 (m, 1H), 1.78 - 1.71 (m, 3H), 1.64 - 1.43 (m, 4H). MS: 553 [M+H]⁺.

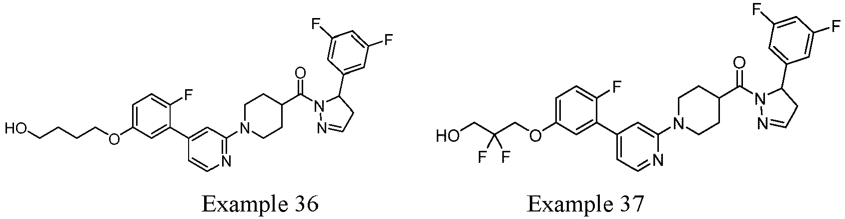

### Example 37: (1-(4-(5-(2,2-difluoro-3-hydroxypropoxy)-2-fluorophenyl)pyridine)-2-yl)piperidin-4-yl) (5-(3,5-difluorophenyl)-4,5-dihydro-1H-pyrazol-1-yl)methanone

The preparation was carried out in a similar manner to Example 1, except that in step 4, intermediate B-30 was used instead of intermediate B-2 for reaction. ¹H NMR (400 MHz, DMSO-*d*₆) δ 8.16 (d, *J* = 5.1 Hz, 1H), 7.33 - 7.05 (m, 5H), 6.95 (s, 1H), 6.89 - 6.75 (m, 3H), 5.67 (t, *J* = 6.2 Hz, 1H), 5.34 (dd, *J* = 12.0, 5.0 Hz, 1H), 4.39 (t, *J* = 12.9 Hz, 4H), 3.76 (td, *J* = 13.8, 6.2 Hz, 2H), 3.48 (m, 2H), 2.94 (dt, *J* = 12.7, 10.4 Hz, 2H), 2.74 (dd, *J* = 19.1, 5.0 Hz, 1H), 1.90 (d, *J* = 12.7 Hz, 1H), 1.76 (d, *J* = 12.9 Hz, 1H), 1.64 - 1.44 (m, 2H). MS:575 [M+H]⁺.

### Example 38: (5-(3,5-difluorophenyl)-4,5-dihydro-1H-pyrazol-1-yl)(1-(4-(2-fluoro-5-(3-hydroxypropoxy)phenyl)pyrimidin-2-yl))piperidin-4-yl)methanone

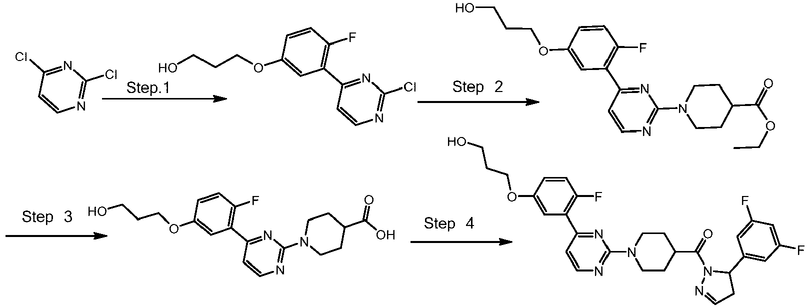

Step 1): 2,4-Dichloropyrimidine (1.00 g, 6.71 mmol), intermediate B-12 (3-(4-fluoro-3-(4,4,5,5-tetramethyl-1,3,2-dioxaborolane-2-yl)phenoxy)propan-1-ol) (2.58 g, 8.73 mmol), potassium carbonate (1.86 g, 13.4 mmol), and Pd[PPh₃]₄ (776mg, 0.671 mmol) were added to a mixed solvent of ethanol (12 mL) and toluene (6 mL). The mixture was heated to 55 °C under argon protection and reacted for 8 hours. The reaction solution was diluted with ethyl acetate, washed with saturated brine, dried over anhydrous sodium sulfate, and filtered. The filtrate was concentrated. The crude product was purified by column chromatography to obtain 1.48 g of 3-(3-(2-chloropyrimidin-4-yl)-4-fluorophenoxy)propyl-1-ol, with a yield of 78%;

Step 2): 3-(3-(2-chloropyrimidin-4-yl)-4-fluorophenoxy)propyl-1-ol (741 mg, 2.62 mmol), ethyl piperidine-4-carboxylate (412.35 mg, 2.62 mmol), and cesium carbonate (1.71 g, 5.25 mmol) were added to DMF (8 mL). The atmosphere was replaced with argon, and the mixture was reacted at 85 °C for 3 hours. The reaction solution was diluted with dichloromethane, and washed with saturated brine. The organic phase was dried over anhydrous sodium sulfate, and filtered. The filtrate was concentrated under reduced pressure to obtain 907 mg of the product with a yield of 85.71%. MS:404 [M+H]⁺;

Step 3): Ethyl 1-(4-(2-fluoro-5-(3-hydroxypropoxy)phenyl)pyrimidin-2-yl)piperidin-4-carboxylate (650 mg, 1.61 mmol) and NaOH (321 mg, 8.03 mmol) were added to MeOH (10 mL) and H₂O (2 mL). The mixture was stirred to react at room temperature for 3 hours. The mixture was adjusted to pH of 3 with hydrochloric acid (2N concentration) at 0 °C. The reaction solution was diluted with dichloromethane and washed with saturated brine. The organic phase was dried over anhydrous sodium sulfate, and filtered. The filtrate was concentrated under reduced pressure to finally obtain 532 mg of 1-(4-(2-fluoro-5-(3-hydroxypropoxy)phenyl)pyrimidin-2-yl)piperidin-4-carboxylic acid with a yield of 87.92%; MS:376[M+H]⁺;

Step 4): 1-(4-(2-fluoro-5-(3-hydroxypropoxy)phenyl)pyrimidin-2-yl)piperidin-4-carboxylic acid (190 mg, 0.5mmol), intermediate A-1 (5-(3,5-difluorophenyl)-4,5-dihydro-1H-pyrazole) (100mg, 0.55 mmol), HATU(190 mg, 0.5 mmol) and DIEA (129 mg, 1 mmol) were added to DMF (3 mL). The mixture was reacted at 25 °C for 2 hours under argon protection. The reaction solution was diluted with dichloromethane and washed with saturated brine. The organic phase was dried over anhydrous sodium sulfate, and filtered. The filtrate was concentrated under reduced pressure. The obtained product was further purified by column chromatography to finally obtain 78 mg of the product, with a yield of 29%. ¹H NMR (400 MHz, DMSO-*d*₆) δ 8.44 (d, *J* = 5.1 Hz, 1H), 7.48 (d, *J* = 6.2 Hz, 1H), 7.32 - 7.19 (m, 2H), 7.18 - 7.05 (m, 2H), 7.00 (d, *J* = 5.1 Hz, 1H), 6.88 - 6.81 (m, 2H), 5.34 (dd, *J* = 12.0, 5.0 Hz, 1H), 4.75 (d, *J* = 13.1 Hz, 2H), 4.60(t, *J* = 5.0 Hz, 1H),4. 08 (t, *J* = 6.3 Hz, 2H), 3.55 (q, *J* = 6.4 Hz, 2H), 3.51 - 3.35 (m, 2H), 3.04 (td, *J* = 12.5, 9.7 Hz, 2H), 2.75 (dd, *J* = 19.0, 5.0 Hz, 1H), 1.98 - 1.76 (m, 4H), 1.56 - 1.49 (m, 2H). MS: 540 [M+H]⁺.

### Example 39: (5-(3,5-difluorophenyl)-4,5-dihydro-1H-pyrazol-1-yl)(1-(4-(2-fluoro-5-(3-hydroxy-3-methylbutoxy)phenyl)pyrimidine)-2-yl)piperidin-4-yl)methanone

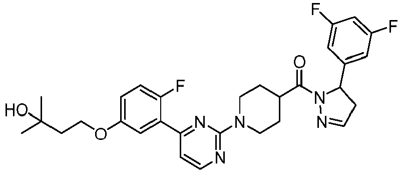

The preparation was carried out in a similar manner to Example 38, except that in step 1, intermediate B-13 was used instead of intermediate B-12. ¹H NMR (400 MHz, DMSO-*d*₆) δ 8.44 (d, *J* = 5.1 Hz, 1H), 7.49 (d, *J* = 6.2 Hz, 1H), 7.31 - 7.21 (m, 2H), 7.17 - 7.04 (m, 2H), 7.00 (d, *J* = 5.1 Hz, 1H), 6.88 - 6.80 (m, 2H), 5.34 (dd, *J* = 12.0, 5.0 Hz, 1H), 4.79 - 4.71 (m, 2H), 4.41 (s, 1H), 4.13 (t, *J* = 7.2 Hz, 2H), 3.55 - 3.35 (m, 2H), 3.04 (td, *J* = 12.5, 8.5 Hz, 2H), 2.75 (dd, *J* = 19.1, 5.0 Hz, 1H), 1.97 - 1.75 (m, 4H), 1.51 (dd, *J* = 17.4, 12.0 Hz, 2H), 1.16 (s, 6H). MS:568[M+H]⁺.

### Separation of stereoisomers

The racemates obtained in Examples 18, 21, 23 and 24 were separated and purified by chiral columns. The separation was performed using a chromatograph (Shimadzu LC-20A) under the following separation conditions:

| Column | : CHIRALPAK IC |
|---|---|
| Column size | : 0.46 cm I.D. × 15 cm L |
| Injection volume | : 5.0 uL |
| Mobile phase | : n-Hexane/EtOH=60/40(V/V) |
| Flow rate | : 1.0mL/min |
| Wavelength | : UV254nm |
| Temperature | : 25 °C |

Of the two peaks separated, the one with the longer retention time was collected and the solvent was removed by rotary evaporation to obtain the pure enantiomer. The absolute configuration of the above active enantiomer was designated as (S).

| Racemate | Retention time (min) | S stereoisomer |
|---|---|---|
| 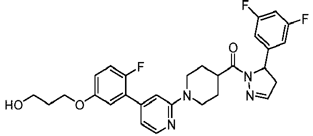 Example 18 | 6.68 | 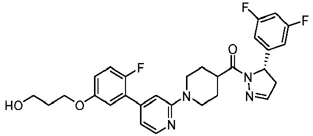 Example 40 |
| 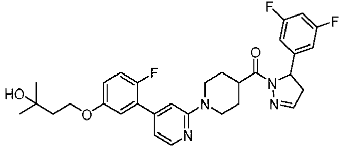 Example 21 | 4.75 | 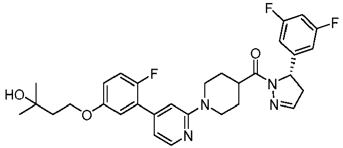 Example 41 |

| | | |
|---|---|---|
| 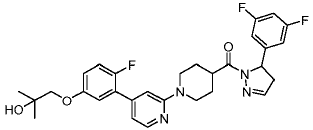 Example 23 | 5.71 | 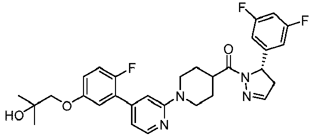 Example 42 |
| 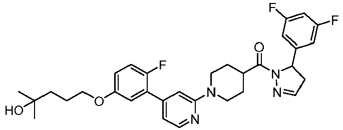 Example 24 | 4.96 | 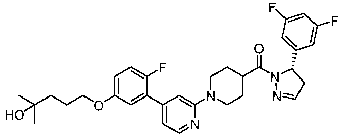 Example 43 |

### Example 44: [5-(3,5-difluorophenyl)-4,5-dihydro-1H-pyrazol-1-yl]-[1-[4-[5-(2-ethyl-2-hydroxybutoxy)-2-fluorophenyl]pyridin-2-yl]piperidin-4-yl]methanone

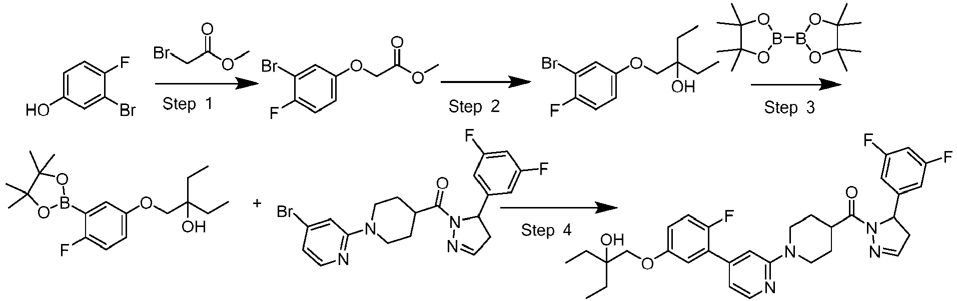

Step 1): Methyl 2-bromoacetate (14.42 g, 94.24 mmol), 3-bromo-4-fluorophenol (6 g, 31.41 mmol), and potassium carbonate (13.03 g, 94.24 mmol) were placed in DMF (30 mL). The mixture was reacted at 80 °C for 12 hours under argon protection. The reaction solution was diluted with ethyl acetate, and then washed with water and saturated brine. The organic phase was dried over anhydrous sodium sulfate, and then filtered. The filtrate was concentrated under reduced pressure, and the crude product was purified by column chromatography to finally obtain 6 grams of methyl 2-(3-bromo-4-fluoro-phenoxy)acetate with a yield of 73%.

Step 2): Methyl 2-(3-bromo-4-fluoro-phenoxy)acetate (1 g, 3.80 mmol) was dissolved in tetrahydrofuran. Ethylmagnesium bromide (2.0M in tetrahydrofuran) (5.7 mL) was added dropwise at 0°C under argon protection. The mixture was reacted at this temperature for 2 hours. Saturated aqueous ammonium chloride solution was added to quench the reaction. The reaction solution was filtered through diatomaceous earth by suction, and the filtrate was extracted with ethyl acetate. The organic phase was washed with water and saturated brine, then dried over anhydrous sodium sulfate, and filtered. The filtrate was concentrated under reduced pressure, and the crude product was purified by column chromatography to obtain 800 mg of 3-[(3-bromo-4-fluoro-phenoxy)methyl]pentan-3-ol, with a yield of 72%.

Step 3): 3-[(3-bromo-4-fluoro-phenoxy)methyl]pentan-3-ol (800 mg, 2.75 mmol), pinacol ester (907.06 mg, 3.57 mmol), Pd(dppf)Cl₂ (120.63 mg, 164.86 µmol) and potassium acetate (539.31 mg, 5.50 mmol) were dissolved in 1,4-dioxane (15 mL). Under argon protection, the mixture was slowly heated to 100 °C and reacted for 8 hours. The mixture was cooled to room temperature. The reaction solution was filtered through diatomaceous earth by suction, diluted with ethyl acetate, washed with water and saturated brine, dried over anhydrous sodium sulfate, and filtered. The filtrate was concentrated under reduced pressure to remove the solvent. The crude product was purified by column chromatography to obtain 800 mg of 3-[[4-fluoro-3-(4,4,5,5-tetramethyl-1,3,2-dioxaborolane-2-yl)phenoxy]methyl]pentan-3-ol with a yield of 90%.

Step 4): (1-(4-bromopyridin-2-yl)piperidin-4-yl)(5-(3,5-difluorophenyl)-4,5-dihydro-1H-pyrazol-1-yl)methanone (100 mg, 222.57 µmol), 3-[[4-fluoro-3-(4,4,5,5-tetramethyl-1,3,2-dioxaborolane-2-yl)phenoxy]methyl]pentan-3-ol (112.92 mg, 333.86 µmol), potassium carbonate (61.52 mg, 445.15 µmol), and Pd(dppf)Cl₂ (16.29 mg, 22.26 µmol) were dissolved in a mixed solvent of 1,4-dioxane (5 mL) and water (0.5 mL). The mixture was slowly heated to 100 °C under argon protection to react for 8 hours. The reaction solution was diluted with ethyl acetate and washed with water and saturated brine. The organic phase was dried over anhydrous sodium sulfate, and filtered. The filtrate was concentrated under reduced pressure to remove the solvent. The product was purified by silica gel column chromatography to finally obtain 40 mg of [5-(3,5-difluorophenyl)-4,5-dihydro-1H-pyrazol-1-yl]-[1-[4-[5-(2-ethyl-2-hydroxybutoxy)-2-fluorophenyl]pyridin-2-yl]piperidin-4-yl]methanone with a yield of 31%.

¹H NMR (400 MHz, DMSO-*d*₆) δ 8.16 (d, *J* = 5.1 Hz, 1H), 7.27 - 7.18 (m, 2H), 7.17 - 6.97 (m, 3H), 6.93 (s, 1H), 6.89 - 6.80 (m, 2H), 6.76 (d, *J* = 5.2 Hz, 1H), 5.34 (dd, *J* = 12.0, 4.9 Hz, 1H), 4.44 - 4.32 (m, 3H), 3.77 (s, 2H), 3.48 (dd, *J* = 18.8, 12.1 Hz, 1H), 3.37 (dd, *J* = 11.7, 3.9 Hz, 1H), 3.00 - 2.87 (m, 2H), 2.80 - 2.66 (m, 1H), 1.89 (d, *J* = 13.3 Hz, 1H), 1.75 (d, *J* = 12.8 Hz, 1H), 1.59 - 1.47 (m, 6H), 0.83 (t, *J* = 7.5 Hz, 6H). MS: 581[M+H]⁺.

### Example 45: [5-(3,5-difluorophenyl)-4,5-dihydro-1H-pyrazol-1-yl]-[1-[4-[2-fluoro-5-[(1-hydroxycyclopropyl)methoxy]phenyl]pyridin-2-yl]piperidin-4-yl]methanone

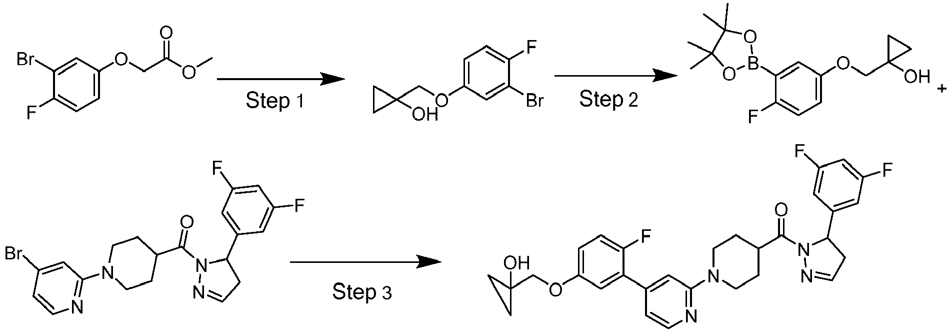

Step 1): Methyl 2-(3-bromo-4-fluoro-phenoxy)acetate (500 mg, 1.80 mmol) was dissolved in THF (8 mL). Ethylmagnesium bromide (2M in tetrahydrofuran, 0.9 ml, 1.80 mmol) was added dropwise under argon protection at 0°C. The mixture was reacted at this temperature for 30 minutes, then slowly heated to 25°C and reacted for another 16 hours. Saturated aqueous ammonium chloride solution was added to quench the reaction. The mixture was filtered. The filtrate was extracted with dichloromethane. The organic phase was washed with saturated brine, dried over anhydrous sodium sulfate, and filtered. The filtrate was concentrated under reduced pressure, and purified by silica gel column chromatography to finally obtain 180 mg of 1-[(3-bromo-4-fluoro-phenoxy)methyl]cyclopropanol with a yield of 38 %.

Step 2): 1-[(3-bromo-4-fluoro-phenoxy)methyl]cyclopropanol (180 mg, 689.42 µmol), pinacol ester (370.63 mg, 1.46 mmol), potassium acetate (238.73 mg, 2.43 mmol), and Pd(dppf)Cl₂ (88.99 mg, 121.63 µmol) were placed in 1,4-dioxane (10 mL). The mixture was reacted at 100 °C for 3 hours under argon protection. The reaction solution was concentrated and purified by column chromatography to finally obtain 170 mg of 1-[4-fluoro-3-(4,4,5,5-tetramethyl-1,3,2-dioxaborolane-2-yl)phenoxy]-2-methyl-cyclopropan-2-ol with a yield of 80 %.

Step 3): (1-(4-Bromopyridin-2-yl)piperidin-4-yl)(5-(3,5-difluorophenyl)-4,5-dihydro-1H-pyrazol-1-yl)methanone (80 mg, 178.06 µmol), 1-[4-fluoro-3-(4,4,5,5-tetramethyl-1,3,2-dioxaborolane-2-yl)phenoxy]-2-methyl-cyclopropan-2-ol (71.33 mg, 231.48 µmol), potassium carbonate (49.22 mg, 356.12 µmol), and Pd(dppf)Cl₂ (13.03 mg, 17.81 µmol) were dissolved in a mixed solvent of 1,4-dioxane (3 mL) and water (0.4 mL). Under argon protection, the mixture was slowly heated to 80 °C and reacted for 6 hours. The reaction solution was diluted with ethyl acetate, washed with saturated brine, dried over anhydrous sodium sulfate, and filtered. The filtrate was concentrated under reduced pressure to remove the solvent. The crude product was purified by column chromatography to finally obtain 28 mg of [5-(3,5-difluorophenyl)-4,5-dihydro-1H-pyrazol-1-yl]-[1-[4-[2-fluoro-5-[(1-hydroxycyclopropyl)methoxy]phenyl]pyridin-2-yl]piperidin-4-yl]methanone with a yield of 29%. ¹H NMR (400 MHz, DMSO-*d*₆) δ 8.15 (d, *J* = 5.3 Hz, 1H), 7.30 - 7.20 (m, 2H), 7.18 - 7.05 (m, 2H), 7.03 - 6.94 (m, 2H), 6.89 - 6.76 (m, 3H), 5.34 (dd, *J* = 12.0, 5.0 Hz, 1H), 4.90 (s, 2H), 4.38 (d, *J* = 13.0 Hz, 2H), 3.55 - 3.32 (m, 2H), 2.99 (d, *J* = 12.0 Hz, 2H), 2.75 (dd, *J* = 19.0, 5.0 Hz, 1H), 2.55 (d, *J* = 7.3 Hz, 1H), 1.91 (d, *J* = 12.9 Hz, 1H), 1.76 (d, *J* = 13.1 Hz, 1H), 1.63 - 1.46 (m, 2H), 0.97 (t, *J* = 7.3 Hz, 4H). MS: 551[M+H]⁺.

### Example 46: (5-(3,5-difluorophenyl)-4,5-dihydro-1H-pyrazol-1-yl)(1-(4-(2-fluoro-5-((1-hydroxycyclobutyl)methoxy)phenyl)pyridin-2-yl)piperidin-4-yl)methanone

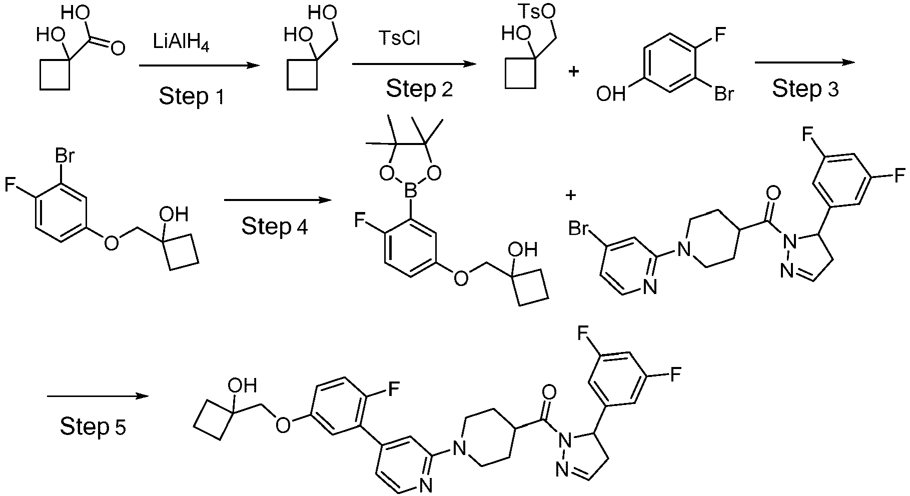

Step 1): 1-Hydroxycyclobutanecarboxylic acid (500 mg, 4.31 mmol) was dissolved in THF (10 mL). Under argon protection, lithium aluminum hydride (490.25 mg, 12.92 mmol) was added in batches at room temperature. The mixture was heated to 80 °C and reacted for another 1 hour. In an ice bath, 0.5 ml of water, 1.5 mL of 20% NaOH aqueous solution, and 0.5 mL of water were added in sequence. The reaction mixture was quenched, and filtered. The filtrate was collected, and concentrated under reduced pressure to finally obtain 400 mg of crude 1-(hydroxymethyl)cyclobutanol with a yield of 91%.

Step 2): 1-(Hydroxymethyl)cyclobutanol (400 mg, 3.92 mmol), p-toluenesulfonyl chloride (1.12 g, 5.87 mmol), triethylamine (1.19 g, 11.75 mmol, 1.64 mL), and DMAP (47.85 mg, 391.65 µmol) were placed in dichloromethane (20 mL). The mixture was reacted at 25 °C for 16 hours under argon protection. Saturated aqueous sodium carbonate solution was added to the reaction solution. The mixture was stirred for 30 minutes, and then extracted three times with ethyl acetate. The organic phase was washed with saturated brine, dried over anhydrous sodium sulfate, and filtered. The filtrate was concentrated under reduced pressure, and the crude product was purified by column chromatography to obtain 430 mg of (1-hydroxycyclobutyl)methyl 4-p-toluenesulfonate with a yield of 43%.

Step 3): (1-hydroxycyclobutyl)methyl 4-p-toluenesulfonate (430 mg, 1.68 mmol), 3-bromo-4-fluoro-phenol (106.81 mg, 559.20 µmol), and potassium carbonate (231.86 mg, 1.68 mmol) were added to DMF (10 mL). The mixture was heated to 80 °C and stirred to react for 16 hours under argon protection. Water was added to the reaction system, and the mixture was extracted three times with ethyl acetate. The organic phase was washed with saturated brine, dried over anhydrous sodium sulfate, and filtered. The filtrate was concentrated under reduced pressure, and purified by column chromatography to finally obtain 150 mg of 1-[(3-bromo-4-fluoro-phenoxy)methyl]cyclobutanol with a yield of 98%.

Step 4): 1-[(3-bromo-4-fluoro-phenoxy)methyl]cyclobutanol (150 mg, 545.23 µmol), 4,4,5,5-tetramethyl-2-(4,4,5,5-tetramethyl-1,3,2-dioxaborolane-2-yl)-1,3,2-dioxaborolane (179.99 mg, 708.80 µmol), Pd(dppf)Cl₂ (23.94 mg, 32.71 µmol), and potassium acetate (107.02 mg, 1.09 mmol) were dissolved in 1,4-dioxane (10 mL). The mixture was slowly heated to 100 °C and reacted for 8 hours under argon protection. The reaction solution was cooled to room temperature and filtered with diatomaceous earth by suction. The filtrate was diluted with ethyl acetate, washed with water and saturated brine, dried over anhydrous sodium sulfate, and filtered. The filtrate was concentrated under reduced pressure to remove the solvent. The crude product was purified by column chromatography (eluent: PE:EA=3:1) to finally obtain 1-[[4-fluoro-3-(4,4,5,5-tetramethyl-1,3,2-dioxaborolane-2-yl)phenoxy]methyl]cyclobutanol (150 mg, 486.77 µmol, yield: 89.28%).

Step 5): (1-(4-bromopyridin-2-yl)piperidin-4-yl)(5-(3,5-difluorophenyl)-4,5-dihydro-1H-pyrazol-1-yl)methanone (100 mg, 222.57 µmol), 1-[[4-fluoro-3-(4,4,5,5-tetramethyl-1,3,2-dioxaborolane-2-yl)phenoxy]methyl]cyclobutanol (107.56 mg, 333.86 µmol), potassium carbonate (61.52 mg, 445.15 µmol), and Pd(dppf)Cl₂ (16.29 mg, 22.26 µmol)were dissolved in a mixed solvent of 1,4-dioxane (5 mL) and water (0.5 mL). The mixture was slowly heated to 100 °C and reacted for 8 hours under argon protection. The reaction solution was diluted with ethyl acetate, washed with water and saturated brine, dried over anhydrous sodium sulfate, and filtered. The filtrate was concentrated under reduced pressure to remove the solvent, and purified by column chromatography to give (5-(3,5-difluorophenyl)-4,5-dihydro-1H-pyrazol-1-yl)(1-(4-(2-fluoro-5-((1-hydroxycyclobutyl)methoxy)phenyl)pyridin-2-yl)piperidin-4-yl)methanone (40 mg, yield 32%). ¹H NMR (400 MHz, DMSO-*d*₆) δ 8.16 (d, *J* = 5.1 Hz, 1H), 7.29 - 7.19 (m, 2H), 7.17 - 7.00 (m, 3H), 6.94 (s, 1H), 6.88 - 6.74 (m, 3H), 5.34 (dd, *J* = 12.0, 5.0 Hz, 1H), 5.25 (s, 1H), 4.40 (dd, *J* = 13.4, 3.7 Hz, 2H), 3.95 (s, 2H), 3.48 (dd, *J* = 19.1, 12.0 Hz, 1H), 3.40 - 3.35 (m, 1H), 3.01 - 2.87 (m, 2H), 2.74 (dd, *J* = 18.9, 4.9 Hz, 1H), 2.11 (t, *J* = 9.1 Hz, 2H), 2.00 (dt, *J* = 12.5, 9.5 Hz, 2H), 1.89 (d, *J* = 13.0 Hz, 1H), 1.75 (d, *J* = 12.9 Hz, 1H), 1.66 - 1.49 (m, 4H). MS: 565[M+H]⁺.

### Example 47: (5-(3,5-difluorophenyl)-4,5-dihydro-1H-pyrazol-1-yl-5-deuterium)(1-(4-(2-fluoro-5-(2-hydroxy-2-methylpropoxy)phenyl)pyridin-2-yl)piperidin-4-yl)methanone

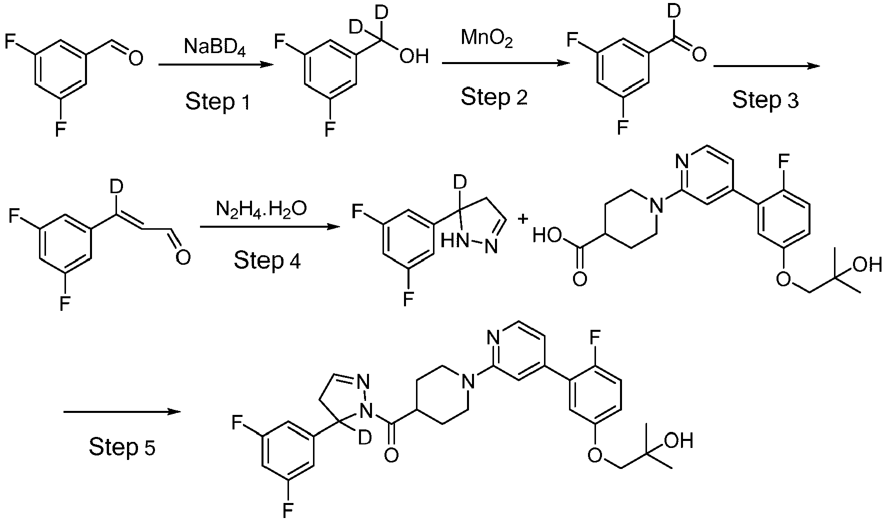

Step 1): 3,5-difluorobenzaldehyde (800 mg, 5.63 mmol) was placed in THF (18 mL). NaBH₄ (354 mg) was added to the solution in batches at 0°C, and the mixture was reacted at 0°C for 2 hours. The reaction solution was quenched with 0.5 mL of acetone and concentrated under reduced pressure. The residue was purified by silica gel column to finally obtain 800 mg of dideuterated-(3,5-difluorophenyl)methanol with a yield of 97.24%.

Step 2): Dideuterated-(3,5-difluorophenyl)methanol (800 mg, 5.47 mmol) was placed in dichloromethane (54 mL), and MnO₂ (2.38 g) was added to the solution. The mixture was reacted at 25°C for 20 hours. The reaction solution was filtered and the filtrate was collected and concentrated under reduced pressure to finally obtain 600 mg of crude deuterated 3,5-difluorobenzaldehyde with a yield of 77%.

Step 3): Deuterated 3,5-difluorobenzaldehyde (143 mg, 999.24 µmol) and acetaldehyde (66.03 mg, 1.50 mmol) were placed in water. 1N aqueous NaOH solution (1.1 mL) was added dropwise to the reaction solution at 0°C under argon. The mixture was reacted at 25°C for 16 hours. TLC showed that there was no raw material. Water was added to the reaction solution, and the mixture was extracted three times with ethyl acetate. The organic phase was washed with saturated brine, dried over anhydrous sodium sulfate, and filtered. The filtrate was concentrated under reduced pressure, and purified by column chromatography (PE:EA=20:1) to finally obtain 60 mg of (E)-3-deuterium-3-(3,5-difluorophenyl)prop-2-enal with a yield of 36%.

Step 4): Hydrazine hydrate (17.05 mg, 532.08 µmol) was added to ethanol (2 mL), and acetic acid (36.21 mg, 603.03 µmol, 34.49 µL) was added with stirring. The mixture was heated to 40°C, and (E)-3-deuterium-3-(3,5-difluorophenyl)prop-2-enal (60 mg, 354.72 µmol) was slowly added dropwise. The mixture was sealed and heated at 80°C to react overnight. The reaction solution was concentrated and purified on a silica gel plate (PE/EtOAc=3:1) to obtain 20 mg of the product 5-deuterium-5-(3,5-difluorophenyl)-4,5-dihydropyrazole, with a yield of 31%.

Step 5): 1-[4-[2-fluoro-5-(2-hydroxy-2-methyl-propoxy)phenyl]-2-pyridinyl]piperidinyl-4-carboxylic acid (178.13 mg, 458.58 µmol), 5-deuterium-5-(3,5-difluorophenyl)-4,5-dihydropyrazole (70 mg, 382.15 µmol), HATU (174.36 mg, 458.58 µmol), and DIEA (148.17 mg, 1.15 mmol, 199.68 µL) were placed in DMF (5 mL). The mixture was reacted at 25 °C for 16 hours under argon protection. The reaction solution was diluted with dichloromethane and washed with saturated brine. The organic phase was dried over anhydrous sodium sulfate, and filtered. The filtrate was concentrated under reduced pressure. The product was further purified by preparative HPLC to finally obtain 50 mg of (5-(3,5-difluorophenyl)-4,5-dihydro-1H-pyrazol-1-yl-5-deuterium)(1-(4-(2-fluoro-5-(2-hydroxy-2-methylpropoxy)phenyl)pyridin-2-yl)piperidin-4-yl)methanone with a yield of 24%.

¹H NMR (400 MHz, DMSO-*d*₆) δ 8.16 (d, *J* = 5.1 Hz, 1H), 7.28 - 7.18 (m, 2H), 7.17 - 6.97 (m, 3H), 6.93 (s, 1H), 6.88 - 6.80 (m, 2H), 6.85 - 6.73 (m, 1H), 4.63 (s, 1H), 4.44 - 4.36 (m, 2H), 3.76 (s, 2H), 3.48 (dd, *J* = 19.1, 1.7 Hz, 1H), 3.37 (dt, *J* = 7.5, 3.6 Hz, 1H), 3.01 - 2.87 (m, 2H), 2.74 (dd, *J* = 19.0, 1.8 Hz, 1H), 1.90 (d, *J* = 12.9 Hz, 1H), 1.76 (d, *J* = 12.9 Hz, 1H), 1.65 - 1.44 (m, 2H), 1.21 (s, 6H). MS: 554[M+H]⁺.

### Synthesis of 1-[4-[2-fluoro-5-(2-hydroxy-2-methyl-propoxy)phenyl]-2-pyridyl]piperidinyl-4-carboxylic acid:

### (I) Synthesis of 1-[4-fluoro-3-(4,4,5,5-tetramethyl-1,3,2-dioxaborolane-2-yl)phenoxy]-2-methyl-propan-2-ol

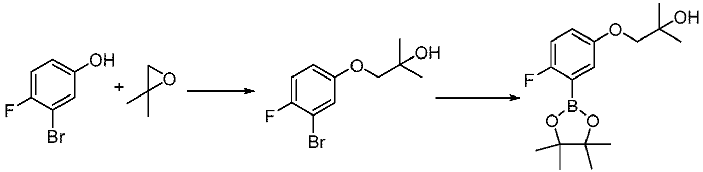

Step 1: 3-bromo-4-fluoro-phenol (5 g, 26.18 mmol), 2,2-dimethyloxirane (5.66 g, 78.54 mmol), and potassium carbonate (10.85 g, 78.54 mmol) were placed in DMF (50 mL). The mixture was reacted at 100 ° C for 16 hours under argon protection. The reaction solution was diluted with dichloromethane and washed with saturated brine. The organic phase was dried over anhydrous sodium sulfate, and filtered. The filtrate was concentrated under reduced pressure to finally obtain 1-(3-bromo-4-fluoro-phenoxy)-2-methyl-propan-2-ol (5 g, 19.00 mmol, with a yield of 72.6%).

Step 2: 1-(3-bromo-4-fluoro-phenoxy)-2-methyl-propan-2-ol (30 g, 114.02 mmol), pinacol ester (34.75 g, 136.83 mmol), potassium acetate (22.38 g, 228.05 mmol), and Pd(dppf)Cl₂ (4.17 g, 5.70 mmol) were placed in 1,4-dioxane (300 mL). The mixture was reacted at 90 °C for 16 hours under argon protection. The reaction solution was filtered. The filtrate was concentrated under reduced pressure, and the crude product was purified by column chromatography to finally obtain 1-[4-fluoro-3-(4,4,5,5-tetramethyl-1,3,2-dioxaborolane-2-yl)phenoxy]-2-methyl-propan-2-ol (25 g, 80.60 mmol, with a yield of 70.7%).

### (II) Synthesis of methyl 1-(4-bromo-2-pyridyl)piperidinyl-4-carboxylate

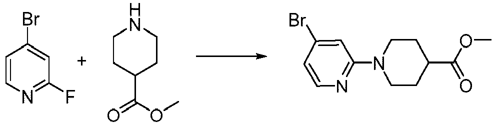

2-fluoro-4-bromo-pyridine (3.52 g, 20 mmol) and methyl piperidinyl-4-carboxylate (2.86 g, 20.00 mmol) were dissolved in DMF (50 mL), and triethylamine (4.05 g, 40.00 mmol, 5.58 mL) was added. The mixture was heated to 80 °C for 3 hours. The reaction solution was cooled to room temperature, diluted with water and extracted twice with ethyl acetate. The organic phases were combined, washed three times with saturated brine, then dried and concentrated. The residue was purified by silica gel column chromatography to obtain the product methyl 1-(4-bromo-2-pyridinyl)piperidinyl-4-carboxylate (5.1 g, 17.05 mmol, yield 85.2%).

### (III) Synthesis of 1-[4-[2-fluoro-5-(2-hydroxy-2-methyl-propoxy)phenyl]-2-pyridinyl] piperidinyl-4-carboxylic acid

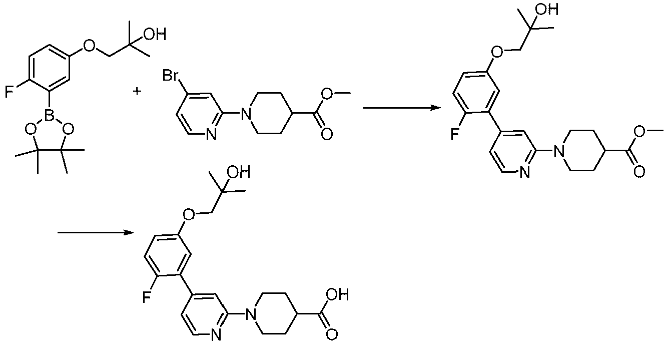

Step 1: 1-[4-Fluoro-3-(4,4,5,5-tetramethyl-1,3,2-dioxaborolan-2-yl)phenoxy]-2-methyl-propan-2-ol (372.20 mg, 1.2 mmol) and methyl 1-(4-bromo-2-pyridyl)piperidinyl-4-carboxylate (299.16 mg, 1.00 mmol) were dissolved in 1,4-dioxane (10 mL). Pd(dppf)Cl₂ (878.04 mg, 1.20 mmol) was added, and the atmosphere was replaced with nitrogen. The mixture was heated to 80°C and reacted for 1 hour. The system was cooled to room temperature, and filtered with diatomaceous earth. The filtrate was concentrated to obtain a crude product. The crude product was purified on a silica gel plate to obtain methyl 1-[4-[2-fluoro-5-(2-hydroxy-2-methyl-propoxy)phenyl]-2-pyridinyl]piperidinyl-4-carboxylate (400 mg, 993.89 µmol, yield 82.8%).

Step 2: Methyl 1-[4-[2-fluoro-5-(2-hydroxy-2-methyl-propoxy)phenyl]-2-pyridinyl]piperidinyl-4-carboxylate (400 mg, 993.89 µmol) was dissolved in MeOH (5 mL). LiOH.H₂O (83.41 mg, 1.99 mmol) was added. The mixture was reacted at room temperature for 1 hour. The reaction solution was concentrated, and water was added. The mixture was adjusted to pH of 6 with hydrochloric acid. A precipitated solid was collected, and dried to obtain the product 1-[4-[2-fluoro-5-(2-hydroxy-2-methyl-propoxy)phenyl]-2-pyridinyl]piperidiny1-4-carboxylic acid (350 mg, 901.06 µmol, yield 90.66%).

### Reference Examples

With reference to the contents disclosed in WO2018/092089, the following two compounds were synthesized as controls.

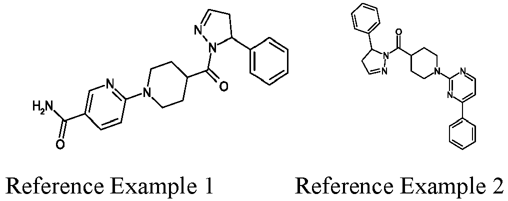

### Assay Example 1. Western blot detection of inhibition of the compound on RIPK1 phosphorylation in HT29 cells

In this assay, Western blot was used to detect the effect of the test compound on intracellular RIPK1 and its phosphorylation during the process of inducing programmed necrosis of HT29 cells to verify the inhibitory effect of the compound on RIPK1 phosphorylation.

### I. Main Assay Reagents/Instruments

**Table 3. Main reagents and instruments used in Assay Example 1**

| Name | Item No. | Brand |
|---|---|---|
| McCoy's 5A | SH30200.01 | Hyclone |
| BCA Protein Concentration Assay Kit | P0012 | Beyotime |
| HT29 cells | HTB-38 | ATCC |
| Phospho-RIP (Ser166) (D1L3S) Rabbit mAb | 65746s | CST |
| RIP (D94C12) XP Rabbit mAb | 3493s | CST |
| Anti-rabbit IgG (H+L) (DyLight 680 Conjugate) | 5366s | CST |
| Anti-rabbit IgG (H+L) (DyLight 800 4X PEG Conjugate) | 5151s | CST |
| P-Actin(8H10D10)Mouse mAb | 3700s | CST |
| Precision Plus ProteinTM Dual Color Standards | 1610374 | Bio-Rad |
| 10%Mini-PROTEAN^{®} TGX Stain-Free^{™} Protein Gels | 4568034 | Bio-Rad |
| AT-406 (SM-406) | S2754 | Selleck chem |
| Z-VAD-FMK | S7023 | Selleck chem |
| Human TNF-α | 300-01A-10 | Peprotech |
| PowerPacTM Basic electrophoresis power supply | 1645050 | Bio-Rad |
| Mini-PROTEAN^{®} Tetra Electrophoresis Tank | 1658004 | Bio-Rad |
| Trans-Blot^{®} TurboTM Universal Protein | 1704155 | Bio-Rad |
| Transfer System | | |
| Odyssey CLX Infrared Fluorescence Scanning | CLX-1688 | LI-COR |
| Imaging System | | |

### II. Assay Procedure

1. Cell collection and protein sample extraction
   a) HT29 cells in good condition were collected, resuspended in McCoy's 5A complete medium and counted. The cells were seeded in a 12-wellplate at a cell density of 1×10⁶/1 mL/well, and placed in a 37°C cell culture incubator overnight.
   b) Z-VAD-FMK (at final concentration of 20 µM) was added to the above 12-well plate. After 30 minutes, the inducer (where the final concentration of TNF-α was 20 ng/mL, and the final concentration of AT-406 was 100 nM), and the test compound of different concentrations was then added, and incubated in a cell incubator at 37° C for 7 hours.
   c) Preparation of complete lysate: A tube of aliquoted RIPA lysate was thawed, and then phosphatase inhibitor and Roche protease inhibitor were added respectively (phosphatase inhibitor: RIPA=1:100; Roche protease inhibitor: RIPA=4:100); the mixture was placed on ice for later use;
   d) The cell culture medium supernatant was discarded, and the cells were washed twice with PBS. 100 µL of the prepared complete cell lysate was added to each well, and the cells were lysed on ice for 15 minutes with intermittent shaking during the lysis;
   e) After the lysis was completed, the lysate was transferred to a 1.5 mL centrifuge tube labeled in advance and centrifuged at 4°C, 14000 rpm for 20 minutes;
   f) The supernatant was transferred to a labeled 600 µL centrifuge tube, and placed on ice.
   g) The BCA kit was used for detection and the protein concentration was calculated;
2. The protein sample to be tested or pre-stained dual-color protein molecular weight standard was added to each well of the precast gel, and the protein loading amount was 40~50 ug/ well.
3. Electrophoresis was performed at a constant voltage of 100 V. When the pre-stained dual-color protein molecular weight standard band on the gel ran out of the lower edge of the lane, electrophoresis was stopped.
4. After the electrophoresis was over, the gel holder was removed. The short glass plate was gently pried up with a peeling spatula, and the upper layer of concentrated gel was discarded. The separation gel was gently peeled off and placed in the transfer buffer.
5. A moderately sized NC membrane was cut according to the size of the gel. The membrane was soaked with pure water, and then immersed in the pre-cooled transfer buffer; at the same time the filter paper was soaked in the transfer buffer solution.
6. The transfer cartridge was assembled in the order of filter paper, NC membrane, gel, and filter paper, and then put into the Trans-Blot^{®} Turbo; the instrument's built-in "Standard SD" program was called to complete the transfer.
7. The protein-loaded NC membrane was briefly rinsed with deionized water. The membrane was cut according to the molecular weight of the protein to be detected based on the marker band indication. Blocking solution was added to submerge the membrane, and the membrane was blocked for 1 hour at room temperature with slow shaking on a shaker.
8. After the blocking was completed, the NC membrane was put into an antibody incubation box, and the diluted primary antibody was added. The box was incubated with gentle shaking on a shaker at room temperature for 1 hour.
9. After the incubation was completed, the membrane was washed three times with TBST for 10 min each time.
10. The secondary antibody was selected according to the source of the primary antibody. The secondary antibody was diluted with the diluent of the secondary antibody at a ratio of 1:10000-1:15000. The secondary antibody was added into the antibody incubation box and the membrane was immersed therein. The box was incubated with gentle shaking on a shaker at room temperature for 1 hour.
11. After the incubation was completed, the membrane was washed three times with TBST for 10 min each time.
12. The membrane was rinsed once with pure water, and the membrane was scanned with the Odyssey CLX infrared fluorescence scanning imaging system to obtain an image. The fluorescence signal value of the band was read using the grayscale reading function of the system.

### III. Typical Assay Results and Analysis

The ratio of the target protein phosphorylation relative to the target protein expression was calculated according to the fluorescence intensity value, and then the normalized expression multiple of the phosphorylation of the target protein in the drug administration group was calculated based on the control group. The calculation method was as follows:
Normalized expression multiple of the phosphorylation of the target protein = A/B
A: Fluorescence intensity value of phosphorylation of the target protein in the drug administration group / fluorescence intensity value of target protein in the drug administration group
B: Fluorescence intensity value of phosphorylation of the target protein in the group without drug addition / fluorescence intensity value of target protein in the group without drug addition

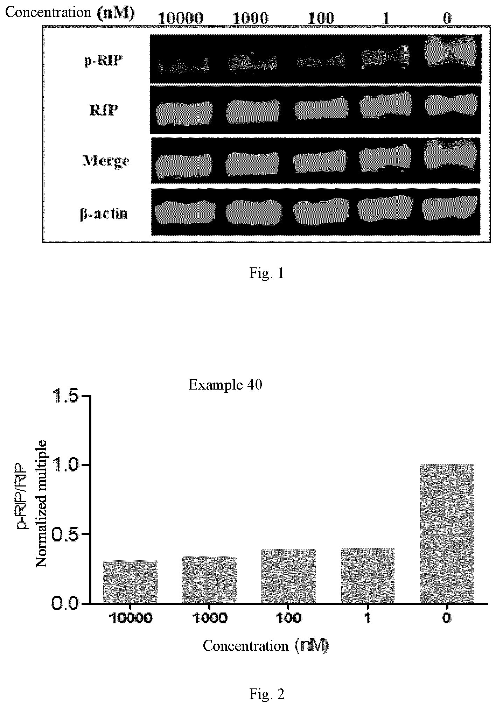

The compound of Example 40 was tested by the above method, and the results were shown in Figs. 1 and 2. Fig. 1 is a Western Blot image of RIPK1 total protein and phosphorylated protein at different concentrations of the compound, and Fig. 2 is the ratio of RIPK1 phosphorylation relative to RIPK1 total protein expression obtained by quantitative calculation based on the fluorescence intensity value of the bands in Fig. 1. The assay results show that the compound of Example 40 directly inhibits the phosphorylation activity of RIPK1 and shows a dose-dependent relationship, but has no obvious inhibitory effect on RIPK1.

### Assay Example 2. Assay method for compounds to inhibit cell necrosis (CCK8 method)

In this assay example 2, under the condition of inducing programmed cell necrosis of HT29 and L929 cell lines, different concentrations of the test compounds were added to test the IC₅₀ of the rescue effect of the test compounds on the programmed necrosis.

### I. Main assay reagents, instruments and materials

**Table 4. Main reagents and instruments used in Assay Example 2**

| Name | Brand | Item No./Name |
|---|---|---|
| DMSO | Sigma | D2650 (100 mL) |
| Trypan blue | GENIA Stock | G8003 |
| McCOY's 5A | Hylcone | SH30200.01 |
| MEM | Hyclone | SH30024.01 |
| Fetal bovine serum | Hyclone | SV30087.03 |
| Horse serum | GIBCO | 16050122 |
| CCK-8 | DOJINDO | CK04 |
| GSK2982772 | Selleckchem | S8484 |
| AT-406 | Selleckchem | S2754 |
| Z-VAD-FMK | Selleckchem | S7023 |
| Human TNF-α | PeproTech | 300-01A |
| Murine TNF-α | PeproTech | 315-01A) |
| HT29 | ATCC | HTB-38 |
| L929 | ATCC | CCL-1 |
| Multi-function plate reader | PerkinElmer | Envision |

**Compounds:** dissolved in DMSO to make a 10 mM solution;
**TNF-α:** initial concentration of 100 µg/mL;
**Z-VAD-FMK:** dissolved in DMSO to make a 10 mM solution;
**AT-406:** dissolved in DMSO to make a 10 mM solution.
**Cell lines:** HT29 cells were cultured in McCoy's 5A medium containing 10% fetal bovine serum, 100 U/mL penicillin, and 100 µg/mL streptomycin, and L929 cells were cultured in MEM medium containing 10% horse serum, 100 U/mL penicillin, and 100 µg/mL streptomycin.

### II. Specific assay methods:

1. The test compound was dissolved in DMSO to form a stock solution, and a gradient dilution was performed; the solution was then diluted with the corresponding culture medium to obtain a solution with 10 times the working concentration.
2. The cells in the logarithmic growth phase were diluted with culture medium to a specific cell concentration. 80 µL of the cell suspension was added to a 96-well plate, so that the cell densities of HT29 and L929 were 1.2*10⁴ cells/well and 2.0*10⁴ cells/well, respectively. The plate was cultured in a 37°C, 5% carbon dioxide incubator overnight.
3. 10 µL of the compound solution was added to each well of the 96-well plate inoculated with cells. The compounds to be tested were serially diluted 4-fold to obtain 9 concentrations in duplicate, starting from a maximum concentration of 10 µM. At the same time, a control group without compound addition was set up.
4. After the cells were cultured for another 30 minutes, 10 µL of the inducer mixture was added to each well. The final concentrations of Human TNF-α, AT-406 and Z-VAD-FMK in HT29 cells were 20 ng/mL, 1 µM and 20 µM, respectively; and the final concentrations of Murine TNF-α and Z-VAD-FMK in L929 cells were 20 ng/mL and 25 µM, respectively.
5. The 96-well plate in which compounds and inducer were added was placed in a 37°C, 5% carbon dioxide incubator for further culture, wherein HT29 cells were cultured for 16 hours and L929 cells were cultured for 4-8 hours. Cell viability was detected by CCK8. GraphPad Prism software was used to prepare a dose-effect curve and IC₅₀ was calculated.

Table 5 lists the results of the determination of some compounds of the present disclosure for inhibiting necrosis of HT29 and L929 cells, wherein A represents IC₅₀ less than or equal to 10 nM, B represents IC₅₀ greater than 10 nM but less than or equal to 50 nM, C represents IC₅₀ greater than 50 nM but less than or equal to 100 nM, D represents IC₅₀ greater than 100 nM but less than or equal to 400 nM, E represents IC₅₀ greater than 400 nM but less than or equal to 4000 nM, and F represents IC₅₀ greater than 4000 nM.

From the results shown in the table below, it can be found that the compounds of the present application exhibit very excellent inhibitory activities against both human HT29 cells and mouse L929 cells, wherein the inhibitory activities against human HT29 cells are better than those against mouse L929 cells, but the difference is small. However, the inhibitory activities of the compounds of Reference Examples 1 and 2 against human HT29 cells are significantly inferior to those of the compounds of the present application. Moreover, the compounds of Reference Examples 1 and 2 have almost no inhibitory activities against mouse L929 cells, and no meaningful test results were obtained.

**Table 5. Results of the determination of the inhibition of necrosis of HT29 cells by some compounds of the present disclosure**

| Example No. | HT29 IC₅₀ (nM) | L929 IC₅₀ (nM) | Example No. | HT29 IC₅₀ (nM) | L929 IC₅₀ (nM) |
|---|---|---|---|---|---|
| 1 | B | C | 23 | A | A |
| 2 | B | C | 24 | A | B |
| 3 | B | C | 25 | A | B |
| 4 | B | B | 26 | A | B |
| 5 | B | C | 27 | A | C |
| 6 | B | C | 28 | A | C |
| 7 | B | B | 29 | B | C |
| 8 | B | B | 30 | A | C |
| 9 | B | B | 31 | A | B |
| 10 | A | A | 32 | A | B |
| 11 | B | C | 33 | A | B |
| 12 | A | C | 34 | B | C |
| 13 | B | C | 35 | B | C |
| 14 | B | B | 36 | A | C |
| 15 | A | B | 37 | A | B |
| 16 | B | C | 38 | B | C |
| 17 | B | C | 39 | A | C |
| 18 | A | C | 40 | A | B |
| 19 | A | B | 41 | A | B |
| 20 | B | B | 42 | A | A |
| 21 | A | B | 43 | A | B |
| 22 | A | C | | | |
| 44 | A | B | 46 | A | B |
| 45 | A | B | 47 | B | B |
| Reference Example 1 | E | F | Reference Example 2 | E | F |

### Assay example 3. Assay of pharmacokinetics of small molecule compounds

In this assay, the pharmacokinetic characteristics and ability to penetrate the blood-brain barrier of the compounds of the present application were assayed after administration of some of the compounds of the present application to SD rats, CD-1 mice or beagle dogs by single oral administration and intravenous injection.

### (i) Reagents, instruments and animals used

**Table 6. Assay reagents**

| Reagent | Supplier | Cat. No. | Lot No. |
|---|---|---|---|
| DMSO | Innochem | D3851 | INAH03010 |
| Kolliphor^{®}HS 15 (Solutol) | SIGMA | 42966 | BCBZ6212 |
| Acetonitrile | Fisher | 75-05-8 | 186342 |
| Formic acid | DIKMA | 64-18-6 | 2486917 |
| Ammonium acetate | SIGMA | 431311 | MKCD5084 |
| Ultra-pure water | Manufactured in-house | - | - |

**Table 7. Assay instruments**

| Instrument | Brand | Model |
|---|---|---|
| Liquid chromatography-mass spectrometry (LC-MS) | AB Sciex | 5500 Q-trap |
| High speed refrigerated centrifuge | Thermo | ST 40R |
| Balance (centi milli-) | Mettler | XPE205 |
| Single well pipette | Eppendorf | Research plus series |
| Electric 12-hole pipette | Thermo | Novus series |

**Table 8. Animals used in the assay**

| | | | |
|---|---|---|---|
| Animal & strain: | SD Rat (Male) | CD-1 mice (male) | Beagle (male) |
| Animal grade: | SPF grade | SPF grade | General |
| Animal source: | Beijing HFK BioSCIENCE Co., Ltd | Beijing HFK BioSCIENCE Co., Ltd | Beijing Marshall Biotechnology Co., Ltd. |
| Weight range, age: | 180 g-350 g on the first day of administration, 8 weeks old (mainly based on body weight) | 20g-30g on the first day of administration, 8 weeks old (mainly based on body weight) | 6-15kg on the first day of administration, 6-12 months old (mainly based on body weight) |

### (ii) Preparation of sample formulation

1. Intravenous (IV) injection group: an appropriate amount of the assay compound was weighed, and completely dissolved in an appropriate volume of vehicle. The mixture was stirred, vortexed and/or sonicated. Once a solution was obtained, the vehicle was gradually added to a final volume to reach the target concentration. The solution was vortexed, and sonicated to obtain a homogeneous solution. The solution was filtered through a 0.22 µm PVDF membrane.
2. Oral (PO) group: an appropriate amount of the assay compound was weighed, and completely dissolved in an appropriate volume of vehicle. The mixture was stirred, vortexed and/or sonicated. Once a solution was obtained, the vehicle was gradually added to a final volume to reach the target concentration. The solution was vortexed, and sonicated to obtain a homogeneous solution.

**Table 9. Pharmacokinetic studies of test compounds and vehicle preparation**

| Compound | Assay | Administration vehicle in an IV/PO group |
|---|---|---|
| Example 18 | Rat PK&BBB | IV/PO: DMSO/Solutol/H₂O=5/10/85 |
| Example 18 | Beagle PK | IV: 0.9%NaCl +3 molar maleic acid |
| | | PO: Solutol/H₂O=10/90 |
| Example 40 | Mouse PK&BBB | IV/PO: DMSO/Solutol/H₂O=5/10/85 |
| Example 40 | Rat PK&BBB | IV/PO: DMSO/Solutol/H₂O=5/10/85 |
| Example 42 | Rat PK&BBB | IV/PO: DMSO/Solutol/H₂O=5/10/85+2 molar maleic acid |
| Example 42 | Beagle PK | IV: 0.9%NaCl +2.5 molar hydrochloric acid |
| | | PO: Solutol/H₂O=5/95 |

### (iii) Administration and sampling

The animals were randomly divided into groups according to their body weights, and the weights of the animals in each group were comparable after grouping (not exceeding ±20% of the average body weight). Meanwhile, the IV group was not fasted and the PO group was fasted overnight (>12 hrs) and given food 2 hrs after drug administration. All animals had free access to water. The dosing regimen and pharmacokinetic sampling regimen are given in Table 10 and Table 11 below, respectively.

**Table 10-1. Rat PK Dosing Scheme for Example 18, Example 40 and Example 42**

| Group | Method of administration | Number of animals | Sample | Dose of administration (mg/kg) | Volume of administration (mL/kg) | Concentration of formulation (mg/mL) |
|---|---|---|---|---|---|---|
| 1 | IV | 3 | Whole blood | 3 | 1 | 3 |
| 2 | PO | 3 | Whole Blood | 30 | 10 | 3 |
| 3 | PO | 3 | Whole blood/ brain | 30 | 10 | 3 |

**Table 10-2. Mouse PK Dosing Scheme for Example 40**

| Group | Method of administration | Number of animals | Sample | Dose of administration (mg/kg) | Volume of administration (mL/kg) | Concentration of formulation (mg/mL) |
|---|---|---|---|---|---|---|
| 1 | IV | 3 | Whole blood | 2 | 5 | 0.4 |
| 2 | PO | 3 | Whole Blood | 20 | 10 | 2 |
| 3 | PO | 3 | Whole blood/brain | 20 | 10 | 2 |

**Table 10-3. Beagle Dog PK Dosing Scheme for Example 18**

| Group | Method of administration | Number of animals | Sample | Dose of administration (mg/kg) | Volume of administration (mL/kg) | Concentration of formulation (mg/mL) |
|---|---|---|---|---|---|---|
| 1 | IV | 3 | Plasma | 1 | 1 | 1 |
| 2 | PO | 2 | Plasma | 3 | 3 | 1 |

**Table 10-4. Beagle Dog PK Dosing Scheme for Example 42**

| Group | Method of administration | Number of animals | Sample | Dose of administration (mg/kg) | Volume of administration (mL/kg) | Concentration of formulation (mg/mL) |
|---|---|---|---|---|---|---|
| 1 | IV | 3 | Whole blood | 1 | 1 | 1 |
| 2 | PO | 3 | Whole blood | 3 | 3 | 1 |

**Table 11-1. Rat Pharmacokinetic Sampling Protocol**

| Group | Method of administration | Sampling | Anticoagulant | Pharmacokinetic time point |
|---|---|---|---|---|
| 1 | IV | Jugular Vein Cannulation (JVC) | K₂EDTA | Whole blood: pre-dose, 5, 15, 30 minutes, 1, 2, 4, 7 and 24 hours post-dose |
| 2 | PO | Jugular Vein Cannulation (JVC) | K₂EDTA | Whole blood: pre-dose, 15, 30 minutes, 1, 2, 4, 7 and 24 hours post-dose |
| 3 | PO | Blood was collected from the heart, the animal was anesthetized, and brain tissue was collected. | K₂EDTA | Whole blood: 2 hours |
| | | | | Brain tissue: 2 hours |

Rats were administered according to the above protocol, and blood and brain tissue samples were collected and processed at predetermined time points (collection and processing were performed according to conventional methods in the art).

**Table 11-2. Mouse Pharmacokinetic Sampling Protocol**

| Group | Method of administration | Sampling | Anticoagulant | Pharmacokinetic time point |
|---|---|---|---|---|
| 1 | IV | Dorsum of foot | K₂EDTA | Whole blood: pre-dose, 5, 15, 30 minutes, 1, 2, 4, 7 and 24 hours post-dose |
| 2 | PO | Dorsum of foot | K₂EDTA | Whole blood: pre-dose, 15, 30 minutes, 1, 2, 4, 7, and 24 hours post-dose |
| 3 | PO | Blood was collected from the heart, animals were anesthetized, and brain tissue was collected. | K₂EDTA | Whole blood: 2 hours |
| | | | | Brain tissue: 2 hours |

Mice were administered according to the above protocol, and blood and brain tissue samples were collected and processed at predetermined time points (collection and processing were performed according to conventional methods in the art).

**Table 11-3. Beagle Dog Pharmacokinetic Sampling Protocol for Example 18**

| Group | Method of administration | Sampling | Anticoagulant | Pharmacokinetic time point |
|---|---|---|---|---|
| 1 | IV | Peripheral vascular puncture (cephalic vein or saphenous vein) | K₂EDTA | Plasma: pre-dose, 5, 15, 30 minutes, 1, 2, 4, 7 and 24 hours post-dose |
| 2 | PO | Peripheral vascular puncture (cephalic vein or saphenous vein) | K₂EDTA | Plasma: pre-dose, 15, 30 minutes, 1, 2, 4, 7 and 24 hours post-dose |

**Table 11-4. Beagle Dog Pharmacokinetic Sampling Protocol for Example 42**

| Group | Method of administration | Sampling | Anticoagulant | Pharmacokinetic time point |
|---|---|---|---|---|
| 1 | IV | Peripheral vascular puncture (cephalic vein or saphenous vein) | K₂EDTA | Whole blood: pre-dose, 5, 15, 30 minutes, 1, 2, 4, 7 and 24 hours post-dose |
| 2 | PO | Peripheral vascular puncture (cephalic vein or saphenous vein) | K₂EDTA | Whole blood: pre-dose, 15, 30 minutes, 1, 2, 4, 7, and 24 hours post-dose |

### (v) Analysis of samples

The brain was weighed and homogenized by adding 4 times of ultrapure water. Six times volume of acetonitrile was added respectively to the whole blood samples and brain homogenate (20 times volume of acetonitrile was added to the whole blood sample of mouse PK). The mixture was vortexed for 1 min and then centrifuged at 4°C, 4500 rpm for 15 min. According to the instrument response, the supernatant was diluted with diluent and the sample was analyzed by LC/MS.

### (vi) Data analysis:

Pharmacokinetic parameters were calculated using WinNonlin software. The following pharmacokinetic parameters were calculated if appropriate plasma/whole blood drug concentration-time data were available: CL (clearance); V_{d} (apparent volume of distribution); T_{1/2} (elimination half-life time); Cₘₐₓ (peak concentration); Tₘₐₓ (time to peak); AUC (area under plasma drug concentration-time curve); MRT (mean retention time); F% (bioavailability).

The test results are shown in Table 12 to Table 14 below, which respectively give the blood drug concentrations of Example compounds 18, 40 and 42 of the present application at various time points, as well as the values of various pharmacokinetic parameters, and also give the concentrations and ratios of Example compounds 18, 40 and 42 of the present application in the brain and blood of rats or mice. From the above results, it can be seen that the compounds of Examples 18, 40 and 42 of the present application exhibit excellent pharmacokinetic properties and the ability to penetrate the blood-brain barrier.

**Table 12-1. Blood drug concentration (ng/mL) of the compound of Example 18 of the present application in rat/beagle dog test**

| **Time point (hr)** | Rat | | Beagle dog | |
|---|---|---|---|---|
| | **IV 3 mg/kg** | **PO 30 mg/kg** | **IV 1 mg/kg** | **PO 3 mg/kg** |
| 0 | 0 | 0 | 0 | 0 |
| 0.083 | 1169 ± 149 | / | 843 ± 237 | / |
| 0.25 | 571 ± 31 | 85.6 ± 26.6 | 575 ± 222 | 469 |
| 0.5 | 275 ± 24 | 239 ± 47 | 334 ± 229 | 1064 |
| 1 | 94.7 ± 10.7 | 309 ± 103 | 170 ± 148 | 1455 |
| 2 | 25.5 ± 4.7 | 165 ± 90 | 57.9 ± 63.2 | 932 |
| 4 | 4.13 ± 0.75 | 68.7 ± 31 | 26.0 ± 24.1 | 138 |
| 7 | 0.927 ± 0.301 | 23.5 ± 5.2 | 10.0 ± NA | 82.4 |
| 24 | NA | 0.557 ± NA | NA | 4.41 |

**Table 12-2. Blood drug concentration (ng/mL) of the compound of Example 40 of the present application in mouse/rat test**

| **Time point (hr)** | Mouse | | Rat | |
|---|---|---|---|---|
| | **IV 2 mg/kg** | **PO 20 mg/kg** | **IV 3 mg/kg** | **PO 30 mg/kg** |
| 0 | 0 | 0 | 0 | 0 |
| 0.083 | 632 ± 86 | / | 865 ± 45 | / |
| 0.25 | 323 ± 81 | 284 ± 26 | 496 ± 33 | 185 ± 86 |
| 0.5 | 150 ± 53 | 405 ± 46 | 295 ± 29 | 416 ± 92 |
| 1 | 60.7 ± 28.5 | 399 ± 77 | 119 ± 14 | 547 ± 248 |
| 2 | 12.3 ± 8.4 | 163 ± 2 | 61.9 ± 8.5 | 372 ± 258 |
| 4 | 1.77 ± 1.12 | 34.9 ± 19.0 | 13.2 ± 5 | 109 ± 99 |
| 7 | 0.854 ± NA | 4.85 ± 3.83 | 2.37 ± 0.77 | 59.8 ± 34.9 |
| 24 | NA | NA | NA | 0.930 ± NA |

**Table 12-3. Blood drug concentration of the compound of Example 42 of the present application in rat/beagle dog test (ng/mL)**

| **Time point (hr)** | Rat | | Beagle | |
|---|---|---|---|---|
| | **IV 3 mg/kg** | **PO 30 mg/kg** | **IV 1 mg/kg** | **PO 3 mg/kg** |
| 0 | 0 | 0 | 0 | 0 |
| 0.083 | 798 ± 302 | / | 535 ± 62 | / |
| 0.25 | 565 ± 166 | 266 ± 89 | 451 ± 77 | 645 ± 362 |
| 0.5 | 445 ± 70 | 654 ± 74 | 390 ± 13 | 1323 ± 406 |
| 1 | 368 ± 53 | 1253 ± 67 | 253 ± 49 | 1174 ± 696 |
| 2 | 303 ± 39 | 1297 ± 178 | 236 ± 69 | 1315 ± 320 |
| 4 | 269 ± 25 | 1380 ± 387 | 176 ± 29 | 560 ± 231 |
| 7 | 199 ± 23 | 1553 ± 153 | 133 ± 14 | 406 ± 145 |
| 24 | 19.9 ± 2.4 | 237 ± 95 | 32.9 ± 13.5 | 260 ± 19 |

**Table 13-1. Pharmacokinetic parameters of the compound of Example 18 of the present application in rat/beagle dog test**

| **Parameter** | Rat | | Beagle dog | |
|---|---|---|---|---|
| | **IV 3 mg/kg** | **PO 30 mg/kg** | **IV 1 mg/kg** | **PO 3 mg/kg** |
| Tₘₐₓ (hr) | / | 1.00 ± 0.00 | / | 1.00 |
| Cₘₐₓ (ng/mL) | / | 309 ± 103 | / | 1455 |
| T_{1/2} (hr) | 1.06 ± 0.07 | 3.10 ± 0.24 | 1.13 ± 0.65 | 4.55 |
| AUC₀₋ₜ (hr*ng/mL) | 559 ± 62 | 922 ± 357 | 682 ± 447 | 4213 |
| AUC_{inf} (hr*ng/mL) | 560 ± 62 | 967 ± 285 | 699 ± 474 | 4239 |
| Cl_{_obs} (mL/min/kg) | 90.1 ± 10.7 | / | 31.4 ± 17.4 | / |
| V_{d} (L/kg) | 8.24 ± 0.68 | / | 2.42 ± 0.54 | / |
| F% | / | 17.3 ± 5.1 | / | 183 |

**Table 13-2. Pharmacokinetic parameters of the compound of Example 40 of the present application in mouse/rat test**

| **Parameter** | Mouse | | Rat | |
|---|---|---|---|---|
| | **IV 2 mg/kg** | **PO 20 mg/kg** | **IV 3 mg/kg** | **PO 30 mg/kg** |
| Tₘₐₓ (hr) | / | 0.833 ± 0.289 | / | 1.17 ± 0.76 |
| Cₘₐₓ (ng/mL) | / | 421 ± 64 | / | 581 228 |
| T_{1/2} (hr) | 0.755 ± 0.299 | 0.967 ± 0.208 | 1.07 ± 0.08 | 2.84 ± 0.46 |
| AUC₀₋ₜ (hr*ng/mL) | 307 ± 82 | 861 ± 54 | 588 ± 56 | 1904 ± 868 |
| AUC_{inf} (hr*ng/mL) | 308 ± 82 | 869 ± 57 | 592 ± 57 | 1968 ± 768 |
| Cl_{_obs} (mL/min/kg) | 114 ± 30.5 | / | 85.0 ± 8.4 | / |
| V_{d} (L/kg) | 6.97 ± 1.13 | / | 7.84 ± 0.50 | / |
| F% | / | 28.2 ± 1.9 | / | 33.2 ± 13.0 |

**Table 13-1. Pharmacokinetic parameters of the compound of Example 42 of the present application in rat/beagle dog test**

| **Parameter** | Rat | | Beagle dog | |
|---|---|---|---|---|
| | **IV 3 mg/kg** | **PO 30 mg/kg** | **IV 1 mg/kg** | **PO 3 mg/kg** |
| Tₘₐₓ (hr) | / | 6.00 ± 1.73 | / | 1.17 ± 0.76 |
| Cₘₐₓ (ng/mL) | / | 1583 ± 205 | / | 1510 ± 396 |
| T_{1/2} (hr) | 5.25 ± 0.14 | 6.33 ± 1.56 | 8.72 ± 3.00 | 18.1 ± 5.0 |
| AUC₀₋ₜ (hr*ng/mL) | 3986 ± 225 | 24192 ± 2747 | 2923 ± 268 | 11174 ± 2941 |
| AUC_{inf} (hr*ng/mL) | 4137 ± 234 | 26495 ± 3062 | 3376 ± 342 | 18035 ±939 |
| Cl_{_obs}(mL/min/kg) | 12.1 ± 0.7 | / | 4.97 ±0.51 | / |
| V_{d} (L/kg) | 5.50 ± 0.38 | / | 3.70 ± 1.05 | / |
| F% | / | 64.0 ± 7.4 | / | 127 ± 28 |

**Table 14. Concentrations and ratios of the compounds of Examples 18, 40 and 42 of the present application in brain and whole blood (sampling time, 2 h after administration)**

| **Example** | **Species** | **Dosage (mg/kg)** | **Blood drug concentration (ng/mL)** | **Brain concentration (ng/g)** | **Brain/blood ratio** |
|---|---|---|---|---|---|
| 18 | Rat | 30 | 266 | 397 | 1.49 |
| 40 | Mouse | 20 | 132 | 244 | 1.85 |
| 40 | Rat | 30 | 274 | 309 | 1.13 |
| 42 | Rat | 30 | 2153 | 1970 | 0.915 |

The biological data provided by the present disclosure indicate that the compounds of the present disclosure are useful for treating or preventing diseases caused by abnormal RIPK1 kinase. Therefore, the compounds of the present disclosure are useful for treating diseases associated with RIPK1, including ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic granulocytic leukemia, acute myelocytic leukemia, non-Hodgkin's lymphoma, nasopharyngeal cancer, esophageal cancer, brain tumor, B-cell and T-cell lymphoma, lymphoma, multiple myeloma, biliary tract carcinosarcoma, cholangiocarcinoma, inflammatory bowel disease, ulcerative colitis, retinal detachment, retinitis pigmentosa, macular degeneration, pancreatitis, atopic dermatitis, spondyloarthritis, gout, SoJIA, Sjogren's syndrome, systemic scleroderma, antiphospholipid syndrome, vasculitis, osteoarthritis, non-alcoholic steatohepatitis, alcoholic steatohepatitis, autoimmune hepatitis, autoimmune hepatobiliary disease, primary sclerosing cholangitis, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic diseases, asthma, atopic dermatitis, multiple sclerosis, type I diabetes, Wegener's granulomatosis, pulmonary sarcoidosis, Behcet's disease, interleukin-1 converzyme-related fever syndrome, chronic obstructive pulmonary disease, tumor necrosis factor receptor related periodic syndrome and periodontitis. The compounds of the present disclosure can be used as a monotherapy or in combination therapy, and can be used in combination with multiple compounds of the present disclosure or in combination with other drugs other than those of the present disclosure.

The above description is only an alternative embodiment of the present disclosure and is not intended to limit the present disclosure. Any modifications, equivalent substitutions and improvements made within the spirit and principles of the present disclosure should be included in the protection scope of the present disclosure.

## Claims

1. A compound represented by formula (I), or a stereoisomer, pharmaceutically acceptable salt, or deuterated derivative thereof, 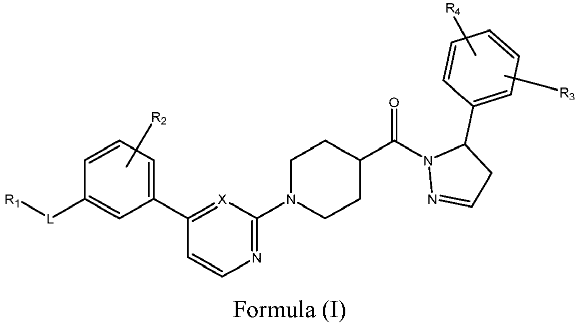
in Formula (I), X is CH or N;
L is O, S, NH, carbonyl, sulfonyl or sulfinyl;
R₁ is C₁-C₁₀ alkyl, C₃-C₈ cycloalkyl, 4- to 8-membered heteroalicyclyl, or C₁-C₁₀ alkyl substituted with 1 to 3 substituents selected from hydroxyl, C₁-C₆ alkoxy, cyano, -NR^{a}R^{b}, C₃-C₈ cycloalkyloxy, -CONH-R₅, C₃-C₈ cycloalkyl, hydroxyl- and/or C₁-C₄ alkyl-substituted C₃-C₈ cycloalkyl, carboxyl, halogen, C₁-C₆ haloalkoxy, -SO₂-R₅, -SO-Rs, -CO-Rs, C₂-C₆ alkynyl, C₂-C₆ alkenyl, C₁-C₄ alkoxyC₁-C₆ alkoxy, 4- to 8-membered heteroalicyclyl, oxo-substituted 4- to 8-membered heteroalicyclyl, hydroxyl- and/or C₁-C₄ alkyl-substituted 4- to 8-membered heteroalicyclyl and C₁-C₆ alkylthio,
the 4- to 8-membered heteroalicyclyl is 4- to 8-membered heteroalicyclyl containing 1-2 atoms selected from N, O, and S as a ring atom,
R₅ is hydrogen, hydroxyl, C₁-C₆ alkyl, C₁-C₆ alkoxyC₁-C₆ alkyl, hydroxyl-substituted C₁-C₆ alkyl, C₃-C₈ cycloalkyl or 4- to 8-membered heteroalicyclyl-substituted C₁-C₆ alkyl,
R^{a} and R^{b} are each independently hydrogen, C₁-C₆ alkyl, C₃-C₈ cycloalkyl, C₁-C₆ alkoxy-substituted C₁-C₆ alkyl, hydroxyl-substituted C₁-C₆ alkyl, C₃-C₈ cycloalkylC₁-C₆ alkyl, 4- to 8-membered heteroalicyclyl-substituted C₁-C₆ alkyl, C₁-C₃ alkylthio-substituted C₁-C₆ alkyl, mono- or di-C₁-C₃ alkyl-substituted or unsubstituted amino-substituted C₁-C₆ alkyl;
R₂ is hydrogen, C₁-C₃ alkyl, C₁-C₃ alkoxy or halogen;
R₃ and R₄ are each independently hydrogen, halogen or methyl.

2. The compound according to claim 1, or a stereoisomer, or pharmaceutically acceptable salt thereof, wherein the compound has a structure of the following formula (II): 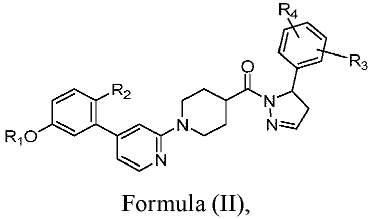
in Formula (II),
R₁ is C₁-C₁₀ alkyl, C₃-C₈ cycloalkyl, 4- to 8-membered heteroalicyclyl, or C₁-C₁₀ alkyl substituted with 1 to 3 substituents selected from hydroxyl, C₁-C₆ alkoxy, cyano, -NR^{a}R^{b}, C₃-C₈ cycloalkyloxy, -CONH-R₅, C₃-C₈ cycloalkyl, hydroxyl- and/or C₁-C₄ alkyl-substituted C₃-C₈ cycloalkyl, carboxyl, halogen, C₁-C₆ haloalkoxy, -SO₂-R₅, -SO-Rs, -CO-Rs, C₂-C₆ alkynyl, C₂-C₆ alkenyl, C₁-C₄ alkoxyC₁-C₆ alkoxy, 4- to 8-membered heteroalicyclyl, oxo-substituted 4- to 8-membered heteroalicyclyl, hydroxyl- and/or C₁-C₄ alkyl-substituted 4- to 8-membered heteroalicyclyl, and C₁-C₆ alkylthio,
the 4- to 8-membered heteroalicyclyl is 4- to 8-membered heteroalicyclyl containing 1-2 atoms selected from N, O, and S as a ring atom,
R₅ is hydrogen, hydroxyl, C₁-C₆ alkyl, C₁-C₆ alkoxyC₁-C₆ alkyl, hydroxyl-substituted C₁-C₆ alkyl, C₃-C₈ cycloalkyl or 4- to 8-membered heteroalicyclyl-substituted C₁-C₆ alkyl,
R^{a} and R^{b} are each independently hydrogen, C₁-C₆ alkyl, C₃-C₈ cycloalkyl, C₁-C₆ alkoxy-substituted C₁-C₆ alkyl, hydroxyl-substituted C₁-C₆ alkyl, C₃-C₈ cycloalkylC₁-C₆ alkyl, 4- to 8-membered heteroalicyclyl-substituted C₁-C₆ alkyl, C₁-C₃ alkylthio-substituted C₁-C₆ alkyl, or mono- or di-C₁-C₃ alkyl-substituted or unsubstituted amino-substituted C₁-C₆ alkyl;
R₂ is hydrogen, C₁-C₃ alkyl, C₁-C₃ alkoxy or halogen;
R₃ and R₄ are each independently hydrogen, halogen or methyl.

3. The compound according to claim 1 or 2, or a stereoisomer, or pharmaceutically acceptable salt thereof, wherein:
R₁ is C₁-C₈ alkyl, C₃-C₆ cycloalkyl, 4- to 6-membered heteroalicyclyl, or C₁-C₈ alkyl substituted with 1 to 3 substituents selected from hydroxyl, C₁-C₃ alkoxy, cyano, C₃-C₆ cycloalkyloxy, C₃-C₆ cycloalkyl, hydroxyl- and/or C₁-C₄ alkyl-substituted C₃-C₆ cycloalkyl, halogen, 4- to 6-membered heteroalicyclyl, oxo-substituted 4- to 6-membered heteroalicyclyl, hydroxyl- and/or C₁-C₄ alkyl-substituted 4- to 6-membered heteroalicyclyl, and C₁-C₃ alkylthio,
the 4- to 6-membered heteroalicyclyl is 4- to 6-membered heteroalicyclyl containing 1-2 atoms selected from N, O, and S as a ring atom.

4. The compound according to claim 3, or a stereoisomer, or pharmaceutically acceptable salt thereof, wherein R₁ is methyl, ethyl, propyl, isopropyl, butyl, isobutyl, tert-butyl, pentyl, isopentyl, hexyl, octyl, cyclobutyl, cyclopentyl, cyclohexyl, tetrahydrofuran-2-yl, tetrahydrofuran-3-yl, tetrahydropyran-2-yl, tetrahydropyran-3-yl, tetrahydropyran-4-yl, or C₁-C₈ alkyl substituted with 1 to 3 substituents selected from hydroxyl, methoxy, ethoxy, propoxy, isopropoxy, cyano, cyclobutyloxy, cyclopentyloxy, cyclohexyloxy, cyclobutyl, cyclopentyl, cyclohexyl, 4-hydroxylcyclohexyl, 4-hydroxyl-4-methylcyclohexyl, fluorine, chlorine, tetrahydrofuran-2-yl, tetrahydrofuran-3-yl, tetrahydropyran-2-yl, tetrahydropyran-3-yl, tetrahydropyran-4-yl, pyrrolidin-1-yl, pyrrolidin-2-yl, piperidin-1-yl, piperidin-4-yl, morpholinyl, thiomorpholinyl, 1-methyl-pyrrolidin-2-yl, 1-methyl-piperidin-4-yl, methylthio, ethylthio, propylthio, and isopropylthio;
alternatively, R₁ is methyl, ethyl, propyl, butyl, pentyl, hexyl, hydroxyethyl, hydroxypropyl, hydroxybutyl, hydroxypentyl, hydroxyhexyl, methoxyethyl, methoxypropyl, methoxybutyl, methoxypentyl, methoxyhexyl, tetrahydropyran-4-yl, 4-methyl-4-hydroxypentyl, tetrahydropyran-4-ylethyl, tetrahydropyran-4-ylmethyl, tetrahydropyran-4-ylpropyl, tetrahydropyran-4-ylbutyl, 3-methyl-3-hydroxylbutyl, 2-methyl-2-hydroxylpropyl, 5-methyl-5-hydroxylhexyl, fluoropropyl, fluoroethyl, or 2,2-difluoro-3-hydroxyl-propyl.

5. The compound according to claim 3, or a stereoisomer, or pharmaceutically acceptable salt thereof, wherein R₁ is C₁-C₈ alkyl substituted with 1-hydroxycyclopropyl, 1-hydroxycyclobutyl, 1-hydroxycyclopentyl or 1-hydroxycyclohexyl;
alternatively, R₁ is 1-hydroxycyclopropylmethyl or 1-hydroxycyclobutylmethyl.

6. The compound according to any one of claims 1 to 4, or a stereoisomer, or pharmaceutically acceptable salt thereof, wherein R₂ is hydrogen, methyl, methoxy, fluorine, chlorine, or bromine.

7. The compound according to any one of claims 1 to 4, or a stereoisomer, or pharmaceutically acceptable salt thereof, wherein R₃ and R₄ are each independently hydrogen, fluorine, chlorine, or methyl.

8. The compound according to claim 1, or a pharmaceutically acceptable salt thereof, wherein the compound has a structure of the following formula (III):

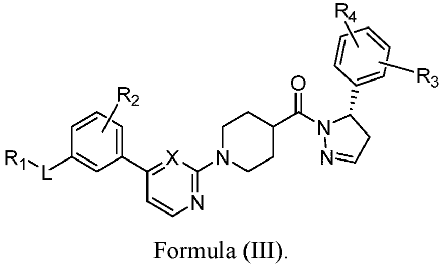

9. The compound according to claim 2, or a pharmaceutically acceptable salt thereof, wherein the compound has a structure of the following formula (IV):

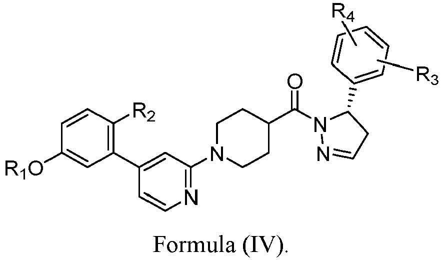

10. The compound according to claim 1, or a stereoisomer, or pharmaceutically acceptable salt thereof, wherein the compound is selected from the following structures:

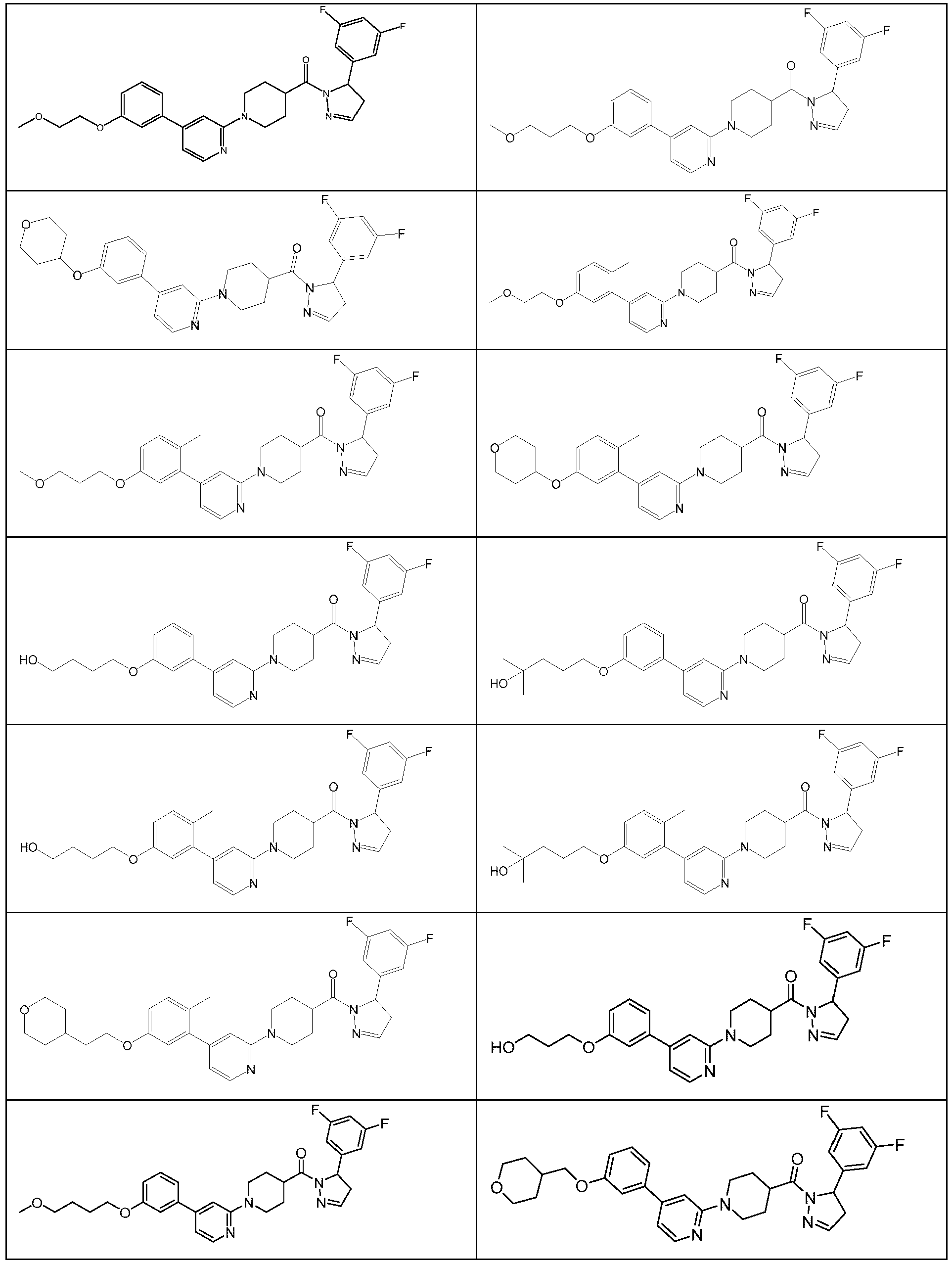

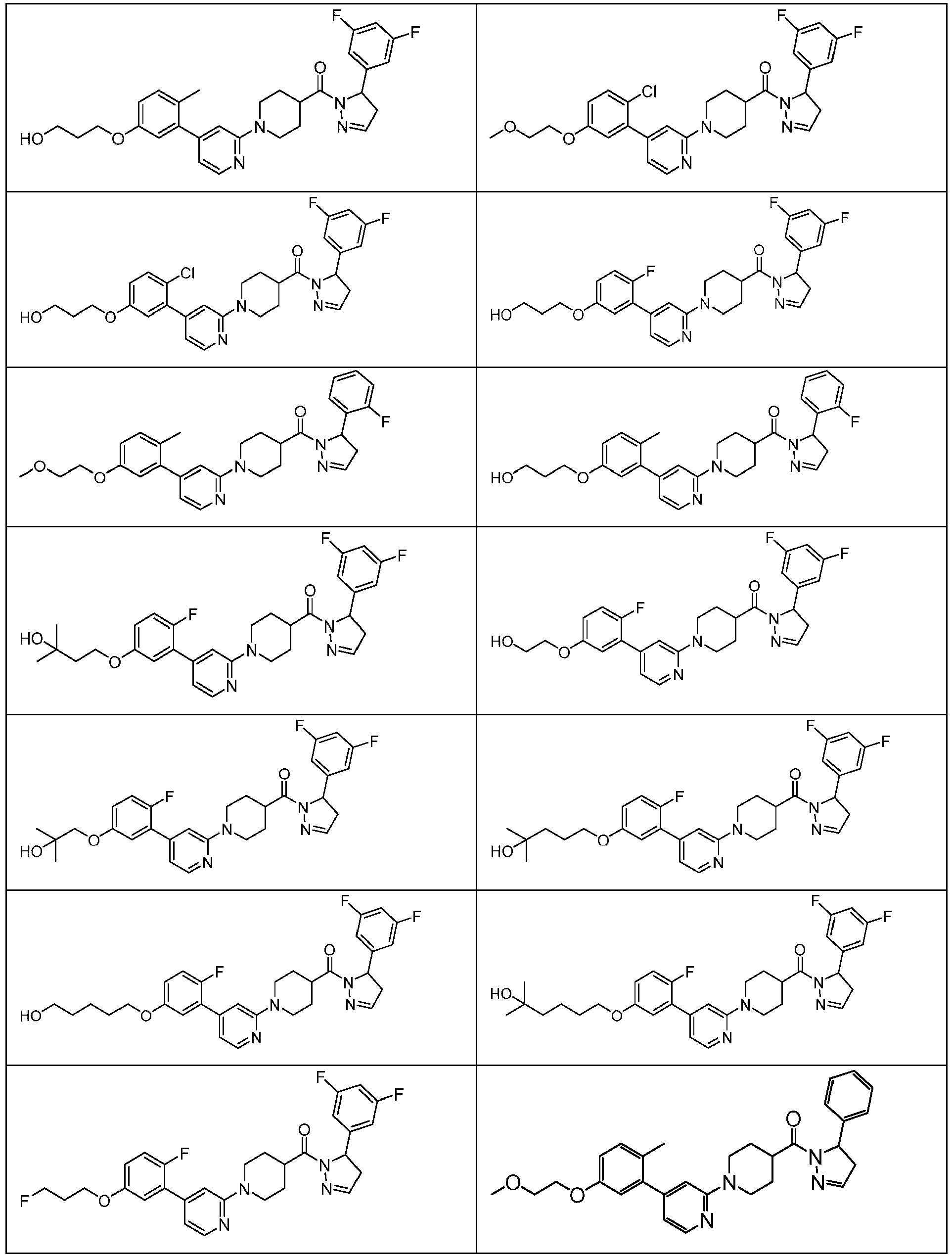

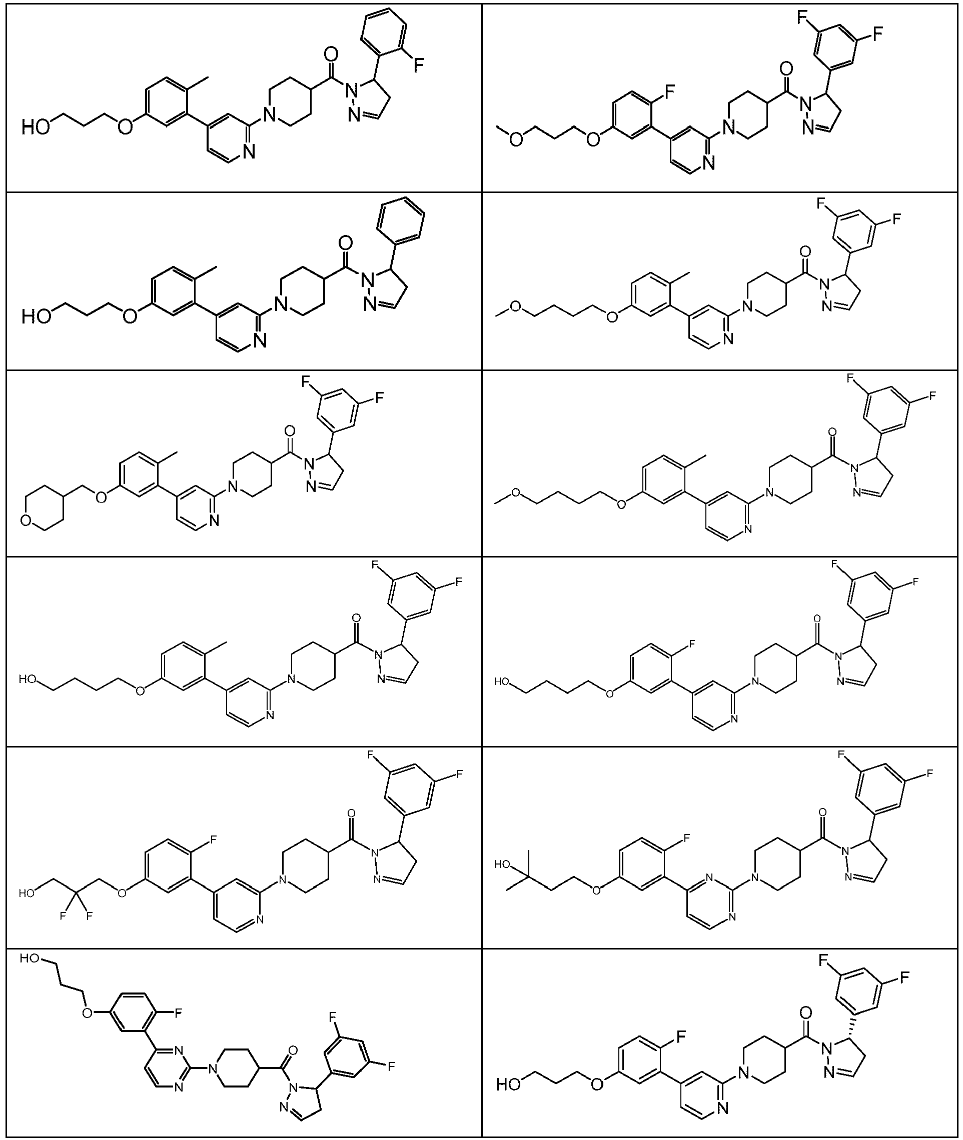

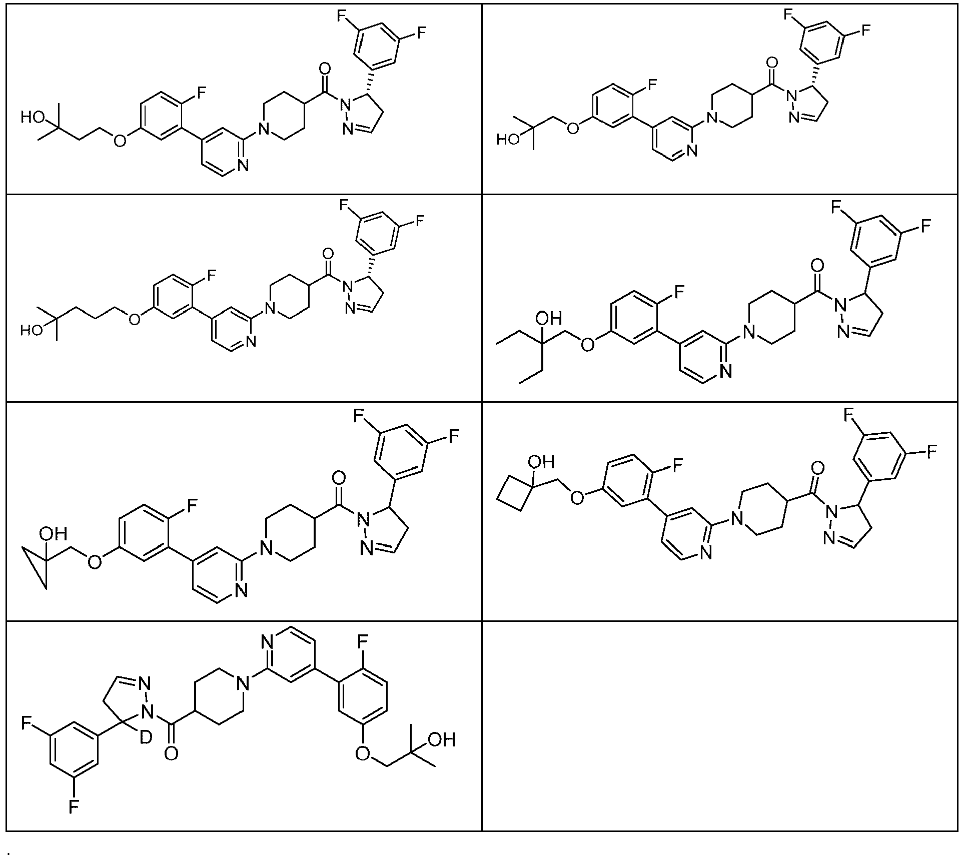

11. A deuterated derivative of the compound according to any one of claims 2 to 10.

12. Use of the compound according to any one of claims 1 to 11, or a stereoisomer, pharmaceutically acceptable salt or deuterated derivative thereof in the manufacture of a medicament for treating diseases associated with RIPK1.

13. The use of claim 12, wherein the diseases associated with RIPK1 include: ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic granulocytic leukemia, acute myelocytic leukemia, non-Hodgkin's lymphoma, nasopharyngeal cancer, esophageal cancer, brain tumor, B-cell and T-cell lymphoma, lymphoma, multiple myeloma, biliary tract carcinosarcoma, cholangiocarcinoma, inflammatory bowel disease, ulcerative colitis, retinal detachment, retinitis pigmentosa, macular degeneration, pancreatitis, atopic dermatitis, spondyloarthritis, gout, SoJIA, Sjogren's syndrome, systemic scleroderma, antiphospholipid syndrome, vasculitis, osteoarthritis, non-alcoholic steatohepatitis, alcoholic steatohepatitis, autoimmune hepatitis, autoimmune hepatobiliary disease, primary sclerosing cholangitis, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic diseases, asthma, atopic dermatitis, multiple sclerosis, type I diabetes, Wegener's granulomatosis, pulmonary sarcoidosis, Behcet's disease, interleukin-1 converzyme-related fever syndrome, chronic obstructive pulmonary disease, tumor necrosis factor receptor related periodic syndrome and periodontitis.

14. A pharmaceutical composition comprising the compound according to any one of claims 1 to 11, or a stereoisomer, pharmaceutically acceptable salt or deuterated derivative thereof, and one or more pharmaceutically acceptable carriers or excipients.

15. The pharmaceutical composition according to claim 14, further comprising one or more other therapeutic agents.
